# EUROPEAN PATENT APPLICATION

(11) **EP 4 725 963 A1**
(43) Date of publication of application: **15.04.2026**
(21) Application number: 24818783.3
(22) Date of filing: 07.06.2024
(51) Int. Cl.: C07K 16/28, C07K 14/58, C07K 14/72, A61K 39/395, A61P 9/12, A61P 9/04

(54) **ANTI-NPR1 ANTIBODY AND PHARMACEUTICAL USE THEREOF**

(30) Priority: 08.06.2023 CN 202310675148
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: YING, Hanxiao, Shanghai 200245 (CN); SHI, Jinping, Shanghai 200245 (CN); XU, Yingxia, Shanghai 200245 (CN); JIN, Xinsheng, Shanghai 200245 (CN); LI, Xun, Shanghai 200245 (CN)
(74) Representative: Potter Clarkson
(86) International application number: PCT/CN2024/098066
(87) International publication number: WO 2024/251255

(57) **Abstract**

Provided are an anti-NPR1 antibody, a pharmaceutical composition comprising the antibody, and an immunoconjugate. Also provided are an isolated nucleic acid encoding the NPR1 antibody, a host cell comprising the isolated nucleic acid, a method for using the NPR1 antibody to detect an NPR1 peptide or a fragment thereof in a sample, and a method for using the NPR1 antibody, the pharmaceutical composition, and the immunoconjugate for treating, preventing or ameliorating diseases or conditions related to NPR1, the diseases or conditions related to NPR1 comprising hypertension.

## Description

The present application claims priority to Chinese Patent Application No. CN202310675148.4, which was filed on June 8, 2023.

### TECHNICAL FIELD

The present disclosure pertains to the technical field of biology. Specifically, the present disclosure relates to an anti-NPR1 antibody and pharmaceutical use thereof.

### BACKGROUND

The statements herein merely provide background information related to the present disclosure and may not necessarily constitute the prior art.

The natriuretic peptide system plays a crucial role in maintaining renal, cardiovascular, endocrine, neural, and skeletal homeostasis. This system consists of polypeptide ligands, including ANP, BNP, and CNP, and membrane receptors, including NPR1 (NPRA), NPR2 (NPRB), and NPR3 (NPRC). NPR1 is mainly expressed in the heart, kidneys, lungs, aorta, etc. By interacting with the ligand ANP or BNP, NPR1 undergoes a conformational change and activates downstream guanylate cyclase activity. The enzyme catalyzes the cyclization of intracellular guanosine triphosphate (GTP), which results in the formation of cyclic guanosine monophosphate (cGMP). cGMP, in turn, activates cGMP-dependent protein kinase (protein G), fulfilling biological functions such as vasodilation, blood pressure reduction, sodium excretion, diuresis, and resistance to cardiac hypertrophy and fibrosis. Hence, the activation of this pathway can be used to treat heart failure and hypertension.

Existing recombinant forms of ANP and BNP have been approved and marketed in China, Japan, and the United States for the treatment of acute heart failure. Recombinant polypeptides of this kind are effective in lowering blood pressure and alleviating heart failure symptoms. However, they do not significantly improve the re-hospitalization rate and mortality, likely due to their relatively short PK, which limits long-term use. Consequently, they have not been approved for chronic heart failure indications.

Novartis and Regeneron have each developed NPR1 agonist antibodies. Regeneron's REGN-5381 (WO2020086406A2) is currently in a phase II clinical trial for evaluating its safety in heart failure patients, and Novartis's XXB750 (WO2020250159A1) is in a phase II clinical trial for evaluating its efficacy against resistant hypertension.

### SUMMARY

The present disclosure provides an anti-NPR1 antibody comprising a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
a. the HCDR1, the HCDR2, and the HCDR3 of the heavy chain variable region comprise the amino acid sequences of a HCDR1, a HCDR2, and a HCDR3 in SEQ ID NO: 82, 80, 81, or 15, respectively, and the LCDR1, the LCDR2, and the LCDR3 of the light chain variable region comprise the amino acid sequences of a LCDR1, a LCDR2, and a LCDR3 in SEQ ID NO: 85, 83, 84, or 16, respectively; or
b. the HCDR1, the HCDR2, and the HCDR3 of the heavy chain variable region comprise the amino acid sequences of a HCDR1, a HCDR2, and a HCDR3 of any one of SEQ ID NO: 51, 48, 49, 50, 52, or 7, respectively, and the LCDR1, the LCDR2, and the LCDR3 of the light chain variable region comprise the amino acid sequences of a LCDR1, a LCDR2, and a LCDR3 in SEQ ID NO: 56, 53, 54, 55, or 8, respectively; or
c. the HCDR1, the HCDR2, and the HCDR3 of the heavy chain variable region comprise the amino acid sequences of a HCDR1, a HCDR2, and a HCDR3 in SEQ ID NO: 68, 66, 67, 69, or 11, respectively, and the LCDR1, the LCDR2, and the LCDR3 of the light chain variable region comprise the amino acid sequences of a LCDR1, a LCDR2, and a LCDR3 in SEQ ID NO: 74, 70, 71, 72, or 12, respectively; or
d. the HCDR1, the HCDR2, and the HCDR3 of the heavy chain variable region comprise the amino acid sequences of a HCDR1, a HCDR2, and a HCDR3 in SEQ ID NO: 78, 76, 77, or 13, respectively, and the LCDR1, the LCDR2, and the LCDR3 of the light chain variable region comprise the amino acid sequences of a LCDR1, a LCDR2, and a LCDR3 in SEQ ID NO: 79 or 14, respectively; or
e. the HCDR1, the HCDR2, and the HCDR3 of the heavy chain variable region comprise the amino acid sequences of a HCDR1, a HCDR2, and a HCDR3 in SEQ ID NO: 58, 59, 60, or 9, respectively, and the LCDR1, the LCDR2, and the LCDR3 of the light chain variable region comprise the amino acid sequences of a LCDR1, a LCDR2, and a LCDR3 in SEQ ID NO: 61, 62, 63, 64, 65, or 10, respectively.

In some embodiments, provided is the aforementioned anti-NPR1 antibody, wherein:
a. the HCDR1, the HCDR2, and the HCDR3 of the heavy chain variable region comprise the amino acid sequences of a HCDR1, a HCDR2, and a HCDR3 in SEQ ID NO: 82, respectively, and the LCDR1, the LCDR2, and the LCDR3 of the light chain variable region comprise the amino acid sequences of a LCDR1, a LCDR2, and a LCDR3 in SEQ ID NO: 85, respectively; or
b. the HCDR1, the HCDR2, and the HCDR3 of the heavy chain variable region comprise the amino acid sequences of a HCDR1, a HCDR2, and a HCDR3 of any one of SEQ ID NO: 51, 48, or 52, respectively, and the LCDR1, the LCDR2, and the LCDR3 of the light chain variable region comprise the amino acid sequences of a LCDR1, a LCDR2, and a LCDR3 in SEQ ID NO: 56, respectively; or
c. the HCDR1, the HCDR2, and the HCDR3 of the heavy chain variable region comprise the amino acid sequences of a HCDR1, a HCDR2, and a HCDR3 in SEQ ID NO: 68, respectively, and the LCDR1, the LCDR2, and the LCDR3 of the light chain variable region comprise the amino acid sequences of a LCDR1, a LCDR2, and a LCDR3 in SEQ ID NO: 74 or 70, respectively; or
d. the HCDR1, the HCDR2, and the HCDR3 of the heavy chain variable region comprise the amino acid sequences of a HCDR1, a HCDR2, and a HCDR3 in SEQ **ID** NO: 78 or 76, respectively, and the LCDR1, the LCDR2, and the LCDR3 of the light chain variable region comprise the amino acid sequences of a LCDR1, a LCDR2, and a LCDR3 in SEQ ID NO: 79, respectively; or
e. the HCDR1, the HCDR2, and the HCDR3 of the heavy chain variable region comprise the amino acid sequences of a HCDR1, a HCDR2, and a HCDR3 in SEQ **ID** NO: 58 or 9, respectively, and the LCDR1, the LCDR2, and the LCDR3 of the light chain variable region comprise the amino acid sequences of a LCDR1, a LCDR2, and a LCDR3 in SEQ ID NO: 61 or 10, respectively.

In some embodiments, provided is the aforementioned anti-NPR1 antibody, wherein:
a. the HCDR1, the HCDR2, and the HCDR3 of the heavy chain variable region comprise the amino acid sequences of a HCDR1, a HCDR2, and a HCDR3 in SEQ ID NO: 82, respectively, and the LCDR1, the LCDR2, and the LCDR3 of the light chain variable region comprise the amino acid sequences of a LCDR1, a LCDR2, and a LCDR3 in SEQ ID NO: 85, respectively; or
b. the HCDR1, the HCDR2, and the HCDR3 of the heavy chain variable region comprise the amino acid sequences of a HCDR1, a HCDR2, and a HCDR3 in SEQ ID NO: 51, respectively, and the LCDR1, the LCDR2, and the LCDR3 of the light chain variable region comprise the amino acid sequences of a LCDR1, a LCDR2, and a LCDR3 in SEQ ID NO: 56, respectively; or
c. the HCDR1, the HCDR2, and the HCDR3 of the heavy chain variable region comprise the amino acid sequences of a HCDR1, a HCDR2, and a HCDR3 in SEQ ID NO: 68, respectively, and the LCDR1, the LCDR2, and the LCDR3 of the light chain variable region comprise the amino acid sequences of a LCDR1, a LCDR2, and a LCDR3 in SEQ ID NO: 74, respectively; or
d. the HCDR1, the HCDR2, and the HCDR3 of the heavy chain variable region comprise the amino acid sequences of a HCDR1, a HCDR2, and a HCDR3 in SEQ ID NO: 78, respectively, and the LCDR1, the LCDR2, and the LCDR3 of the light chain variable region comprise the amino acid sequences of a LCDR1, a LCDR2, and a LCDR3 in SEQ ID NO: 79, respectively; or
e. the HCDR1, the HCDR2, and the HCDR3 of the heavy chain variable region comprise the amino acid sequences of a HCDR1, a HCDR2, and a HCDR3 in SEQ ID NO: 58, respectively, and the LCDR1, the LCDR2, and the LCDR3 of the light chain variable region comprise the amino acid sequences of a LCDR1, a LCDR2, and a LCDR3 in SEQ ID NO: 61, respectively.

In some embodiments, provided is the aforementioned anti-NPR1 antibody, wherein:
a. the HCDR1, the HCDR2, and the HCDR3 of the heavy chain variable region comprise the amino acid sequences of a HCDR1, a HCDR2, and a HCDR3 in SEQ ID NO: 82, respectively, and the LCDR1, the LCDR2, and the LCDR3 of the light chain variable region comprise the amino acid sequences of a LCDR1, a LCDR2, and a LCDR3 in SEQ ID NO: 85, respectively; or
b. the HCDR1, the HCDR2, and the HCDR3 of the heavy chain variable region comprise the amino acid sequences of a HCDR1, a HCDR2, and a HCDR3 in SEQ ID NO: 51, respectively, and the LCDR1, the LCDR2, and the LCDR3 of the light chain variable region comprise the amino acid sequences of a LCDR1, a LCDR2, and a LCDR3 in SEQ ID NO: 56, respectively; or
c. the HCDR1, the HCDR2, and the HCDR3 of the heavy chain variable region comprise the amino acid sequences of a HCDR1, a HCDR2, and a HCDR3 in SEQ ID NO: 68, respectively, and the LCDR1, the LCDR2, and the LCDR3 of the light chain variable region comprise the amino acid sequences of a LCDR1, a LCDR2, and a LCDR3 in SEQ ID NO: 74, respectively; or
d. the HCDR1, the HCDR2, and the HCDR3 of the heavy chain variable region comprise the amino acid sequences of a HCDR1, a HCDR2, and a HCDR3 in SEQ ID NO: 78, respectively, and the LCDR1, the LCDR2, and the LCDR3 of the light chain variable region comprise the amino acid sequences of a LCDR1, a LCDR2, and a LCDR3 in SEQ ID NO: 79, respectively.

In some embodiments, provided is the aforementioned anti-NPR1 antibody, wherein:
a. the HCDR1, the HCDR2, and the HCDR3 of the heavy chain variable region comprise the amino acid sequences of a HCDR1, a HCDR2, and a HCDR3 in SEQ ID NO: 15, respectively, and the LCDR1, the LCDR2, and the LCDR3 of the light chain variable region comprise the amino acid sequences of a LCDR1, a LCDR2, and a LCDR3 in SEQ ID NO: 16, respectively; or
b. the HCDR1, the HCDR2, and the HCDR3 of the heavy chain variable region comprise the amino acid sequences of a HCDR1, a HCDR2, and a HCDR3 in SEQ ID NO: 7, respectively, and the LCDR1, the LCDR2, and the LCDR3 of the light chain variable region comprise the amino acid sequences of a LCDR1, a LCDR2, and a LCDR3 in SEQ ID NO: 8, respectively; or
c. the HCDR1, the HCDR2, and the HCDR3 of the heavy chain variable region comprise the amino acid sequences of a HCDR1, a HCDR2, and a HCDR3 in SEQ ID NO: 11, respectively, and the LCDR1, the LCDR2, and the LCDR3 of the light chain variable region comprise the amino acid sequences of a LCDR1, a LCDR2, and a LCDR3 in SEQ ID NO: 12, respectively; or
d. the HCDR1, the HCDR2, and the HCDR3 of the heavy chain variable region comprise the amino acid sequences of a HCDR1, a HCDR2, and a HCDR3 in SEQ ID NO: 13, respectively, and the LCDR1, the LCDR2, and the LCDR3 of the light chain variable region comprise the amino acid sequences of a LCDR1, a LCDR2, and a LCDR3 in SEQ ID NO: 14, respectively; or
e. the HCDR1, the HCDR2, and the HCDR3 of the heavy chain variable region comprise the amino acid sequences of a HCDR1, a HCDR2, and a HCDR3 in SEQ ID NO: 9, respectively, and the LCDR1, the LCDR2, and the LCDR3 of the light chain variable region comprise the amino acid sequences of a LCDR1, a LCDR2, and a LCDR3 in SEQ ID NO: 10, respectively.

In some embodiments, provided is the anti-NPR1 antibody according to any one of the above, wherein the HCDR1, the HCDR2, and the HCDR3 of the heavy chain variable region and the LCDR1, the LCDR2, and the LCDR3 of the light chain variable region are defined according to a numbering scheme selected from the group consisting of Kabat, IMGT, Chothia, AbM, and Contact. In some embodiments, the HCDR1, the HCDR2, and the HCDR3 of the heavy chain variable region and the LCDR1, the LCDR2, and the LCDR3 of the light chain variable region are defined according to the Kabat numbering scheme. In some embodiments, the HCDR1, the HCDR2, and the HCDR3 of the heavy chain variable region and the LCDR1, the LCDR2, and the LCDR3 of the light chain variable region are defined according to the IMGT numbering scheme. In some embodiments, the HCDR1, the HCDR2, and the HCDR3 of the heavy chain variable region and the LCDR1, the LCDR2, and the LCDR3 of the light chain variable region are defined according to the Chothia numbering scheme. In some embodiments, the HCDR1, the HCDR2, and the HCDR3 of the heavy chain variable region and the LCDR1, the LCDR2, and the LCDR3 of the light chain variable region are defined according to the AbM numbering scheme. In some embodiments, the HCDR1, the HCDR2, and the HCDR3 of the heavy chain variable region and the LCDR1, the LCDR2, and the LCDR3 of the light chain variable region are defined according to the Contact numbering scheme.

In some embodiments, provided is the anti-NPR1 antibody according to any one of the above, wherein:
a. in the heavy chain variable region, the HCDR1 comprises the amino acid sequence of SEQ ID NO: 29, the HCDR2 comprises the amino acid sequence of SEQ ID NO: 40, and the HCDR3 comprises the amino acid sequence of SEQ ID NO: 41, and in the light chain variable region, the LCDR1 comprises the amino acid sequence of SEQ ID NO: 42, the LCDR2 comprises the amino acid sequence of SEQ ID NO: 33, and the LCDR3 comprises the amino acid sequence of SEQ ID NO: 34; or
b. in the heavy chain variable region, the HCDR1 comprises the amino acid sequence of SEQ ID NO: 45, 17, or 35, the HCDR2 comprises the amino acid sequence of SEQ ID NO: 46 or 18, and the HCDR3 comprises the amino acid sequence of SEQ ID NO: 19, and in the light chain variable region, the LCDR1 comprises the amino acid sequence of SEQ ID NO: 20, the LCDR2 comprises the amino acid sequence of SEQ ID NO: 21, and the LCDR3 comprises the amino acid sequence of SEQ ID NO: 22; or
c. in the heavy chain variable region, the HCDR1 comprises the amino acid sequence of SEQ ID NO: 29, the HCDR2 comprises the amino acid sequence of SEQ ID NO: 30, and the HCDR3 comprises the amino acid sequence of SEQ ID NO: 31, and in the light chain variable region, the LCDR1 comprises the amino acid sequence of SEQ ID NO: 32, the LCDR2 comprises the amino acid sequence of SEQ ID NO: 73 or 33, and the LCDR3 comprises the amino acid sequence of SEQ ID NO: 34; or
d. in the heavy chain variable region, the HCDR1 comprises the amino acid sequence of SEQ ID NO: 35, the HCDR2 comprises the amino acid sequence of SEQ ID NO: 75 or 36, and the HCDR3 comprises the amino acid sequence of SEQ ID NO: 37, and in the light chain variable region, the LCDR1 comprises the amino acid sequence of SEQ ID NO: 38, the LCDR2 comprises the amino acid sequence of SEQ ID NO: 21, and the LCDR3 comprises the amino acid sequence of SEQ ID NO: 39; or
e. in the heavy chain variable region, the HCDR1 comprises the amino acid sequence of SEQ ID NO: 23, the HCDR2 comprises the amino acid sequence of SEQ ID NO: 57 or 24, and the HCDR3 comprises the amino acid sequence of SEQ ID NO: 25, and in the light chain variable region, the LCDR1 comprises the amino acid sequence of SEQ ID NO: 26, the LCDR2 comprises the amino acid sequence of SEQ ID NO: 27, and the LCDR3 comprises the amino acid sequence of SEQ ID NO: 28.

In some embodiments, provided is the anti-NPR1 antibody according to any one of the above, wherein:
a. in the heavy chain variable region, the HCDR1 comprises the amino acid sequence of SEQ ID NO: 29, the HCDR2 comprises the amino acid sequence of SEQ ID NO: 40, and the HCDR3 comprises the amino acid sequence of SEQ ID NO: 41, and in the light chain variable region, the LCDR1 comprises the amino acid sequence of SEQ ID NO: 42, the LCDR2 comprises the amino acid sequence of SEQ ID NO: 33, and the LCDR3 comprises the amino acid sequence of SEQ ID NO: 34; or
b. in the heavy chain variable region, the HCDR1 comprises the amino acid sequence of SEQ ID NO: 45, the HCDR2 comprises the amino acid sequence of SEQ ID NO: 46, and the HCDR3 comprises the amino acid sequence of SEQ ID NO: 19, and in the light chain variable region, the LCDR1 comprises the amino acid sequence of SEQ ID NO: 20, the LCDR2 comprises the amino acid sequence of SEQ ID NO: 21, and the LCDR3 comprises the amino acid sequence of SEQ ID NO: 22; or
   in the heavy chain variable region, the HCDR1 comprises the amino acid sequence of SEQ ID NO: 17 or 35, the HCDR2 comprises the amino acid sequence of SEQ ID NO: 18, and the HCDR3 comprises the amino acid sequence of SEQ ID NO: 19, and in the light chain variable region, the LCDR1 comprises the amino acid sequence of SEQ ID NO: 20, the LCDR2 comprises the amino acid sequence of SEQ ID NO: 21, and the LCDR3 comprises the amino acid sequence of SEQ ID NO: 22; or
c. in the heavy chain variable region, the HCDR1 comprises the amino acid sequence of SEQ ID NO: 29, the HCDR2 comprises the amino acid sequence of SEQ ID NO: 30, and the HCDR3 comprises the amino acid sequence of SEQ ID NO: 31, and in the light chain variable region, the LCDR1 comprises the amino acid sequence of SEQ ID NO: 32, the LCDR2 comprises the amino acid sequence of SEQ ID NO: 73 or 33, and the LCDR3 comprises the amino acid sequence of SEQ ID NO: 34; or
d. in the heavy chain variable region, the HCDR1 comprises the amino acid sequence of SEQ ID NO: 35, the HCDR2 comprises the amino acid sequence of SEQ ID NO: 75 or 36, and the HCDR3 comprises the amino acid sequence of SEQ ID NO: 37, and in the light chain variable region, the LCDR1 comprises the amino acid sequence of SEQ ID NO: 38, the LCDR2 comprises the amino acid sequence of SEQ ID NO: 21, and the LCDR3 comprises the amino acid sequence of SEQ ID NO: 39; or
e. in the heavy chain variable region, the HCDR1 comprises the amino acid sequence of SEQ ID NO: 23, the HCDR2 comprises the amino acid sequence of SEQ ID NO: 57 or 24, and the HCDR3 comprises the amino acid sequence of SEQ ID NO: 25, and in the light chain variable region, the LCDR1 comprises the amino acid sequence of SEQ ID NO: 26, the LCDR2 comprises the amino acid sequence of SEQ ID NO: 27, and the LCDR3 comprises the amino acid sequence of SEQ ID NO: 28.

In some embodiments, provided is the anti-NPR1 antibody according to any one of the above, wherein:
a. in the heavy chain variable region, the HCDR1 comprises the amino acid sequence of SEQ ID NO: 29, the HCDR2 comprises the amino acid sequence of SEQ ID NO: 40, and the HCDR3 comprises the amino acid sequence of SEQ ID NO: 41, and in the light chain variable region, the LCDR1 comprises the amino acid sequence of SEQ ID NO: 42, the LCDR2 comprises the amino acid sequence of SEQ ID NO: 33, and the LCDR3 comprises the amino acid sequence of SEQ ID NO: 34; or
b. in the heavy chain variable region, the HCDR1 comprises the amino acid sequence of SEQ ID NO: 45, the HCDR2 comprises the amino acid sequence of SEQ ID NO: 46, and the HCDR3 comprises the amino acid sequence of SEQ ID NO: 19, and in the light chain variable region, the LCDR1 comprises the amino acid sequence of SEQ ID NO: 20, the LCDR2 comprises the amino acid sequence of SEQ ID NO: 21, and the LCDR3 comprises the amino acid sequence of SEQ ID NO: 22; or
c. in the heavy chain variable region, the HCDR1 comprises the amino acid sequence of SEQ ID NO: 29, the HCDR2 comprises the amino acid sequence of SEQ ID NO: 30, and the HCDR3 comprises the amino acid sequence of SEQ ID NO: 31, and in the light chain variable region, the LCDR1 comprises the amino acid sequence of SEQ ID NO: 32, the LCDR2 comprises the amino acid sequence of SEQ ID NO: 73, and the LCDR3 comprises the amino acid sequence of SEQ ID NO: 34; or
d. in the heavy chain variable region, the HCDR1 comprises the amino acid sequence of SEQ ID NO: 35, the HCDR2 comprises the amino acid sequence of SEQ ID NO: 75, and the HCDR3 comprises the amino acid sequence of SEQ ID NO: 37, and in the light chain variable region, the LCDR1 comprises the amino acid sequence of SEQ ID NO: 38, the LCDR2 comprises the amino acid sequence of SEQ ID NO: 21, and the LCDR3 comprises the amino acid sequence of SEQ ID NO: 39; or
e. in the heavy chain variable region, the HCDR1 comprises the amino acid sequence of SEQ ID NO: 23, the HCDR2 comprises the amino acid sequence of SEQ ID NO: 57, and the HCDR3 comprises the amino acid sequence of SEQ ID NO: 25, and in the light chain variable region, the LCDR1 comprises the amino acid sequence of SEQ ID NO: 26, the LCDR2 comprises the amino acid sequence of SEQ ID NO: 27, and the LCDR3 comprises the amino acid sequence of SEQ ID NO: 28.

In some embodiments, provided is the anti-NPR1 antibody according to any one of the above, wherein:
a. in the heavy chain variable region, the HCDR1 comprises the amino acid sequence of SEQ ID NO: 29, the HCDR2 comprises the amino acid sequence of SEQ ID NO: 40, and the HCDR3 comprises the amino acid sequence of SEQ ID NO: 41, and in the light chain variable region, the LCDR1 comprises the amino acid sequence of SEQ ID NO: 42, the LCDR2 comprises the amino acid sequence of SEQ ID NO: 33, and the LCDR3 comprises the amino acid sequence of SEQ ID NO: 34; or
b. in the heavy chain variable region, the HCDR1 comprises the amino acid sequence of SEQ ID NO: 17, the HCDR2 comprises the amino acid sequence of SEQ ID NO: 18, and the HCDR3 comprises the amino acid sequence of SEQ ID NO: 19, and in the light chain variable region, the LCDR1 comprises the amino acid sequence of SEQ ID NO: 20, the LCDR2 comprises the amino acid sequence of SEQ ID NO: 21, and the LCDR3 comprises the amino acid sequence of SEQ ID NO: 22; or
c. in the heavy chain variable region, the HCDR1 comprises the amino acid sequence of SEQ ID NO: 29, the HCDR2 comprises the amino acid sequence of SEQ ID NO: 30, and the HCDR3 comprises the amino acid sequence of SEQ ID NO: 31, and in the light chain variable region, the LCDR1 comprises the amino acid sequence of SEQ ID NO: 32, the LCDR2 comprises the amino acid sequence of SEQ ID NO: 33, and the LCDR3 comprises the amino acid sequence of SEQ ID NO: 34; or
d. in the heavy chain variable region, the HCDR1 comprises the amino acid sequence of SEQ ID NO: 35, the HCDR2 comprises the amino acid sequence of SEQ ID NO: 36, and the HCDR3 comprises the amino acid sequence of SEQ ID NO: 37, and in the light chain variable region, the LCDR1 comprises the amino acid sequence of SEQ ID NO: 38, the LCDR2 comprises the amino acid sequence of SEQ ID NO: 21, and the LCDR3 comprises the amino acid sequence of SEQ ID NO: 39; or
e. in the heavy chain variable region, the HCDR1 comprises the amino acid sequence of SEQ ID NO: 23, the HCDR2 comprises the amino acid sequence of SEQ ID NO: 24, and the HCDR3 comprises the amino acid sequence of SEQ ID NO: 25, and in the light chain variable region, the LCDR1 comprises the amino acid sequence of SEQ ID NO: 26, the LCDR2 comprises the amino acid sequence of SEQ ID NO: 27, and the LCDR3 comprises the amino acid sequence of SEQ ID NO: 28.

In some embodiments, provided is the anti-NPR1 antibody according to any one of the above, wherein the HCDR1, the HCDR2, and the HCDR3 of the heavy chain variable region and the LCDR1, the LCDR2, and the LCDR3 of the light chain variable region are defined according to the Kabat numbering scheme.

In some embodiments, provided is the anti-NPR1 antibody according to any one of the above, wherein the anti-NPR1 antibody is a murine antibody, a chimeric antibody, a humanized antibody, or a fully human-derived antibody. In some embodiments, the antibody is a chimeric antibody. In some embodiments, the antibody is a humanized antibody.

In some embodiments, the anti-NPR1 antibody according to any one of the above comprises human antibody framework regions (FRs).

In some embodiments, provided is the anti-NPR1 antibody according to any one of the above, wherein the heavy chain variable region comprises a FR1, a FR2, and a FR3 that are derived from IGHV1-46*01 and a FR4 derived from IGHJ6*01, and the FRs are unsubstituted or comprise one or more amino acid substitutions selected from the group consisting of 1E, 40R, 43R, 48I, 69L, 71V, 73K, 74P, 75S, 77P, 78A, 80I, and 81Q; and/or the light chain variable region comprises a FR1, a FR2, and a FR3 that are derived from IGKV3-11*01 and a FR4 derived from IGKJ2*01, and the FRs are unsubstituted or comprise one or more amino acid substitutions selected from the group consisting of 20Q, 58V, 60D, 70S, 71Y, 77R, and 100A. In some embodiments, provided is the anti-NPR1 antibody, wherein in the heavy chain variable region, the HCDR1 comprises the amino acid sequence of SEQ ID NO: 29, the HCDR2 comprises the amino acid sequence of SEQ ID NO: 40, and the HCDR3 comprises the amino acid sequence of SEQ ID NO: 41, and the FRs of the heavy chain variable region are unsubstituted or comprise one or more amino acid substitutions selected from the group consisting of 1E, 40R, 43R, 48I, 69L, 71V, 73K, 74P, 75S, 77P, 78A, 80I, and 81Q; and in the light chain variable region, the LCDR1 comprises the amino acid sequence of SEQ ID NO: 42, the LCDR2 comprises the amino acid sequence of SEQ ID NO: 33, and the LCDR3 comprises the amino acid sequence of SEQ ID NO: 34, and the FRs of the light chain variable region are unsubstituted or comprise one or more amino acid substitutions selected from the group consisting of 20Q, 58V, 60D, 70S, 71Y, 77R, and 100A. In some embodiments, the variable regions and CDRs described above are defined according to the Kabat numbering scheme.

In some embodiments, provided is the anti-NPR1 antibody according to any one of the above, wherein the heavy chain variable region comprises a FR1, a FR2, and a FR3 that are derived from IGHV3-21*01 and a FR4 derived from IGHJ6*01, and the FRs are unsubstituted or comprise one or more amino acid substitutions selected from the group consisting of 1D, 3K, 9E, 44R, 49A, 87S, and 93T; and/or the light chain variable region comprises a FR1, a FR2, and a FR3 that are derived from IGKV3-11*01 or IGKV1-39*01 and a FR4 derived from IGKJ4*01, and the FRs are unsubstituted or comprise one or more amino acid substitutions selected from the group consisting of 4L, 43S, 45K, 46R, 47W, 58V, and 71Y. In some embodiments, provided is the anti-NPR1 antibody, wherein in the heavy chain variable region, the HCDR1 comprises the amino acid sequence of SEQ ID NO: 45, 17, or 35, the HCDR2 comprises the amino acid sequence of SEQ ID NO: 46, and the HCDR3 comprises the amino acid sequence of SEQ ID NO: 19, and the FRs of the heavy chain variable region are unsubstituted or comprise one or more amino acid substitutions selected from the group consisting of 1D, 3K, 9E, 44R, 49A, 87S, and 93T; and in the light chain variable region, the LCDR1 comprises the amino acid sequence of SEQ ID NO: 20, the LCDR2 comprises the amino acid sequence of SEQ ID NO: 21, and the LCDR3 comprises the amino acid sequence of SEQ ID NO: 22, and the FRs of the light chain variable region are unsubstituted or comprise one or more amino acid substitutions selected from the group consisting of 4L, 43S, 45K, 46R, 47W, 58V, and 71Y. In some embodiments, provided is the anti-NPR1 antibody, wherein in the heavy chain variable region, the HCDR1 comprises the amino acid sequence of SEQ ID NO: 45, 17, or 35, the HCDR2 comprises the amino acid sequence of SEQ ID NO: 18, and the HCDR3 comprises the amino acid sequence of SEQ ID NO: 19, and the FRs of the heavy chain variable region are unsubstituted or comprise one or more amino acid substitutions selected from the group consisting of 1D, 3K, 9E, 44R, 49A, 87S, and 93T; and in the light chain variable region, the LCDR1 comprises the amino acid sequence of SEQ ID NO: 20, the LCDR2 comprises the amino acid sequence of SEQ ID NO: 21, and the LCDR3 comprises the amino acid sequence of SEQ ID NO: 22, and the FRs of the light chain variable region are unsubstituted or comprise one or more amino acid substitutions selected from the group consisting of 4L, 43S, 45K, 46R, 47W, 58V, and 71Y. In some embodiments, the variable regions and CDRs described above are defined according to the Kabat numbering scheme.

In some embodiments, provided is the anti-NPR1 antibody according to any one of the above, wherein the heavy chain variable region comprises a FR1, a FR2, and a FR3 that are derived from IGHV1-46*01 and a FR4 derived from IGHJ6*01, and the FRs are unsubstituted or comprise one or more amino acid substitutions selected from the group consisting of 1E, 40R, 43R, 69L, 71V, 73K, 74P, 75S, 77P, and 78A; and/or the light chain variable region comprises a FR1, a FR2, and a FR3 that are derived from IGKV3-20*02 or 6-21*02 and a FR4 derived from IGKJ2*01, and the FRs are unsubstituted or comprise one or more amino acid substitutions selected from the group consisting of22K, 42S, 43S, 49Y, 57R, and 71Y. In some embodiments, provided is the anti-NPR1 antibody, wherein in the heavy chain variable region, the HCDR1 comprises the amino acid sequence of SEQ ID NO: 29, the HCDR2 comprises the amino acid sequence of SEQ ID NO: 30, and the HCDR3 comprises the amino acid sequence of SEQ ID NO: 31, and the FRs of the heavy chain variable region are unsubstituted or comprise one or more amino acid substitutions selected from the group consisting of 1E, 40R, 43R, 69L, 71V, 73K, 74P, 75S, 77P, and 78A; and in the light chain variable region, the LCDR1 comprises the amino acid sequence of SEQ ID NO: 32, the LCDR2 comprises the amino acid sequence of SEQ ID NO: 73 or 33, and the LCDR3 comprises the amino acid sequence of SEQ ID NO: 34, and the FRs of the light chain variable region are unsubstituted or comprise one or more amino acid substitutions selected from the group consisting of 22K, 42S, 43S, 49Y, 57R, and 71Y. In some embodiments, the variable regions and CDRs described above are defined according to the Kabat numbering scheme.

In some embodiments, provided is the anti-NPR1 antibody according to any one of the above, wherein the heavy chain variable region comprises a FR1, a FR2, and a FR3 that are derived from IGHV3-21*01 and a FR4 derived from IGHJ6*01, and the FRs are unsubstituted or comprise one or more amino acid substitutions selected from the group consisting of 1D, 3K, 9E, 28S, 29I, 44R, 49A, and 93T; and/or the light chain variable region comprises a FR1, a FR2, and a FR3 that are derived from IGKV1-39*01 and a FR4 derived from IGKJ4*01, and the FRs are unsubstituted or comprise one or more amino acid substitutions selected from the group consisting of 4L, 43S, 46R, 47W, and 71Y. In some embodiments, provided is the anti-NPR1 antibody, wherein in the heavy chain variable region, the HCDR1 comprises the amino acid sequence of SEQ ID NO: 35, the HCDR2 comprises the amino acid sequence of SEQ ID NO: 75 or 36, and the HCDR3 comprises the amino acid sequence of SEQ ID NO: 37, and the FRs of the heavy chain variable region are unsubstituted or comprise one or more amino acid substitutions selected from the group consisting of 1D, 3K, 9E, 28S, 29I, 44R, 49A, and 93T; and in the light chain variable region, the LCDR1 comprises the amino acid sequence of SEQ ID NO: 38, the LCDR2 comprises the amino acid sequence of SEQ ID NO: 21, and the LCDR3 comprises the amino acid sequence of SEQ ID NO: 39, and the FRs of the light chain variable region are unsubstituted or comprise one or more amino acid substitutions selected from the group consisting of 4L, 43S, 46R, 47W, and 71Y. In some embodiments, the variable regions and CDRs described above are defined according to the Kabat numbering scheme.

In some embodiments, provided is the anti-NPR1 antibody according to any one of the above, wherein the heavy chain variable region comprises a FR1, a FR2, and a FR3 that are derived from IGHV1-46*01 and a FR4 derived from IGHJ6*01, and the FRs are unsubstituted or comprise one or more amino acid substitutions selected from the group consisting of 1E, 2A, 3Y, 28S, 43K, 69L, 71V, and 73K; and/or the light chain variable region comprises a FR1, a FR2, and a FR3 that are derived from IGKV4-1*01 or 1-39*01 and a FR4 derived from IGKJ2*01, and the FRs are unsubstituted or comprise one or more amino acid substitutions selected from the group consisting of 1N, 4L, 17Q, 43P, 46V, 60A, 68R, and 79E. In some embodiments, provided is the anti-NPR1 antibody, wherein in the heavy chain variable region, the HCDR1 comprises the amino acid sequence of SEQ ID NO: 23, the HCDR2 comprises the amino acid sequence of SEQ ID NO: 57 or 24, and the HCDR3 comprises the amino acid sequence of SEQ ID NO: 25, and the FRs of the heavy chain variable region are unsubstituted or comprise one or more amino acid substitutions selected from the group consisting of 1E, 2A, 3Y, 28S, 43K, 69L, 71V, and 73K; and in the light chain variable region, the LCDR1 comprises the amino acid sequence of SEQ ID NO: 26, the LCDR2 comprises the amino acid sequence of SEQ ID NO: 27, and the LCDR3 comprises the amino acid sequence of SEQ ID NO: 28, and the FRs of the light chain variable region are unsubstituted or comprise one or more amino acid substitutions selected from the group consisting of 1N, 4L, 17Q, 43P, 46V, 60A, 68R, and 79E. In some embodiments, the variable regions and CDRs described above are defined according to the Kabat numbering scheme.

In some embodiments, provided is the anti-NPR1 antibody according to any one of the above, wherein:
a. the heavy chain variable region comprises SEQ ID NO: 82, 80, or 81 or an amino acid sequence having at least 85% (e.g., at least 85%, 87%, 90%, 93%, 94%, 95%, 96%, 97%, 98%, or 99%) sequence identity thereto, and the light chain variable region comprises SEQ ID NO: 85, 83, or 84 or an amino acid sequence having at least 85% (e.g., at least 85%, 87%, 90%, 93%, 94%, 95%, 96%, 97%, 98%, or 99%) sequence identity thereto; or
b. the heavy chain variable region comprises SEQ ID NO: 51, 48, 49, 50, or 52 or an amino acid sequence having at least 85% (e.g., at least 85%, 87%, 90%, 93%, 94%, 95%, 96%, 97%, 98%, or 99%) sequence identity thereto, and the light chain variable region comprises SEQ ID NO: 56, 53, 54, or 55 or an amino acid sequence having at least 85% (e.g., at least 85%, 87%, 90%, 93%, 94%, 95%, 96%, 97%, 98%, or 99%) sequence identity thereto; or
c. the heavy chain variable region comprises SEQ ID NO: 68, 66, 67, or 69 or an amino acid sequence having at least 85% (e.g., at least 85%, 87%, 90%, 93%, 94%, 95%, 96%, 97%, 98%, or 99%) sequence identity thereto, and the light chain variable region comprises SEQ ID NO: 74, 70, 71, or 72 or an amino acid sequence having at least 85% (e.g., at least 85%, 87%, 90%, 93%, 94%, 95%, 96%, 97%, 98%, or 99%) sequence identity thereto; or
d. the heavy chain variable region comprises SEQ ID NO: 78, 76, or 77 or an amino acid sequence having at least 85% (e.g., at least 85%, 87%, 90%, 93%, 94%, 95%, 96%, 97%, 98%, or 99%) sequence identity thereto, and the light chain variable region comprises SEQ ID NO: 79 or an amino acid sequence having at least 85% (e.g., at least 85%, 87%, 90%, 93%, 94%, 95%, 96%, 97%, 98%, or 99%) sequence identity thereto; or
e. the heavy chain variable region comprises SEQ ID NO: 58, 59, or 60 or an amino acid sequence having at least 85% (e.g., at least 85%, 87%, 90%, 93%, 94%, 95%, 96%, 97%, 98%, or 99%) sequence identity thereto, and the light chain variable region comprises SEQ ID NO: 61, 62, 63, 64, or 65 or an amino acid sequence having at least 85% (e.g., at least 85%, 87%, 90%, 93%, 94%, 95%, 96%, 97%, 98%, or 99%) sequence identity thereto; or
f. the heavy chain variable region comprises SEQ ID NO: 15 or an amino acid sequence having at least 80% (e.g., at least 81%, 83%, 85%, 87%, 90%, 93%, 94%, 95%, 96%, 97%, 98%, or 99%) sequence identity thereto, and the light chain variable region comprises SEQ ID NO: 16 or an amino acid sequence having at least 80% (e.g., at least 81%, 83%, 85%, 87%, 90%, 93%, 94%, 95%, 96%, 97%, 98%, or 99%) sequence identity thereto; or
g. the heavy chain variable region comprises SEQ ID NO: 7 or an amino acid sequence having at least 80% (e.g., at least 81%, 83%, 85%, 87%, 90%, 93%, 94%, 95%, 96%, 97%, 98%, or 99%) sequence identity thereto, and the light chain variable region comprises SEQ ID NO: 8 or an amino acid sequence having at least 80% (e.g., at least 81%, 83%, 85%, 87%, 90%, 93%, 94%, 95%, 96%, 97%, 98%, or 99%) sequence identity thereto; or
h. the heavy chain variable region comprises SEQ ID NO: 11 or an amino acid sequence having at least 80% (e.g., at least 81%, 83%, 85%, 87%, 90%, 93%, 94%, 95%, 96%, 97%, 98%, or 99%) sequence identity thereto, and the light chain variable region comprises SEQ ID NO: 12 or an amino acid sequence having at least 80% (e.g., at least 81%, 83%, 85%, 87%, 90%, 93%, 94%, 95%, 96%, 97%, 98%, or 99%) sequence identity thereto; or
i. the heavy chain variable region comprises SEQ ID NO: 13 or an amino acid sequence having at least 80% (e.g., at least 81%, 83%, 85%, 87%, 90%, 93%, 94%, 95%, 96%, 97%, 98%, or 99%) sequence identity thereto, and the light chain variable region comprises SEQ ID NO: 14 or an amino acid sequence having at least 80% (e.g., at least 81%, 83%, 85%, 87%, 90%, 93%, 94%, 95%, 96%, 97%, 98%, or 99%) sequence identity thereto; or
j. the heavy chain variable region comprises SEQ ID NO: 9 or an amino acid sequence having at least 80% (e.g., at least 81%, 83%, 85%, 87%, 90%, 93%, 94%, 95%, 96%, 97%, 98%, or 99%) sequence identity thereto, and the light chain variable region comprises SEQ ID NO: 10 or an amino acid sequence having at least 80% (e.g., at least 81%, 83%, 85%, 87%, 90%, 93%, 94%, 95%, 96%, 97%, 98%, or 99%) sequence identity thereto.

In some embodiments, provided is the anti-NPR1 antibody according to any one of the above, wherein:
a. the heavy chain variable region comprises SEQ ID NO: 82, 80, or 81 or an amino acid sequence having at least 85% (e.g., at least 85%, 87%, 90%, 93%, 94%, 95%, 96%, 97%, 98%, or 99%) sequence identity thereto, and the light chain variable region comprises SEQ ID NO: 85, 83, or 84 or an amino acid sequence having at least 85% (e.g., at least 85%, 87%, 90%, 93%, 94%, 95%, 96%, 97%, 98%, or 99%) sequence identity thereto; or
b. the heavy chain variable region comprises SEQ ID NO: 51, 48, 49, 50, or 52 or an amino acid sequence having at least 85% (e.g., at least 85%, 87%, 90%, 93%, 94%, 95%, 96%, 97%, 98%, or 99%) sequence identity thereto, and the light chain variable region comprises SEQ ID NO: 56, 53, 54, or 55 or an amino acid sequence having at least 85% (e.g., at least 85%, 87%, 90%, 93%, 94%, 95%, 96%, 97%, 98%, or 99%) sequence identity thereto; or
c. the heavy chain variable region comprises SEQ ID NO: 68, 66, 67, or 69 or an amino acid sequence having at least 85% (e.g., at least 85%, 87%, 90%, 93%, 94%, 95%, 96%, 97%, 98%, or 99%) sequence identity thereto, and the light chain variable region comprises SEQ ID NO: 74, 70, 71, or 72 or an amino acid sequence having at least 85% (e.g., at least 85%, 87%, 90%, 93%, 94%, 95%, 96%, 97%, 98%, or 99%) sequence identity thereto; or
d. the heavy chain variable region comprises SEQ ID NO: 78, 76, or 77 or an amino acid sequence having at least 85% (e.g., at least 85%, 87%, 90%, 93%, 94%, 95%, 96%, 97%, 98%, or 99%) sequence identity thereto, and the light chain variable region comprises SEQ ID NO: 79 or an amino acid sequence having at least 85% (e.g., at least 85%, 87%, 90%, 93%, 94%, 95%, 96%, 97%, 98%, or 99%) sequence identity thereto; or
e. the heavy chain variable region comprises SEQ ID NO: 58, 59, or 60 or an amino acid sequence having at least 85% (e.g., at least 85%, 87%, 90%, 93%, 94%, 95%, 96%, 97%, 98%, or 99%) sequence identity thereto, and the light chain variable region comprises SEQ ID NO: 61, 62, 63, 64, or 65 or an amino acid sequence having at least 85% (e.g., at least 85%, 87%, 90%, 93%, 94%, 95%, 96%, 97%, 98%, or 99%) sequence identity thereto.

In some embodiments, provided is the anti-NPR1 antibody according to any one of the above, wherein:
a. the heavy chain variable region comprises the amino acid sequence of SEQ ID NO: 82, 80, or 81, and the light chain variable region comprises the amino acid sequence of SEQ ID NO: 85, 83, or 84; or
b. the heavy chain variable region comprises the amino acid sequence of SEQ ID NO: 51, 48, 49, 50, or 52, and the light chain variable region comprises the amino acid sequence of SEQ ID NO: 56, 53, 54, or 55; or
c. the heavy chain variable region comprises the amino acid sequence of SEQ ID NO: 68, 66, 67, or 69, and the light chain variable region comprises the amino acid sequence of SEQ ID NO: 74, 70, 71, or 72; or
d. the heavy chain variable region comprises the amino acid sequence of SEQ ID NO: 78, 76, or 77, and the light chain variable region comprises the amino acid sequence of SEQ ID NO: 79; or
e. the heavy chain variable region comprises the amino acid sequence of SEQ ID NO: 58, 59, or 60, and the light chain variable region comprises the amino acid sequence of SEQ ID NO: 61, 62, 63, 64, or 65; or
f. the heavy chain variable region comprises the amino acid sequence of SEQ ID NO: 15, and the light chain variable region comprises the amino acid sequence of SEQ ID NO: 16; or
g. the heavy chain variable region comprises the amino acid sequence of SEQ ID NO: 7, and the light chain variable region comprises the amino acid sequence of SEQ ID NO: 8; or
h. the heavy chain variable region comprises the amino acid sequence of SEQ ID NO: 11, and the light chain variable region comprises the amino acid sequence of SEQ ID NO: 12; or
i. the heavy chain variable region comprises the amino acid sequence of SEQ ID NO: 13, and the light chain variable region comprises the amino acid sequence of SEQ ID NO: 14; or
j. the heavy chain variable region comprises the amino acid sequence of SEQ ID NO: 9, and the light chain variable region comprises the amino acid sequence of SEQ ID NO: 10.

In some embodiments, provided is the anti-NPR1 antibody according to any one of the above, wherein:
a. the heavy chain variable region comprises the amino acid sequence of SEQ ID NO: 82, 80, or 81, and the light chain variable region comprises the amino acid sequence of SEQ ID NO: 85, 83, or 84; or
b. the heavy chain variable region comprises the amino acid sequence of SEQ ID NO: 51, 48, 49, 50, or 52, and the light chain variable region comprises the amino acid sequence of SEQ ID NO: 56, 53, 54, or 55; or
c. the heavy chain variable region comprises the amino acid sequence of SEQ ID NO: 68, 66, 67, or 69, and the light chain variable region comprises the amino acid sequence of SEQ ID NO: 74, 70, 71, or 72; or
d. the heavy chain variable region comprises the amino acid sequence of SEQ ID NO: 78, 76, or 77, and the light chain variable region comprises the amino acid sequence of SEQ ID NO: 79; or
e. the heavy chain variable region comprises the amino acid sequence of SEQ ID NO: 58, 59, or 60, and the light chain variable region comprises the amino acid sequence of SEQ ID NO: 61, 62, 63, 64, or 65.

In some embodiments, provided is the anti-NPR1 antibody according to any one of the above, wherein:
a. the heavy chain variable region comprises the amino acid sequence of SEQ ID NO: 82, and the light chain variable region comprises the amino acid sequence of SEQ ID NO: 85; or
   the heavy chain variable region comprises the amino acid sequence of SEQ ID NO: 81 or 82, and the light chain variable region comprises the amino acid sequence of SEQ ID NO: 84; or
b. the heavy chain variable region comprises the amino acid sequence of SEQ ID NO: 51, 49, 50, or 52, and the light chain variable region comprises the amino acid sequence of SEQ ID NO: 56; or
c. the heavy chain variable region comprises the amino acid sequence of SEQ ID NO: 68, and the light chain variable region comprises the amino acid sequence of SEQ ID NO: 74, 70, or 71; or the heavy chain variable region comprises the amino acid sequence of SEQ ID NO: 67, and the light chain variable region comprises the amino acid sequence of SEQ ID NO: 70 or 71; or
d. the heavy chain variable region comprises the amino acid sequence of SEQ ID NO: 78, 76, or 77, and the light chain variable region comprises the amino acid sequence of SEQ ID NO: 79; or
e. the heavy chain variable region comprises the amino acid sequence of SEQ ID NO: 58, and the light chain variable region comprises the amino acid sequence of SEQ ID NO: 61, 62, 64, or 65; or
   the heavy chain variable region comprises the amino acid sequence of SEQ ID NO: 59, and the light chain variable region comprises the amino acid sequence of SEQ ID NO: 61, 62, 64, or 65; or
   the heavy chain variable region comprises the amino acid sequence of SEQ ID NO: 60, and the light chain variable region comprises the amino acid sequence of SEQ ID NO: 61, 62, 64, or 65; or
f. the heavy chain variable region comprises the amino acid sequence of SEQ ID NO: 15, and the light chain variable region comprises the amino acid sequence of SEQ ID NO: 16; or
g. the heavy chain variable region comprises the amino acid sequence of SEQ ID NO: 7, and the light chain variable region comprises the amino acid sequence of SEQ ID NO: 8; or
h. the heavy chain variable region comprises the amino acid sequence of SEQ ID NO: 11, and the light chain variable region comprises the amino acid sequence of SEQ ID NO: 12; or
i. the heavy chain variable region comprises the amino acid sequence of SEQ ID NO: 13, and the light chain variable region comprises the amino acid sequence of SEQ ID NO: 14; or
j. the heavy chain variable region comprises the amino acid sequence of SEQ ID NO: 9, and the light chain variable region comprises the amino acid sequence of SEQ ID NO: 10.

In some specific embodiments, provided is the anti-NPR1 antibody according to any one of the above, wherein the heavy chain variable region comprises the amino acid sequence of SEQ ID NO: 82, and the light chain variable region comprises the amino acid sequence of SEQ ID NO: 85.

In some specific embodiments, provided is the anti-NPR1 antibody according to any one of the above, wherein the heavy chain variable region comprises the amino acid sequence of SEQ ID NO: 51, and the light chain variable region comprises the amino acid sequence of SEQ ID NO: 56.

In some specific embodiments, provided is the anti-NPR1 antibody according to any one of the above, wherein the heavy chain variable region comprises the amino acid sequence of SEQ ID NO: 68, and the light chain variable region comprises the amino acid sequence of SEQ ID NO: 74.

In some specific embodiments, provided is the anti-NPR1 antibody according to any one of the above, wherein the heavy chain variable region comprises the amino acid sequence of SEQ ID NO: 78, and the light chain variable region comprises the amino acid sequence of SEQ ID NO: 79.

In some specific embodiments, provided is the anti-NPR1 antibody according to any one of the above, wherein the heavy chain variable region comprises the amino acid sequence of SEQ ID NO: 15, and the light chain variable region comprises the amino acid sequence of SEQ ID NO: 16.

In some specific embodiments, provided is the anti-NPR1 antibody according to any one of the above, wherein the heavy chain variable region comprises the amino acid sequence of SEQ ID NO: 7, and the light chain variable region comprises the amino acid sequence of SEQ ID NO: 8.

In some specific embodiments, provided is the anti-NPR1 antibody according to any one of the above, wherein the heavy chain variable region comprises the amino acid sequence of SEQ ID NO: 11, and the light chain variable region comprises the amino acid sequence of SEQ ID NO: 12.

In some specific embodiments, provided is the anti-NPR1 antibody according to any one of the above, wherein the heavy chain variable region comprises the amino acid sequence of SEQ ID NO: 13, and the light chain variable region comprises the amino acid sequence of SEQ ID NO: 14.

In some specific embodiments, provided is the anti-NPR1 antibody according to any one of the above, wherein the heavy chain variable region comprises the amino acid sequence of SEQ ID NO: 9, and the light chain variable region comprises the amino acid sequence of SEQ ID NO: 10.

In some embodiments, provided is the anti-NPR1 antibody according to any one of the above, wherein the anti-NPR1 antibody is an antibody fragment; in some embodiments, the antibody fragment is selected from the group consisting of Fab, Fab', F(ab')2, Fd, Fv, scFv, dsFv, and dAb. In some embodiments, provided is the anti-NPR1 antibody according to any one of the above, wherein the anti-NPR1 antibody comprises a heavy chain constant region and a light chain constant region; in some embodiments, the heavy chain constant region is a heavy chain constant region of human IgG1, IgG2, IgG3, IgG4, or a variant thereof; in some embodiments, the light chain constant region is a human κ or λ light chain constant region; in some embodiments, the heavy chain constant region comprises the amino acid sequence of SEQ ID NO: 86 or 43, and the light chain constant region comprises the amino acid sequence of SEQ ID NO: 44.

In some embodiments, provided is the anti-NPR1 antibody according to any one of the above, wherein the anti-NPR1 antibody comprises a heavy chain and a light chain, wherein:
a. the heavy chain comprises SEQ ID NO: 89 or 127 or an amino acid sequence having at least 85% (e.g., at least 85%, 87%, 90%, 93%, 94%, 95%, 96%, 97%, 98%, or 99%) sequence identity thereto, and the light chain comprises SEQ ID NO: 90 or 128 or an amino acid sequence having at least 85% (e.g., at least 85%, 87%, 90%, 93%, 94%, 95%, 96%, 97%, 98%, or 99%) sequence identity thereto; or
   the heavy chain comprises SEQ ID NO: 110 or an amino acid sequence having at least 85% (e.g., at least 85%, 87%, 90%, 93%, 94%, 95%, 96%, 97%, 98%, or 99%) sequence identity thereto, and the light chain comprises SEQ ID NO: 111 or an amino acid sequence having at least 85% (e.g., at least 85%, 87%, 90%, 93%, 94%, 95%, 96%, 97%, 98%, or 99%) sequence identity thereto; or
b. the heavy chain comprises an amino acid sequence having at least 85% (e.g., at least 85%, 86%, 87%, 88%, 89%, 90%, 91%, 92%, 93%, 94%, 95%, 96%, 97%, 98%, or 99%) sequence identity to SEQ ID NO: 87, 112, 113, or 114, and the light chain comprises SEQ ID NO: 88 or an amino acid sequence having at least 85% (e.g., at least 85%, 87%, 90%, 93%, 94%, 95%, 96%, 97%, 98%, or 99%) sequence identity thereto; or
   the heavy chain comprises SEQ ID NO: 102 or an amino acid sequence having at least 85% (e.g., at least 85%, 87%, 90%, 93%, 94%, 95%, 96%, 97%, 98%, or 99%) sequence identity thereto, and the light chain comprises SEQ ID NO: 103 or an amino acid sequence having at least 85% (e.g., at least 85%, 87%, 90%, 93%, 94%, 95%, 96%, 97%, 98%, or 99%) sequence identity thereto; or
c. the heavy chain comprises SEQ ID NO: 99 or 122 or an amino acid sequence having at least 85% (e.g., at least 85%, 87%, 90%, 93%, 94%, 95%, 96%, 97%, 98%, or 99%) sequence identity thereto, and the light chain comprises SEQ ID NO: 100, 123, or 124 or an amino acid sequence having at least 85% (e.g., at least 85%, 87%, 90%, 93%, 94%, 95%, 96%, 97%, 98%, or 99%) sequence identity thereto; or
   the heavy chain comprises SEQ ID NO: 106 or an amino acid sequence having at least 85% (e.g., at least 85%, 87%, 90%, 93%, 94%, 95%, 96%, 97%, 98%, or 99%) sequence identity thereto, and the light chain comprises SEQ ID NO: 107 or an amino acid sequence having at least 85% (e.g., at least 85%, 87%, 90%, 93%, 94%, 95%, 96%, 97%, 98%, or 99%) sequence identity thereto; or
d. the heavy chain comprises SEQ ID NO: 101, 125, or 126 or an amino acid sequence having at least 85% (e.g., at least 85%, 87%, 90%, 93%, 94%, 95%, 96%, 97%, 98%, or 99%) sequence identity thereto, and the light chain comprises SEQ ID NO: 47 or an amino acid sequence having at least 85% (e.g., at least 85%, 87%, 90%, 93%, 94%, 95%, 96%, 97%, 98%, or 99%) sequence identity thereto; or
   the heavy chain comprises SEQ ID NO: 108 or an amino acid sequence having at least 85% (e.g., at least 85%, 87%, 90%, 93%, 94%, 95%, 96%, 97%, 98%, or 99%) sequence identity thereto, and the light chain comprises SEQ ID NO: 109 or an amino acid sequence having at least 85% (e.g., at least 85%, 87%, 90%, 93%, 94%, 95%, 96%, 97%, 98%, or 99%) sequence identity thereto; or
e. the heavy chain comprises SEQ ID NO: 115, 116, or 117 or an amino acid sequence having at least 85% (e.g., at least 85%, 87%, 90%, 93%, 94%, 95%, 96%, 97%, 98%, or 99%) sequence identity thereto, and the light chain comprises SEQ ID NO: 118, 119, 120, or 121 or an amino acid sequence having at least 85% (e.g., at least 85%, 87%, 90%, 93%, 94%, 95%, 96%, 97%, 98%, or 99%) sequence identity thereto; or
   the heavy chain comprises SEQ ID NO: 104 or an amino acid sequence having at least 85% (e.g., at least 85%, 87%, 90%, 93%, 94%, 95%, 96%, 97%, 98%, or 99%) sequence identity thereto, and the light chain comprises SEQ ID NO: 105 or an amino acid sequence having at least 85% (e.g., at least 85%, 87%, 90%, 93%, 94%, 95%, 96%, 97%, 98%, or 99%) sequence identity thereto.

In some embodiments, provided is the anti-NPR1 antibody according to any one of the above, wherein the anti-NPR1 antibody comprises a heavy chain and a light chain, wherein:
a. the heavy chain comprises the amino acid sequence of SEQ ID NO: 89, and the light chain comprises the amino acid sequence of SEQ ID NO: 90 or 128; or
   the heavy chain comprises the amino acid sequence of SEQ ID NO: 127, and the light chain comprises the amino acid sequence of SEQ ID NO: 128; or
   the heavy chain comprises the amino acid sequence of SEQ ID NO: 110, and the light chain comprises the amino acid sequence of SEQ ID NO: 111; or
b. the heavy chain comprises the amino acid sequence of SEQ ID NO: 87, 112, 113, or 114, and the light chain comprises the amino acid sequence of SEQ ID NO: 88; or
   the heavy chain comprises the amino acid sequence of SEQ ID NO: 102, and the light chain comprises the amino acid sequence of SEQ ID NO: 103; or
c. the heavy chain comprises the amino acid sequence of SEQ ID NO: 99, and the light chain comprises the amino acid sequence of SEQ ID NO: 100, 123, or 124; or
   the heavy chain comprises the amino acid sequence of SEQ ID NO: 122, and the light chain comprises the amino acid sequence of SEQ ID NO: 123 or 124; or
   the heavy chain comprises the amino acid sequence of SEQ ID NO: 106, and the light chain comprises the amino acid sequence of SEQ ID NO: 107; or
d. the heavy chain comprises the amino acid sequence of SEQ ID NO: 101, 125, or 126, and the light chain comprises the amino acid sequence of SEQ ID NO: 47; or
   the heavy chain comprises the amino acid sequence of SEQ ID NO: 108, and the light chain comprises the amino acid sequence of SEQ ID NO: 109; or
e. the heavy chain comprises the amino acid sequence of SEQ ID NO: 115, and the light chain comprises the amino acid sequence of SEQ ID NO: 118, 119, 120, or 121; or
   the heavy chain comprises the amino acid sequence of SEQ ID NO: 116, and the light chain comprises the amino acid sequence of SEQ ID NO: 118, 119, 120, or 121; or
   the heavy chain comprises the amino acid sequence of SEQ ID NO: 117, and the light chain comprises the amino acid sequence of SEQ ID NO: 118, 119, 120, or 121; or
   the heavy chain comprises the amino acid sequence of SEQ ID NO: 104, and the light chain comprises the amino acid sequence of SEQ ID NO: 105.

In some embodiments, provided is the anti-NPR1 antibody according to any one of the above, wherein the anti-NPR1 antibody comprises a heavy chain and a light chain, wherein:
a. the heavy chain comprises SEQ ID NO: 89 or an amino acid sequence having at least 85% (e.g., at least 85%, 87%, 90%, 93%, 94%, 95%, 96%, 97%, 98%, or 99%) sequence identity thereto, and the light chain comprises SEQ ID NO: 90 or an amino acid sequence having at least 85% (e.g., at least 85%, 87%, 90%, 93%, 94%, 95%, 96%, 97%, 98%, or 99%) sequence identity thereto; or
b. the heavy chain comprises an amino acid sequence having at least 85% (e.g., at least 85%, 86%, 87%, 88%, 89%, 90%, 91%, 92%, 93%, 94%, 95%, 96%, 97%, 98%, or 99%) sequence identity to SEQ ID NO: 87, and the light chain comprises SEQ ID NO: 88 or an amino acid sequence having at least 85% (e.g., at least 85%, 87%, 90%, 93%, 94%, 95%, 96%, 97%, 98%, or 99%) sequence identity thereto; or
c. the heavy chain comprises SEQ ID NO: 99 or an amino acid sequence having at least 85% (e.g., at least 85%, 87%, 90%, 93%, 94%, 95%, 96%, 97%, 98%, or 99%) sequence identity thereto, and the light chain comprises SEQ ID NO: 100 or an amino acid sequence having at least 85% (e.g., at least 85%, 87%, 90%, 93%, 94%, 95%, 96%, 97%, 98%, or 99%) sequence identity thereto; or
d. the heavy chain comprises SEQ ID NO: 101 or an amino acid sequence having at least 85% (e.g., at least 85%, 87%, 90%, 93%, 94%, 95%, 96%, 97%, 98%, or 99%) sequence identity thereto, and the light chain comprises SEQ ID NO: 47 or an amino acid sequence having at least 85% (e.g., at least 85%, 87%, 90%, 93%, 94%, 95%, 96%, 97%, 98%, or 99%) sequence identity thereto.

In some embodiments, provided is the anti-NPR1 antibody according to any one of the above, wherein the anti-NPR1 antibody comprises a heavy chain and a light chain, wherein:
a. the heavy chain comprises the amino acid sequence of SEQ ID NO: 89, and the light chain comprises the amino acid sequence of SEQ ID NO: 90; or
b. the heavy chain comprises the amino acid sequence of SEQ ID NO: 87, and the light chain comprises the amino acid sequence of SEQ ID NO: 88; or
c. the heavy chain comprises the amino acid sequence of SEQ ID NO: 99, and the light chain comprises the amino acid sequence of SEQ ID NO: 100; or
d. the heavy chain comprises the amino acid sequence of SEQ ID NO: 101, and the light chain comprises the amino acid sequence of SEQ ID NO: 47.

In some specific embodiments, provided is the anti-NPR1 antibody according to any one of the above, wherein the anti-NPR1 antibody comprises a heavy chain and a light chain, wherein the heavy chain comprises the amino acid sequence of SEQ ID NO: 89, and the light chain comprises the amino acid sequence of SEQ ID NO: 90.

In some specific embodiments, provided is the anti-NPR1 antibody according to any one of the above, wherein the anti-NPR1 antibody comprises a heavy chain and a light chain, wherein the heavy chain comprises the amino acid sequence of SEQ ID NO: 87, and the light chain comprises the amino acid sequence of SEQ ID NO: 88.

In some specific embodiments, provided is the anti-NPR1 antibody according to any one of the above, wherein the anti-NPR1 antibody comprises a heavy chain and a light chain, wherein the amino acid sequence of the heavy chain is set forth in SEQ ID NO: 89, and the amino acid sequence of the light chain is set forth in SEQ ID NO: 90.

In some specific embodiments, provided is the anti-NPR1 antibody according to any one of the above, wherein the anti-NPR1 antibody comprises a heavy chain and a light chain, wherein the amino acid sequence of the heavy chain is set forth in SEQ ID NO: 87, and the amino acid sequence of the light chain is set forth in SEQ ID NO: 88.

In some specific embodiments, provided is the anti-NPR1 antibody according to any one of the above, wherein the anti-NPR1 antibody comprises a heavy chain and a light chain, wherein the amino acid sequence of the heavy chain is set forth in SEQ ID NO: 99, and the amino acid sequence of the light chain is set forth in SEQ ID NO: 100.

In some specific embodiments, provided is the anti-NPR1 antibody according to any one of the above, wherein the anti-NPR1 antibody comprises a heavy chain and a light chain, wherein the amino acid sequence of the heavy chain is set forth in SEQ ID NO: 101, and the amino acid sequence of the light chain is set forth in SEQ ID NO: 47.

In some embodiments, the present disclosure further provides an isolated anti-NPR1 antibody that competes for binding to human NPR1 or an epitope thereof with the anti-NPR1 antibody according to any one of the foregoing.

In some embodiments, the anti-NPR1 antibody provided by the present disclosure specifically binds to hNPR1 and does not bind to the other NPR family members, such as hNPR2 and/or hNPR3.

In some embodiments, the isolated anti-NPR1 antibody of the present disclosure binds to human NPR1 with an EC₅₀ value of less than 30 nM (e.g., less than 27 nM, less than 10 nM, less than 9 nM, less than 7 nM, less than 5 nM, less than 4 nM, less than 2 nM, less than 1.5 nM, less than 0.1 nM, or less than 0.05 nM), wherein the EC₅₀ value is measured by ELISA.

In some embodiments, the isolated anti-NPR1 antibody of the present disclosure is capable of binding to hNPR1 CHO-K1 and cNPR1 CHO-K1, with or without 10 nM ANP.

In some embodiments, the isolated anti-NPR1 antibody of the present disclosure can stimulate cGMP production in hNPR1-CHOK1 cells.

In some embodiments, the isolated anti-NPR1 antibody of the present disclosure has cGMP production with a smaller difference on cells after endocytosis occurs than a positive antibody does.

In another aspect, the present disclosure provides a pharmaceutical composition comprising the anti-NPR1 antibody according to any one of the above and one or more pharmaceutically acceptable carriers, diluents, or excipients; in some embodiments, the pharmaceutical composition further comprises at least one second therapeutic agent.

In some embodiments, the pharmaceutical composition is administered subcutaneously, intravenously, intradermally, intraperitoneally, or intramuscularly.

In another aspect, the present disclosure further provides an immunoconjugate comprising the anti-NPR1 antibody according to any one of the foregoing and an effector molecule, wherein the effector molecule is conjugated to the anti-NPR1 antibody; preferably, the effector molecule is selected from the group consisting of an anti-tumor agent, an immunomodulator, a biological response modifier, a lectin, a cytotoxic drug, a chromophore, a fluorophore, a chemiluminescent compound, an enzyme, a metal ion, and any combination thereof.

In another aspect, the present disclosure provides an isolated nucleic acid encoding the anti-NPR1 antibody according to any one of the above.

In another aspect, the present disclosure provides a vector comprising the isolated nucleic acid according to any one of the above.

In another aspect, the present disclosure provides a host cell comprising the isolated nucleic acid according to any one of the above.

In another aspect, the present disclosure provides a method for detecting an NPR1 peptide or a fragment thereof in a sample, and the method comprises contacting the sample with the anti-NPR1 antibody according to any one of the foregoing and detecting the presence of a complex between the anti-NPR1 antibody and the NPR1 peptide or the fragment thereof, wherein that the complex is detected indicates the presence of the NPR1 peptide or the fragment thereof.

In another aspect, the present disclosure provides a method for treating, preventing, or ameliorating an NPR1-related disease or disorder, and the method comprises administering to a subject the anti-NPR1 antibody according to any one of the above, or the pharmaceutical composition according to any one of the above, or the immunoconjugate according to any one of the above.

In another aspect, the present disclosure provides use in the manufacture of a medicament for treating, preventing, or ameliorating an NPR1-related disease or disorder, comprising administering to a subject a therapeutically or prophylactically effective amount of the anti-NPR1 antibody according to any one of the above, or the pharmaceutical composition according to any one of the above, or the immunoconjugate according to any one of the above.

In another aspect, the present disclosure provides the anti-NPR1 antibody according to any one of the above, or the pharmaceutical composition according to any one of the above, or the immunoconjugate according to any one of the above for use as a medicament. In some embodiments, the medicament is used for treating, preventing, or ameliorating an NPR1-related disease or disorder.

In some embodiments, the disease or disorder according to any one of the above is selected from the group consisting of heart failure, hypertension, peripheral vascular disease, coronary artery disease (CAD), ischemic heart disease (IHD), mitral stenosis and regurgitation, angina pectoris, hypertrophic cardiomyopathy (HCM), diabetic cardiomyopathy, supraventricular and ventricular cardiac arrhythmias, arrhythmia, atrial fibrillation (AF), new-onset atrial fibrillation, recurrent atrial fibrillation, cardiac fibrosis, atrial flutter, adverse vascular remodeling, plaque stabilization, myocardial infarction (MI), preeclampsia, obesity, renal failure, renal disorder, cytokine release syndrome, chronic kidney disease, macular edema, glaucoma, stroke, pulmonary disease, pulmonary fibrosis, inflammation, asthma, skeletal growth disorder, fracture, diabetes, and cancer. In some embodiments, the heart failure according to any one of the above is selected from the group consisting of heart failure with preserved ejection fraction (HFpEF), heart failure with reduced ejection fraction (HFrEF), heart failure with mildly reduced ejection fraction (HFmrEF), heart failure after acute myocardial infarction, and acute decompensated heart failure; in some embodiments, the heart failure according to any one of the above is heart failure with preserved ejection fraction (HFpEF).

In some embodiments, the hypertension according to any one of the above is resistant hypertension.

### BRIEF DESCRIPTION OF THE DRAWINGS

FIG. 1A shows ELISA results for the binding of anti-NPR1 antibodies to the hNPR1 antigen.
FIG. 1B shows ELISA results for the binding of anti-NPR1 antibodies to the hNPR2 antigen.
FIG. 1C shows ELISA results for the binding of anti-NPR1 antibodies to the hNPR3 antigen.
FIG. 2 shows experimental results for the stimulation of cGMP production in hNPR1-CHOK1 cells by anti-NPR1 antibodies.
FIG. 3 shows experimental results for the endocytosis of anti-NPR1 antibodies after binding to NPR1.
FIG. 4A shows experimental results for the stimulation of cGMP production by antibody Hu102H4L6 in cells before and after endocytosis treatment.
FIG. 4B shows experimental results for the stimulation of cGMP production by antibody Hu140H5L5-11 in cells before and after endocytosis treatment.
FIG. 5A shows the effects of anti-NPR1 antibodies on the systolic blood pressure of hNPR1 homozygous transgenic mice with ANGII-induced hypertension.
FIG. 5B shows the effects of anti-NPR1 antibodies on the diastolic blood pressure of hNPR1 homozygous transgenic mice with ANGII-induced hypertension.
FIG. 5C shows the effects of anti-NPR1 antibodies on the mean arterial pressure of hNPR1 homozygous transgenic mice with ANGII-induced hypertension.
FIG. 5D shows the effects of anti-NPR1 antibodies on the heart rate of hNPR1 homozygous transgenic mice with ANGII-induced hypertension.
FIG. 5E shows the effects of anti-NPR1 antibodies on the cGMP concentration in the plasma of hNPR1 homozygous transgenic mice with ANGII-induced hypertension.
FIG. 5F shows the effects of anti-NPR1 antibodies on the cGMP concentration in the urine of hNPR1 homozygous transgenic mice with ANGII-induced hypertension.
FIG. 5G shows endpoint plasma concentrations in the plasma of hNPR1 homozygous transgenic mice with ANGII-induced hypertension after administration.
FIG. 5H shows the effects of anti-NPR1 antibodies on the mean NT-proBNP concentration in the plasma of hNPR1 homozygous transgenic mice with ANGII-induced hypertension.
FIG. 5I shows the effects of anti-NPR1 antibodies on the concentration of sodium ions in the urine of hNPR1 homozygous transgenic mice with ANGII-induced hypertension.

### DETAILED DESCRIPTION

### Terminology

To facilitate the understanding of the present disclosure, certain technical and scientific terms are described below. Unless otherwise specifically defined herein, all technical and scientific terms used herein have the same meaning as commonly understood by those of ordinary skill in the art. The singular forms "a", "an", and "the" used in the description and claims include plural reference unless the context clearly dictates otherwise.

Unless otherwise clearly stated in the context, throughout the description and the claims, the words "comprise", "have", "include", and the like, should be construed in an inclusive sense as opposed to an exclusive or exhaustive sense.

The term "cytokine" is a general term for proteins that are released by a cell population to act on other cells as intercellular mediators. Examples of such cytokines include lymphokines, monokines, chemokines, and traditional polypeptide hormones. Exemplary cytokines include mIL-2, IFNγ, TNFα, CCL-2, and IL-6.

The term "and/or" is meant to include the two meanings "and" and "or". For example, the phrase "A, B, and/or C" is intended to encompass each of the following: A, B, and C; A, B, or C; A or C; A or B; B or C; A and C; A and B; B and C; A (alone); B (alone); and C (alone).

The three-letter and single-letter codes for amino acids used in the present disclosure are as described in J. Biol. Chem., 243, p3558 (1968).

The term "NPR1" refers to natriuretic peptide receptor 1, also known as atrial natriuretic peptide receptor type A (ANP-A, ANPR-A, or NPR-A) and guanylate cyclase A (GC-A). The term "NPR1" refers to a naturally occurring NPR1 protein (for example, but not limited to, precursor, mature, modified, or splice variant forms thereof). The human NPR1 protein comprises the amino acid sequence of SEQ ID NO: 4. The amino acid sequences of NPR1 molecules from non-human species (e.g., mice, rats, monkeys, rabbits, dogs, and pigs) may be obtained from public sources; examples include cynomolgus monkey NPR1 protein (NCBI No. XP-005541809.1) and rat NPR1 protein (Uniprot No. P18910). Although specific database accession numbers are given herein, skilled persons understand that the NPR1 referred to herein also encompasses corresponding sequences reported in other databases or documents.

The term "amino acid" refers to naturally occurring and synthetic amino acids, as well as amino acid analogs and amino acid mimics that function similarly to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have an identical basic chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain an identical basic chemical structure to naturally occurring amino acids. Amino acid mimics refer to chemical compounds that have a structure different from the general chemical structure of amino acids, but function similarly to naturally occurring amino acids.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In some embodiments, the amino acid mutation is a non-conservative amino acid substitution, i.e., the replacement of one amino acid with another amino acid having different structural and/or chemical properties. Amino acid substitutions include replacement with non-naturally occurring amino acids or with derivatives of the 20 natural amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the amino acid residue at a particular position may be denoted by position + amino acid residue; for example, 102S means that the amino acid residue at position 102 is S. C102S means that the amino acid residue at position 102 is mutated from C to S. When the residue at a characteristic position is defined with "position + amino acid residue" in a claim, the original residue at that position does not limit the scope of protection. The term "antibody" is used in the broadest sense and encompasses a variety of antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding moieties), as long as they exhibit the desired antigen-binding activity.

The term "natural antibody" refers to a naturally occurring immunoglobulin molecule. For example, a natural IgG antibody is a heterotetrameric glycoprotein of about 150,000 Daltons composed of two light chains and two heavy chains linked by a disulfide bond. From the N-terminus to the C-terminus, each heavy chain comprises one variable region (VH, also known as variable heavy domain or heavy chain variable region) followed by a heavy chain constant region. Generally, a natural IgG heavy chain constant region comprises three constant domains (CH1, CH2, and CH3). Similarly, from the N-terminus to the C-terminus, each light chain comprises one variable region (VL, also known as variable light domain or light chain variable domain) followed by one constant light domain (light chain constant region, CL).

The terms "full-length antibody", "intact antibody", and "whole antibody" are used herein interchangeably and refer to an antibody having a structure substantially similar to a natural antibody structure or comprising a heavy chain with an Fc region as defined herein. In a natural intact antibody, the light chain comprises light chain variable region VL and constant region CL, wherein the VL is positioned at the amino terminus of the light chain, and the light chain constant region comprises a κ chain and a λ chain; the heavy chain comprises a variable region VH and a constant region (CH1, CH2, and CH3), wherein the VH is positioned at the amino terminus of the heavy chain, and the constant region is positioned at the carboxyl terminus, wherein the CH3 is closest to the carboxyl terminus of the polypeptide, and the heavy chain can be of any isotype, including IgG (including subtypes IgG1, IgG2, IgG3, and IgG4), IgA (including subtypes IgA1 and IgA2), IgM, and IgE.

The term "variable region" or "variable domain" of an antibody refers to a domain in an antibody heavy or light chain that is involved in the binding of the antibody to an antigen. Herein, the heavy chain variable region (VH) and light chain variable region (VL) of the antibody each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Each VH and VL is composed of three CDRs and four FRs arranged from the amino terminus (also known as N-terminus) to the carboxyl terminus (also known as C-terminus) in the following order: FR1, CDR1, FR2, CDR2, FR3, CDR3, and FR4.

The amino acid sequence boundaries of the CDRs can be determined by a variety of well-known schemes, for example, the "Kabat" numbering scheme (see Kabat et al., (1991), "Sequences of Proteins of Immunological Interest", 5th Edition, Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), and the ImMunoGenTics (IMGT) numbering scheme (Lefranc, M.P. et al., Dev. Comp. Immunol., 27, 55-77 (2003); Front Immunol. 2018 Oct 16; 9:2278), and the like. The corresponding relationships between the various numbering schemes are well known to those skilled in the art and, illustratively, are shown in Table 1 below.

**Table 1. The relationships between CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the variable regions and CDRs in examples of the present disclosure. Although the Kabat numbering scheme is employed to define amino acid residues in specific embodiments, corresponding technical solutions for other numbering schemes are to be considered as equivalent technical solutions.

The term "antibody fragment" refers to a molecule different from an intact antibody, which comprises a moiety of an intact antibody that binds to an antigen to which the intact antibody binds. Examples of antibody fragments include, but are not limited to, Fv, Fab, Fab', Fab'-SH, F(ab')2, single-domain antibodies, single-chain Fab (scFab), diabodies, linear antibodies, single-chain antibodies (e.g., scFv), and multispecific antibodies formed from antibody fragments.

The term "Fc region" or "fragment crystallizable region" is used to define the C-terminal region of the heavy chain of an antibody, including native Fc regions and engineered Fc regions. In some embodiments, the Fc region comprises two identical or different subunits. In some embodiments, the Fc region of the human IgG heavy chain is defined as extending from the amino acid residue at position Cys226 or from Pro230 to its carboxyl terminus. Suitable Fc regions used for the antibodies described herein include Fc regions of human IgG1, IgG2 (IgG2A and IgG2B), IgG3, and IgG4. In some embodiments, the boundaries of the Fc region may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Unless otherwise stated, the numbering scheme for the Fc region is the EU numbering scheme, also known as the EU index.

The term "chimeric" antibody refers to an antibody in which a portion of the heavy and/or light chain is derived from a particular source or species, while the remainder of the heavy and/or light chain is derived from another different source or species.

The term "humanized" antibody refers to an antibody that retains the reactivity of a non-human antibody while having low immunogenicity in humans. For example, this can be achieved by retaining the non-human CDRs and replacing the remainder of the antibody with its human counterparts (i.e., the constant regions and the framework region portion of the variable regions). The terms "human antibody", "fully human antibody", and "complete human antibody" are used interchangeably and refer to antibodies in which the variable regions and constant regions are human sequences. The term encompasses antibodies that are derived from human genes but have, for example, sequences that have been altered to, e.g., reduce possible immunogenicity, increase affinity, and eliminate cysteines or glycosylation sites that may cause undesired folding. The term encompasses antibodies recombinantly produced in non-human cells (that may confer glycosylation not characteristic of human cells). The term also encompasses antibodies that have been produced in transgenic mice comprising some or all of the human immunoglobulin heavy and light chain loci. The meaning of the human antibody specifically excludes humanized antibodies comprising non-human antigen-binding residues.

The term "affinity" refers to the overall strength of the non-covalent interaction between a single binding site of a molecule (e.g., an antibody) and its binding ligand (e.g., an antigen). Unless otherwise indicated, as used herein, binding "affinity" refers to an internal binding affinity that reflects the 1:1 interaction between members of a binding pair (e.g., an antibody and an antigen). The affinity of molecule X for its ligand Y can be generally denoted by the dissociation constant (KD). Affinity can be determined by conventional methods known in the art, including those described herein.

As used herein, the term "kassoc" or "ka" refers to the association rate of a particular antibody-antigen interaction, while the term "kdis" or "kd" refers to the dissociation rate of a particular antibody-antigen interaction. The term "KD" refers to the dissociation constant, which is obtained from the ratio of kd to ka (i.e., kd/ka) and denoted by a molar concentration (M). The KD value of an antibody can be determined using methods well known in the art. For example, surface plasmon resonance is measured using a biosensing system such as a system (e.g., Biacore), or the affinity in a solution is measured by solution equilibrium titration (SET).

The term "surface plasmon resonance" refers to an optical phenomenon that allows for the analysis of real-time interactions by detecting changes in protein concentrations within a biosensor matrix, for example, using the BIAcoreTM system (Biacore Life Sciences division of GE Healthcare, Piscataway, NJ).

The term "effector function" refers to biological activities that can be attributed to the Fc region of an antibody (either the natural sequence Fc region or the amino acid sequence variant Fc region) and vary with the antibody isotype. Examples of effector functions of an antibody include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "monoclonal antibody" refers to a population of substantially homogeneous antibodies, that is, the amino acid sequences of the antibody molecules comprised in the population are identical, except for a small number of natural mutations that may exist. In contrast, a polyclonal antibody formulation generally comprises several different antibodies comprising different amino acid sequences in their variable domains, which are generally specific for different epitopes. "Monoclonal" refers to the characteristics of an antibody obtained from a substantially homogeneous antibody population and should not be construed as requiring the production of the antibody by any particular method. In some embodiments, the antibody provided by the present disclosure is a monoclonal antibody.

The term "antigen" refers to a molecule or molecular portion that can be bound by, for example, antigen-binding proteins (including, for example, antibodies). An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed from contiguous strings of amino acids (linear epitope) or comprise non-contiguous amino acids (conformational epitope), e.g., coming in spatial proximity due to the folding of the antigen (i.e., by the tertiary folding of an antigen of a protein nature). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, including, for example, but not limited to, alanine scanning, peptide blotting, peptide cleavage analysis, epitope excision, epitope extraction, chemical modification of the antigen (Prot. Sci. 9 (2000) 487-496), and cross-blocking.

The term "capable of specifically binding", "specifically bind", or "bind" means that an antibody is capable of binding to a certain antigen or an epitope of the antigen with higher affinity than to other antigens or epitopes. Generally, an antibody binds to an antigen or an epitope in the antigen with an equilibrium dissociation constant (KD) of about 1 × 10⁻⁷ M or less (e.g., about 1 × 10⁻⁸ M, 1 × 10⁻⁹ M, 1 × 10⁻¹⁰ M, 1 × 10⁻¹¹ M, or less). In some embodiments, the KD for the binding of an antibody to an antigen is 10% or less (e.g., 1%) of the KD for the binding of the antibody to a nonspecific antigen (e.g., BSA or casein). KD may be determined using known methods, for example, by a BIACORE^{®} surface plasmon resonance assay. However, an antibody that specifically binds to an antigen or an epitope in the antigen may have cross-reactivity with other related antigens, for example, with corresponding antigens from other species (homologous) (e.g., humans or monkeys, such as Macaca fascicularis (cynomolgus, cyno), Pan troglodytes (chimpanzee, chimp), or Callithrix jacchus (commonmarmoset, marmoset)).

The term "not bind" means that an antibody is not capable of binding to a certain antigen or an epitope thereof in the specific binding manner described above, for example, when the antibody binds to the antigen or the epitope thereof with an equilibrium dissociation constant (KD) of about 1 × 10⁻⁶ M or greater.

The terms "anti-NPR1 antibody" and "antibody that binds to NPR1" refer to an antibody that is capable of binding to NPR1 or an epitope thereof with sufficient affinity.

The term "activating antibody" or "agonist antibody" (or "an antibody that increases or enhances NPR1 activity" or "an antibody that stabilizes the activated conformation") is intended to refer to an antibody whose binding to NPR1 results in activation of at least one biological activity of NPR1. For example, an antibody of the present disclosure may reduce mean arterial blood pressure upon administration to a subject in need thereof.

The term "antibody-dependent cellular cytotoxicity", "antibody-dependent cell-mediated cytotoxicity", or "ADCC" is a mechanism for inducing cell death, which depends on the interaction of antibody-coated target cells with effector cells with lytic activity, such as natural killer cells (NK), monocytes, macrophages, and neutrophils, via an Fcγ receptor (FcγR) expressed on the effector cells. For example, NK cells express FcγRIIIa, while monocytes express FcγRI, FcγRII, and FcγRIIIa. The ADCC activity of the antibodies provided herein can be assessed by *in vitro* assays, using cells expressing the antigen as target cells and NK cells as effector cells. Cell lysis is detected based on the release of a label (e.g., radioactive substrate, fluorescent dye, or natural intracellular protein) from the lysed cells.

The term "antibody-dependent cellular phagocytosis (ADCP)" refers to a mechanism by which antibody-coated target cells are eliminated by internalization of phagocytic cells (such as macrophages or dendritic cells).

The term "complement-dependent cytotoxicity" or "CDC" refers to a mechanism for inducing cell death in which the Fc effector domain of a target-binding antibody binds to and activates a complement component C1q, and C1q then activates the complement cascade, resulting in the death of the target cell. The activation of a complement may also result in the deposition of complement components on the surface of target cells, and these complement components promote CDC by binding to complement receptors on leukocytes (e.g., CR3).

The term "ANGII" refers to angiotensin II, which is a polypeptide substance produced by the hydrolysis of angiotensin I under the action of angiotensin-converting enzyme. Angiotensin receptors are present on vascular smooth muscle, cells of the zona glomerulosa of the adrenal cortex, and cells of some parts of the brain, heart, and kidney organs. Angiotensin II binds to angiotensin receptors, causing corresponding physiological effects, including: 1) causing systemic constriction of arterioles and veins, which results in an increase in blood pressure and an increase in the volume of blood returned to the heart; 2) increasing the release of sympathetic vasoconstrictor fiber transmitters; 3) increasing the tonicity of the sympathetic vasoconstrictor center; 4) stimulating the synthesis and release of aldosterone by the adrenal gland; and 5) causing or enhancing thirst, which results in water drinking behavior. In some embodiments, a model is established by inducing an increase in the blood pressure of mice using ANGII. In some embodiments, a model is established by inducing progressive heart failure in mice using ANGII. The term "nucleic acid" is used interchangeably herein with the term "polynucleotide" and refers to deoxyribonucleotide or ribonucleotide and a polymer thereof in either single-stranded or doublestranded form. The term encompasses nucleic acids comprising known nucleotide analogs or modified backbone residues or linkages, which are synthetic, naturally occurring, and non-naturally occurring, have similar binding properties to the reference nucleic acid, and are metabolized similarly to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. The isolated nucleic acid includes a nucleic acid molecule contained in a cell that generally comprises the nucleic acid molecule, but the nucleic acid molecule is present extrachromosomally or at a chromosomal position different from its natural chromosomal position. An isolated nucleic acid encoding a polypeptide or a fusion protein refers to one or more nucleic acid molecules encoding the polypeptide or fusion protein, including such one or more nucleic acid molecules in a single vector or separate vectors, and such one or more nucleic acid molecules present at one or more positions in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues.

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term sequence "identity" refers to the degree (percentage) to which the amino acids/nucleic acids of two sequences are identical at equivalent positions, wherein when the two sequences are optimally aligned, gaps are introduced, if necessary, to achieve the maximum sequence identity percentage, and any conservative substitutions are not considered part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

The term "vector" means a polynucleotide molecule capable of transporting another polynucleotide linked thereto. One type of vector is a "plasmid", which refers to a circular doublestranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that can be transformed into a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progeny derived therefrom, regardless of the number of passages. Progeny may not be completely identical to parent cells in terms of nucleic acid content and may contain mutations. Mutant progenies that have the same function or biological activity as the cells screened or selected from the initially transformed cells are included herein. Host cells include prokaryotic and eukaryotic host cells, wherein the eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichia pastoris, Pichia finlandica, Pichia trehalophila, Pichia koclamae, Pichia membranaefaciens, Pichia minuta (Ogataea minuta, Pichia lindneri), Pichia opuntiae, Pichia thermotolerans, Pichia salictaria, Pichia guercuum, Pichia pijperi, Pichia stiptis, Pichia methanolica, Pichia, Saccharomyces cerevisiae, Saccharomyces, Hansenula polymorpha, Kluyveromyces, Kluyveromyces lactis, Candida albicans, Aspergillus, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella patens,* and *Neurospora crassa.*

"Optional" or "optionally" means that the event or circumstance subsequently described may, but does not necessarily, occur. This description includes the instance where the event or circumstance occurs or does not occur.

The term "pharmaceutical composition" refers to a mixture comprising one or more of the anti-NPR1 antibodies described herein and other chemical components; the other components are, for example, physiological/pharmaceutically acceptable carriers and excipients.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "subject" or "individual" includes humans and non-human animals. Non-human animals include all vertebrates (e.g., mammals and non-mammals) such as non-human primates, sheep, dogs, cows, chickens, amphibians, and reptiles. Unless indicated, the terms "patient" and "subject" are used interchangeably herein. In certain embodiments, the individual or subject is a human.

"Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection of similar fluids, cells, or tissues isolated from a subject, as well as fluids, cells, or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned culture media); lavage fluids; and the like), tissue biopsy samples, fine needle punctures, surgically excised tissues, organ cultures, or cell cultures.

"Treatment" or "treat" (and grammatical variations thereof) refers to clinical intervention in an attempt to alter the natural course of the treated individual, which may be performed either for prophylaxis or during the course of clinical pathology. Desirable effects of the treatment include, but are not limited to, preventing the occurrence or recurrence of a disease, palliating symptoms, palliating/reducing any direct or indirect pathological consequences of the disease, preventing metastasis, decreasing the rate of disease progression, ameliorating or palliating the disease state, and regressing or improving prognosis. In some embodiments, an antibody of the present disclosure is used to delay the development of or slow the progression of a disease.

The term "recurrence", "relapse", or "relapsed" refers to the return of a cancer or disease after clinical assessment of the disappearance of disease. A diagnosis of distant metastasis or local recurrence can be considered a relapse.

The term "refractory" or "resistant" refers to a cancer or disease that has not responded to treatment. "Effective amount" is generally an amount sufficient to reduce the severity and/or frequency of symptoms, eliminate symptoms and/or underlying causes, prevent the appearance of symptoms and/or their underlying causes, and/or ameliorate or alleviate damage (e.g., lung disease) caused by or associated with a disease state. In some examples, the effective amount is a therapeutically effective amount or a prophylactically effective amount. "Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health of a patient.

### Anti-NPR1 Antibodies of the Present Disclosure

The present disclosure provides anti-NPR1 antibodies or antigen-binding fragments thereof, having a number of advantageous properties, such as good therapeutic activity, safety, pharmacokinetic properties, and druggability (e.g., solubility, viscosity, purity, and stability).

### Exemplary Anti-NPR1 Antibodies

Examples of the present disclosure disclose antibody series 102, 127, 128, 135, and 140. Antibodies 140 and 102 are described below as examples of the antibodies of the present disclosure. Illustratively, provided is an anti-NPR1 antibody of the present disclosure, wherein the anti-NPR1 antibody comprises a heavy chain variable region and a light chain variable region, wherein:
a. the heavy chain variable region comprises a HCDR1, a HCDR2, and a HCDR3 that comprise the amino acid sequences of a HCDR1, a HCDR2, and a HCDR3 in SEQ ID NO: 82, respectively, and the light chain variable region comprises a LCDR1, a LCDR2, and a LCDR3 that comprise the amino acid sequences of a LCDR1, a LCDR2, and a LCDR3 in SEQ ID NO: 85, respectively; or
b. the heavy chain variable region comprises a HCDR1, a HCDR2, and a HCDR3 that comprise the amino acid sequences of a HCDR1, a HCDR2, and a HCDR3 of any one of SEQ ID NO: 51, 48, or 52, respectively, and the light chain variable region comprises a LCDR1, a LCDR2, and a LCDR3 that comprise the amino acid sequences of a LCDR1, a LCDR2, and a LCDR3 in SEQ ID NO: 56, respectively.

Illustratively, provided is an anti-NPR1 antibody of the present disclosure, wherein the anti-NPR1 antibody comprises a heavy chain variable region and a light chain variable region, wherein:
a. the heavy chain variable region comprises a HCDR1, a HCDR2, and a HCDR3 that comprise the amino acid sequences of a HCDR1, a HCDR2, and a HCDR3 in SEQ ID NO: 82, respectively, and the light chain variable region comprises a LCDR1, a LCDR2, and a LCDR3 that comprise the amino acid sequences of a LCDR1, a LCDR2, and a LCDR3 in SEQ ID NO: 85, respectively; or
b. the heavy chain variable region comprises a HCDR1, a HCDR2, and a HCDR3 that comprise the amino acid sequences of a HCDR1, a HCDR2, and a HCDR3 in SEQ ID NO: 51, respectively, and the light chain variable region comprises a LCDR1, a LCDR2, and a LCDR3 that comprise the amino acid sequences of a LCDR1, a LCDR2, and a LCDR3 in SEQ ID NO: 56, respectively.

Illustratively, provided is an anti-NPR1 antibody of the present disclosure, wherein the anti-NPR1 antibody comprises a heavy chain variable region and a light chain variable region, wherein:
a. the heavy chain variable region comprises a HCDR1 comprising the amino acid sequence of SEQ ID NO: 29, a HCDR2 comprising the amino acid sequence of SEQ ID NO: 40, and a HCDR3 comprising the amino acid sequence of SEQ ID NO: 41, and the light chain variable region comprises a LCDR1 comprising the amino acid sequence of SEQ ID NO: 42, a LCDR2 comprising the amino acid sequence of SEQ ID NO: 33, and a LCDR3 comprising the amino acid sequence of SEQ ID NO: 34; or
b. the heavy chain variable region comprises a HCDR1 comprising the amino acid sequence of SEQ ID NO: 45, 17, or 35, a HCDR2 comprising the amino acid sequence of SEQ ID NO: 46 or 18, and a HCDR3 comprising the amino acid sequence of SEQ ID NO: 19, and the light chain variable region comprises a LCDR1 comprising the amino acid sequence of SEQ ID NO: 20, a LCDR2 comprising the amino acid sequence of SEQ ID NO: 21, and a LCDR3 comprising the amino acid sequence of SEQ ID NO: 22.

Illustratively, provided is an anti-NPR1 antibody of the present disclosure, wherein the anti-NPR1 antibody comprises a heavy chain variable region and a light chain variable region, wherein:
the heavy chain variable region comprises a HCDR1 comprising the amino acid sequence of SEQ ID NO: 29, a HCDR2 comprising the amino acid sequence of SEQ ID NO: 40, and a HCDR3 comprising the amino acid sequence of SEQ ID NO: 41, and the light chain variable region comprises a LCDR1 comprising the amino acid sequence of SEQ ID NO: 42, a LCDR2 comprising the amino acid sequence of SEQ ID NO: 33, and a LCDR3 comprising the amino acid sequence of SEQ ID NO: 34.

Illustratively, provided is an anti-NPR1 antibody of the present disclosure, wherein the anti-NPR1 antibody comprises a heavy chain variable region and a light chain variable region, wherein:
the heavy chain variable region comprises a HCDR1 whose amino acid sequence is set forth in SEQ ID NO: 29, a HCDR2 whose amino acid sequence is set forth in SEQ ID NO: 40, and a HCDR3 whose amino acid sequence is set forth in SEQ ID NO: 41, and the light chain variable region comprises a LCDR1 whose amino acid sequence is set forth in SEQ ID NO: 42, a LCDR2 whose amino acid sequence is set forth in SEQ ID NO: 33, and a LCDR3 whose amino acid sequence is set forth in SEQ ID NO: 34.

Illustratively, provided is an anti-NPR1 antibody of the present disclosure, wherein the anti-NPR1 antibody comprises a heavy chain variable region and a light chain variable region, wherein:
the heavy chain variable region comprises a HCDR1 comprising the amino acid sequence of SEQ ID NO: 45, a HCDR2 comprising the amino acid sequence of SEQ ID NO: 46, and a HCDR3 comprising the amino acid sequence of SEQ ID NO: 19, and the light chain variable region comprises a LCDR1 comprising the amino acid sequence of SEQ ID NO: 20, a LCDR2 comprising the amino acid sequence of SEQ ID NO: 21, and a LCDR3 comprising the amino acid sequence of SEQ ID NO: 22.

Illustratively, provided is an anti-NPR1 antibody of the present disclosure, wherein the anti-NPR1 antibody comprises a heavy chain variable region and a light chain variable region, wherein:
the heavy chain variable region comprises a HCDR1 whose amino acid sequence is set forth in SEQ ID NO: 45, a HCDR2 whose amino acid sequence is set forth in SEQ ID NO: 46, and a HCDR3 whose amino acid sequence is set forth in SEQ ID NO: 19, and the light chain variable region comprises a LCDR1 whose amino acid sequence is set forth in SEQ ID NO: 20, a LCDR2 whose amino acid sequence is set forth in SEQ ID NO: 21, and a LCDR3 whose amino acid sequence is set forth in SEQ ID NO: 22.

Illustratively, provided is an anti-NPR1 antibody of the present disclosure, wherein the anti-NPR1 antibody is a murine antibody, a chimeric antibody, a humanized antibody, or a fully human-derived antibody. In some embodiments, the antibody is a chimeric antibody. In some embodiments, the antibody is a humanized antibody.

Illustratively, provided is an anti-NPR1 antibody of the present disclosure, wherein the anti-NPR1 antibody comprises human antibody framework regions (FRs).

Illustratively, provided is an anti-NPR1 antibody of the present disclosure, wherein the heavy chain variable region comprises a FR1, a FR2, and a FR3 that are derived from IGHV1-46*01 and a FR4 derived from IGHJ6*01, and the FRs are unsubstituted or comprise one or more amino acid substitutions selected from the group consisting of 1E, 40R, 43R, 48I, 69L, 71V, 73K, 74P, 75S, 77P, 78A, 80I, and 81Q; and/or the light chain variable region comprises a FR1, a FR2, and a FR3 that are derived from IGKV3-11*01 and a FR4 derived from IGKJ2*01, and the FRs are unsubstituted or comprise one or more amino acid substitutions selected from the group consisting of 20Q, 58V, 60D, 70S, 71Y, 77R, and 100A. In some embodiments, provided is the anti-NPR1 antibody, wherein in the heavy chain variable region, the HCDR1 comprises the amino acid sequence of SEQ ID NO: 29, the HCDR2 comprises the amino acid sequence of SEQ ID NO: 40, and the HCDR3 comprises the amino acid sequence of SEQ ID NO: 41, and the FRs of the heavy chain variable region are unsubstituted or comprise one or more amino acid substitutions selected from the group consisting of 1E, 40R, 43R, 48I, 69L, 71V, 73K, 74P, 75S, 77P, 78A, 80I, and 81Q; and in the light chain variable region, the LCDR1 comprises the amino acid sequence of SEQ ID NO: 42, the LCDR2 comprises the amino acid sequence of SEQ ID NO: 33, and the LCDR3 comprises the amino acid sequence of SEQ ID NO: 34, and the FRs of the light chain variable region are unsubstituted or comprise one or more amino acid substitutions selected from the group consisting of 20Q, 58V, 60D, 70S, 71Y, 77R, and 100A. In some embodiments, the variable regions and CDRs described above are defined according to the Kabat numbering scheme.

Illustratively, provided is an anti-NPR1 antibody of the present disclosure, wherein the heavy chain variable region comprises a FR1, a FR2, and a FR3 that are derived from IGHV3-21*01 and a FR4 derived from IGHJ6*01, and the FRs are unsubstituted or comprise one or more amino acid substitutions selected from the group consisting of 1D, 3K, 9E, 44R, 49A, 87S, and 93T; and/or the light chain variable region comprises a FR1, a FR2, and a FR3 that are derived from IGKV3-11*01 or IGKV1-39*01 and a FR4 derived from IGKJ4*01, and the FRs are unsubstituted or comprise one or more amino acid substitutions selected from the group consisting of 4L, 43S, 45K, 46R, 47W, 58V, and 71Y. In some embodiments, provided is the anti-NPR1 antibody, wherein in the heavy chain variable region, the HCDR1 comprises the amino acid sequence of SEQ ID NO: 45, the HCDR2 comprises the amino acid sequence of SEQ ID NO: 46, and the HCDR3 comprises the amino acid sequence of SEQ ID NO: 19, and the FRs of the heavy chain variable region are unsubstituted or comprise one or more amino acid substitutions selected from the group consisting of 1D, 3K, 9E, 44R, 49A, 87S, and 93T; and in the light chain variable region, the LCDR1 comprises the amino acid sequence of SEQ ID NO: 20, the LCDR2 comprises the amino acid sequence of SEQ ID NO: 21, and the LCDR3 comprises the amino acid sequence of SEQ ID NO: 22, and the FRs of the light chain variable region are unsubstituted or comprise one or more amino acid substitutions selected from the group consisting of 4L, 43S, 45K, 46R, 47W, 58V, and 71Y. In some embodiments, the variable regions and CDRs described above are defined according to the Kabat numbering scheme.

Illustratively, provided is an anti-NPR1 antibody of the present disclosure, wherein:
a. the heavy chain variable region comprises an amino acid sequence having at least 85% (e.g., at least 85%, 87%, 90%, 93%, 94%, 95%, 96%, 97%, 98%, or 99%) sequence identity to SEQ ID NO: 82, and the light chain variable region comprises an amino acid sequence having at least 85% (e.g., at least 85%, 87%, 90%, 93%, 94%, 95%, 96%, 97%, 98%, or 99%) sequence identity to SEQ ID NO: 85; or
b. the heavy chain variable region comprises an amino acid sequence having at least 85% (e.g., at least 85%, 87%, 90%, 93%, 94%, 95%, 96%, 97%, 98%, or 99%) sequence identity to SEQ ID NO: 51, and the light chain variable region comprises an amino acid sequence having at least 85% (e.g., at least 85%, 87%, 90%, 93%, 94%, 95%, 96%, 97%, 98%, or 99%) sequence identity to SEQ ID NO: 56.

Illustratively, provided is an anti-NPR1 antibody of the present disclosure, wherein:
a. the heavy chain variable region comprises the amino acid sequence of SEQ ID NO: 82, and the light chain variable region comprises the amino acid sequence of SEQ ID NO: 85; or
b. the heavy chain variable region comprises the amino acid sequence of SEQ ID NO: 51, and the light chain variable region comprises the amino acid sequence of SEQ ID NO: 56.

Illustratively, provided is an anti-NPR1 antibody of the present disclosure, wherein:
a. the amino acid sequence of the heavy chain variable region is set forth in SEQ ID NO: 82, and the amino acid sequence of the light chain variable region is set forth in SEQ ID NO: 85; or
b. the amino acid sequence of the heavy chain variable region is set forth in SEQ ID NO: 51, and the amino acid sequence of the light chain variable region is set forth in SEQ ID NO: 56. Illustratively, provided is an anti-NPR1 antibody of the present disclosure, wherein the anti-NPR1 antibody is an antibody fragment; in some embodiments, the antibody fragment is selected from the group consisting of Fab, Fab', F(ab')2, Fd, Fv, scFv, dsFv, and dAb.

Illustratively, provided is an anti-NPR1 antibody of the present disclosure, wherein the anti-NPR1 antibody comprises a heavy chain constant region and a light chain constant region; in some embodiments, the heavy chain constant region is a heavy chain constant region of human IgG1, IgG2, IgG3, IgG4, or a variant thereof; in some embodiments, the light chain constant region is a human κ or λ light chain constant region; in some embodiments, the heavy chain constant region comprises the amino acid sequence of SEQ ID NO: 86 or 43, and the light chain constant region comprises the amino acid sequence of SEQ ID NO: 44.

Illustratively, provided is an anti-NPR1 antibody of the present disclosure, wherein the anti-NPR1 antibody comprises a heavy chain and a light chain, wherein:
a. the heavy chain comprises an amino acid sequence having at least 85% (e.g., at least 85%, 87%, 90%, 93%, 94%, 95%, 96%, 97%, 98%, or 99%) sequence identity to SEQ ID NO: 89, and the light chain comprises an amino acid sequence having at least 85% (e.g., at least 85%, 87%, 90%, 93%, 94%, 95%, 96%, 97%, 98%, or 99%) sequence identity to SEQ ID NO: 90; or
b. the heavy chain comprises an amino acid sequence having at least 85% (e.g., at least 85%, 87%, 90%, 93%, 94%, 95%, 96%, 97%, 98%, or 99%) sequence identity to SEQ ID NO: 87, and the light chain comprises an amino acid sequence having at least 85% (e.g., at least 85%, 87%, 90%, 93%, 94%, 95%, 96%, 97%, 98%, or 99%) sequence identity to SEQ ID NO: 88.

In some embodiments, provided is the anti-NPR1 antibody according to any one of the above, wherein the anti-NPR1 antibody comprises a heavy chain and a light chain, wherein:
a. the amino acid sequence of the heavy chain is set forth in SEQ ID NO: 89, and the amino acid sequence of the light chain is set forth in SEQ ID NO: 90; or
b. the amino acid sequence of the heavy chain is set forth in SEQ ID NO: 87, and the amino acid sequence of the light chain is set forth in SEQ ID NO: 88.

Illustratively, the present disclosure further provides an isolated anti-NPR1 antibody that competes for binding to human NPR1 or an epitope thereof with the anti-NPR1 antibody according to any one of the foregoing.

Illustratively, the NPR1 antibody provided by the present disclosure specifically binds to hNPR1 or an epitope thereof and does not bind to the other NPR family members, such as hNPR2 and/or hNPR3.

Illustratively, the isolated anti-NPR1 antibody of the present disclosure binds to human NPR1 or an epitope thereof with an EC50 value of less than 30 nM (e.g., less than 27 nM, less than 10 nM, less than 9 nM, less than 7 nM, less than 5 nM, less than 4 nM, less than 2 nM, less than 1.5 nM, less than 0.1 nM, or less than 0.05 nM), wherein the EC50 value is measured by ELISA.

Illustratively, the isolated anti-NPR1 antibody of the present disclosure is capable of binding to hNPR1 CHO-K1 and cNPR1 CHO-K1, with or without 10 nM ANP.

Illustratively, the isolated anti-NPR1 antibody of the present disclosure can stimulate cGMP production in NPR1-expressing cells (e.g., hNPR1-CHOK1 cells).

In some embodiments, the isolated anti-NPR1 antibody of the present disclosure has cGMP production with a smaller difference on cells after endocytosis occurs than a positive antibody does.

Illustratively, the present disclosure can use an NPR1^{+/+} humanized mouse model of heart failure accompanied by hypertension induced by subcutaneous implantation of Ang II to evaluate the efficacy of different doses of anti-NPR1 antibodies. In some embodiments, the isolated anti-NPR1 antibody of the present disclosure is expected to be capable of improving the EDV (end-diastolic volume) and ESV (end-systolic volume) parameters of model mice. In some embodiments, the isolated anti-NPR1 antibody of the present disclosure is expected to be capable of reducing left ventricular weight. In some embodiments, the isolated anti-NPR1 antibody of the present disclosure is expected to be capable of significantly reducing heart index (heart index = heart weight/mouse body weight). In some embodiments, the isolated anti-NPR1 antibody of the present disclosure is expected to be capable of delaying myocardial fibrosis and improving heart function in model mice.

Illustratively, the anti-NPR1 antibody of the present disclosure has better pharmacokinetic performance (e.g., a longer half-life, higher bioavailability, better absorption, and smaller clearance) than positive antibodies (particularly XX16 V).

### Antibody Structure

In some embodiments, the antibody provided herein is a full-length antibody.

In some embodiments, the antibody provided herein is an antibody fragment.

In some embodiments, the antibody fragment is a Fab, Fab', Fab'-SH, or F(ab')₂ fragment, particularly a Fab fragment. "Fab" is a monovalent fragment consisting of the VL, VH, CL, and CH1 domains. "Fab fragment" may be produced by papain cleavage of an antibody. "Fab'" comprises VL, CL, and VH and CH1, and further comprises the region between the CH1 and CH2 domains, so that an interchain disulfide bond can be formed between the two heavy chains of two Fab' fragments to form a F(ab')2 molecule. "Fab'-SH" is a Fab' fragment in which the cysteine residues of the constant regions have a free sulfhydryl group. "F(ab')₂" is a bivalent fragment comprising two Fab fragments linked by a disulfide bond at the hinge region.

In some other embodiments, the antibody fragment is a diabody, a triabody, or a tetrabody. Diabodies are antibody fragments comprising two antigen-binding sites. The fragment comprises a VH and a VL linked in the same polypeptide chain (VH-VL). By using a linker that is too short to allow pairing between two domains on the same chain, the domains are forced to pair with the complementary domains of another chain, thereby resulting in two antigen-binding sites. The two antigens may be identical or different.

In some other embodiments, the antibody fragment is a single-chain Fab fragment. "Single-chain Fab fragment" or "scFab" is a polypeptide consisting of VH, CH1, VL, CL, and a linker, wherein the antibody domains and the linker have one of the following orders in the N-terminus to C-terminus direction: a) VH-CH1-linker-VL-CL, b) VL-CL-linker-VH-CH1, c) VH-CL-linker-VL-CH1, or d) VL-CH1-linker-VH-CL. In some embodiments, the linker is a polypeptide having at least 30 amino acids. In some other embodiments, the linker is a polypeptide having between 32 and 50 amino acids. The single-chain Fab fragment is stabilized via the natural disulfide bond between CL and CH1. In addition, these single-chain Fab molecules may be further stabilized by generating interchain disulfide bonds through insertion of cysteine residues (e.g., position 44 in the heavy chain variable region and position 100 in the light chain variable region, according to Kabat numbering).

In some other embodiments, the antibody fragment is an Fv fragment consisting of the VH and VL domains of a single arm of the antibody.

In some other embodiments, the antibody fragment is a single-chain variable fragment (scFv).

"scFv" is a fusion protein comprising at least one antibody fragment comprising a light chain variable region and at least one antibody fragment comprising a heavy chain variable region, wherein the light and heavy chain variable regions are contiguously linked by a short flexible peptide linker and are capable of being expressed as a single-chain polypeptide, and wherein the scFv retains the specificity of the intact antibody from which it is derived. Unless specifically indicated, an scFv may comprise VL and VH variable regions in any order herein; for example, with respect to the N-terminus and C-terminus of the polypeptide, the scFv may comprise VL-linker-VH or may comprise VH-linker-VL.

In some other embodiments, the antibody fragment is a dsFv obtained by linking polypeptides in which one amino acid residue of each of VH and VL is substituted with a cysteine residue by a disulfide bond between the cysteine residues. The amino acid residues substituted with cysteine residues can be selected according to known methods (Protein Engineering, 7:697 (1994)) based on the prediction of the three-dimensional structure of the antibody.

In some other embodiments, the antibody fragment is a single-domain antibody (dAb). Single-domain antibodies are antibody fragments comprising all or a portion of the heavy chain variable domain or all or a portion of the light chain variable domain of an antibody.

In some embodiments, the antibody provided herein is a chimeric antibody. In some embodiments, the chimeric antibody comprises a non-human variable region (e.g., a variable region derived from a mouse, rat, hamster, domestic rabbit, or non-human primate, such as a monkey) and a human constant region. In some embodiments, the chimeric antibody is a "class switched" antibody in which the class or subclass has been changed from the class or subclass of the parent antibody. In some embodiments, the antibody is a humanized antibody. Typically, non-human antibodies are humanized to reduce immunogenicity to humans while retaining the specificity and affinity of the parent non-human antibody. Generally, the humanized antibody comprises one or more variable regions in which the CDRs or portions thereof are derived from a non-human antibody and the FRs or portions thereof are derived from a human antibody. Optionally, the humanized antibody can further comprise a portion of a human constant region. In some embodiments, some FR residues in a humanized antibody may be replaced with corresponding residues from a non-human antibody (e.g., an antibody that provides CDR sequences).

Humanized antibodies and methods for their production are reviewed, e.g., in Almagro and Fransson, Front. Biosci. 13:1619-1633 (2008), and further described, e.g., in Riechmann et al., Nature 332:323-329 (1988); Queen et al., Proc. Nat'l Acad. Sci. USA 86:10029-10033 (1989); U.S. Patent Nos. 5,821,337, 7,527,791, 6,982,321, and 7,087,409; Kashmiri et al., Methods 36:25-34 (2005) (describing specificity-determining region (SDR) grafting); Padlan, Mol. Immunol. 28:489-498 (1991) (describing "resurfuacing"); Dall'Acqua et al., Methods 36:43-60 (2005) (describing "FR shuffling"); and Osbourn et al., Methods 36:61-68 (2005) and Klimka et al., Br. J. Cancer 83:252-260 (2000) (describing the "guided selection" method for FR shuffling).

Human framework regions that can be used for humanization include, but are not limited to: framework regions selected using the "best-fit" method (see, e.g., Sims et al., J. Immunol. 151:2296 (1993)); framework regions derived from the consensus sequence of human antibodies of a particular subgroup of light or heavy chain variable regions (see, e.g., Carter et al., Proc. Natl. Acad. Sci. USA, 89:4285 (1992); and Presta et al., J. Immunol., 151:2623 (1993)); human mature (somatically mutated) framework regions or human germline framework regions (see, e.g., Almagro and Fransson, Front. Biosci. 13:1619-1633 (2008)); and framework regions obtained by screening FR libraries (see, e.g., Baca et al., J. Biol. Chem. 272:10678-10684 (1997) and Rosok et al., J. Biol. Chem. 271:22611-22618 (1996)).

### Variants of Anti-NPR1 Antibodies

In certain embodiments, amino acid sequence variants of the anti-NPR1 antibodies provided herein are encompassed. For example, it may be desirable to improve the binding affinity and/or other biological properties of the antibodies. The amino acid sequence variants of the antibodies can be prepared by introducing appropriate modifications into the nucleotide sequences that encode the antibodies, or by peptide synthesis. Such modifications include, for example, deletions and/or insertions and/or substitutions of residues within the amino acid sequences of the anti-NPR1 antibodies. Any combination of deletions, insertions, and substitutions can be made to obtain the final construct, as long as the final construct possesses the desired characteristics, e.g., antigen binding properties.

### Substitution, Insertion, and Deletion Variants

In certain embodiments, antibody variants comprising one or more amino acid substitutions are provided. Positions of interest for substitutional mutagenesis include CDRs and FRs. Conservative substitutions are shown in Table 2 under the heading "preferred substitution". More substantial changes are provided in Table 2 under the heading "exemplary substitution", and as further described below with reference to amino acid side chain classes. Amino acid substitutions can be introduced into an antibody of interest, and the products are screened for a desired activity, e.g., retained/improved antigen binding, reduced immunogenicity, or improved ADCC or CDC.

**Table 2. Amino acid substitutions**

| Original residue | Exemplary substitution | Preferred substitution |
|---|---|---|
| Ala(A) | Val; Leu; Ile | Val |
| Arg(R) | Lys; Gln; Asn | Lys |
| Asn(N) | Gln; His; Asp,Lys; Arg | Gln |
| Asp(D) | Glu; Asn | Glu |
| Cys(C) | Ser; Ala | Ser |
| Gln(Q) | Asn; Glu | Asn |
| Glu(E) | Asp; Gln | Asp |
| Gly(G) | Ala | Ala |
| His(H) | Asn; Gln; Lys; Arg | Arg |
| Ile(I) | Leu; Val; Met; Ala; Phe; norleucine | Leu |
| Leu(L) | Norleucine; Ile; Val; Met; Ala; Phe | Ile |
| Lys(K) | Arg; Gln; Asn | Arg |
| Met(M) | Leu; Phe; Ile | Leu |
| Phe(F) | Trp; Leu; Val; Ile; Ala; Tyr | Tyr |
| Pro(P) | Ala | Ala |
| Ser(S) | Thr | Thr |
| Thr(T) | Ser | Ser |
| Trp(W) | Tyr; Phe | Tyr |
| Tyr(Y) | Trp; Phe; Thr; Ser | Phe |
| Val(V) | Ile; Leu; Met; Phe; Ala; norleucine | Leu |

According to common side-chain properties, amino acids can be grouped as follows:
(1) hydrophobic: norleucine, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

Non-conservative substitutions will involve substituting a member of one of these classes for a member of another class.

One type of substitution variant involves substituting one or more CDR residues of a parent antibody (e.g., a humanized or human antibody). Generally, the resulting variant selected for further study will have changes (e.g., improvements) in certain biological properties (e.g., increased affinity or reduced immunogenicity) relative to the parent antibody, and/or will have substantially retained certain biological properties of the parent antibody. An exemplary substitution variant is an affinity-matured antibody that can be conveniently produced, for example, using phage display-based affinity maturation techniques (e.g., those described herein). Briefly, one or more CDR residues are mutated, and the variant antibodies are displayed on phages and screened for a particular biological activity (e.g., binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VH or VL is tested for binding affinity. In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method for introducing diversity involves CDR-directed methods in which several CDR residues (e.g., 4-6 residues at a time) are randomized. CDR residues involved in antigen binding can be specifically identified, for example, using alanine scanning mutagenesis or modeling. Particularly, HCDR3 and LCDR3 are often targeted.

In some embodiments, substitutions, insertions, or deletions may occur within one or more CDRs, as long as such changes do not substantially reduce the ability of the antibody to bind to the antigen. For example, conservative changes (e.g., conservative substitutions as provided herein) that do not substantially reduce binding affinity may be made in CDRs. Such changes may be, for example, outside of the antigen-contacting residues in CDRs. In some embodiments of the variant VH and VL sequences provided above, each CDR is unaltered or contains no more than 1, 2, or 3 amino acid substitutions.

A useful method for identifying residues or regions of an antibody that can be used as mutagenesis targets is called "alanine scanning mutagenesis". In this method, a residue or a group of target residues (e.g., charged residues such as Arg, Asp, His, Lys, and Glu) is identified and replaced with a neutral or negatively charged amino acid (e.g., Ala or polyalanine) to determine whether the interaction of the antibody with the antigen is affected. Further substitutions may be introduced at amino acid locations that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include amino- and/or carboxyl-terminal fusions ranging in length from 1 residue to polypeptides containing 100 or more residues, and intrasequence insertions of single or multiple amino acid residues. Examples of terminal insertions include an antibody with an N-terminal methionyl residue. Other insertion variants of the antibody molecule include the fusion of the N- or C-terminus of the antibody to an enzyme or a polypeptide that increases the serum half-life of the antibody.

### Recombination Methods

Anti-NPR1 antibodies can be produced using recombinant methods. For these methods, one or more isolated nucleic acids encoding the anti-NPR1 antibodies are provided.

In some embodiments, the present disclosure provides isolated nucleic acids encoding the aforementioned anti-NPR1 antibodies. Such nucleic acids may each independently encode any one of the aforementioned polypeptide chains. In another aspect, the present disclosure provides one or more vectors (e.g., expression vectors) comprising such nucleic acids. In another aspect, the present disclosure provides a host cell comprising such nucleic acids. In some embodiments, provided is a method for preparing a polypeptide or fusion protein, wherein the method comprises culturing a host cell comprising a nucleic acid encoding the polypeptide or fusion protein, as provided above, under conditions suitable for expression, and optionally recovering the anti-NPR1 antibody from the host cell (or host cell culture medium).

For recombinant production of the anti-NPR1 antibody, the nucleic acid encoding the protein is isolated and inserted into one or more vectors for further cloning and/or expression in the host cell. Such nucleic acids can be readily isolated and sequenced using conventional procedures, or produced by recombination methods, or obtained by chemical synthesis.

Suitable host cells for cloning or expressing a vector encoding the anti-NPR1 antibody include the prokaryotic or eukaryotic cells described herein. For example, it can be produced in bacteria, particularly when glycosylation and Fc effector functions are not needed. After expression, it can be isolated from the bacterial cell paste in a soluble fraction and can be further purified.

In addition to prokaryotes, eukaryotic microorganisms such as filamentous fungi or yeast are suitable cloning or expression hosts for vectors encoding fusion proteins, including fungal and yeast strains. Suitable host cells for expression of the fusion protein may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US5959177, US 6040498, US6420548, US 7125978, and US6417429; vertebrate cells can also be used as hosts, e.g., mammalian cell lines adapted for growth in a suspension. Other examples of suitable mammalian host cell lines include an SV40-transformed monkey kidney CVlline (COS-7); a human embryonic kidney line (293 or 293T cells); baby hamster kidney cells (BHK); mouse Sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC 5 cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for antibody production, see, e.g., Yazaki, P. and Wu, A.M., Methods in Molecular Biology, Vol. 248, Lo, B.K.C. (eds.), Humana Press, Totowa, NJ (2004), pp. 255-268.

### Assays

The physical/chemical characteristics and/or biological activity of the anti-NPR1 antibodies provided herein can be identified, screened, or characterized through a variety of assays known in the art. In one aspect, the activity of the anti-NPR1 antibodies of the present disclosure is tested, for example, by known methods, such as ELISA and western blot.

### Treatment Method and Route of Administration

Any anti-NPR1 antibody provided by the present disclosure can be used in a treatment method. In yet another aspect, the present disclosure provides use of the anti-NPR1 antibody in the manufacture or preparation of a medicament. In some embodiments, the disease is an NPR1-related disease or disorder. In some embodiments, the disease or disorder is selected from the group consisting of heart failure, hypertension, peripheral vascular disease, coronary artery disease (CAD), ischemic heart disease (IHD), mitral stenosis and regurgitation, angina pectoris, hypertrophic cardiomyopathy (HCM), diabetic cardiomyopathy, supraventricular and ventricular cardiac arrhythmias, arrhythmia, atrial fibrillation (AF), new-onset atrial fibrillation, recurrent atrial fibrillation, cardiac fibrosis, atrial flutter, adverse vascular remodeling, plaque stabilization, myocardial infarction (MI), preeclampsia, obesity, renal failure, renal disorder, cytokine release syndrome, chronic kidney disease, macular edema, glaucoma, stroke, pulmonary disease, pulmonary fibrosis, inflammation, asthma, skeletal growth disorder, fracture, diabetes, and cancer; in some embodiments, the heart failure according to any one of the above is selected from the group consisting of heart failure with preserved ejection fraction (HFpEF), heart failure with reduced ejection fraction (HFrEF), heart failure with mildly reduced ejection fraction (HFmrEF), heart failure after acute myocardial infarction, and acute decompensated heart failure; in some embodiments, the hypertension according to any one of the above is resistant hypertension; in some embodiments, the heart failure according to any one of the above is heart failure with preserved ejection fraction (HFpEF).

In yet another aspect, provided is a pharmaceutical composition comprising the anti-NPR1 antibody, e.g., for use in any one of the above pharmaceutical uses or treatment methods. In some embodiments, the pharmaceutical composition comprises any anti-NPR1 antibody or antigen-binding fragment thereof provided herein and a pharmaceutically acceptable carrier. In some other embodiments, the pharmaceutical composition further comprises at least one additional therapeutic agent.

The anti-NPR1 antibody of the present disclosure may be used alone or in combination with other agents for treatment. For example, the antibody of the present disclosure can be co-administered with at least one additional therapeutic agent.

The anti-NPR1 antibody of the present disclosure (and any additional therapeutic agent) may be administered by any suitable means, including parenteral, intrapulmonary, and intranasal administration, and, if local treatment is needed, intralesional administration. Parenteral infusion includes intramuscular, intravenous, intra-arterial, intraperitoneal, or subcutaneous administration. Administration may be performed via any suitable route, e.g., by injection, such as intravenous or subcutaneous injection, depending in part on whether the administration is short-term or long-term. Various administration schedules are contemplated herein, including but not limited to single administration or multiple administrations at multiple time points, bolus injection administration, and pulse infusion.

The anti-NPR1 antibody of the present disclosure will be formulated, administered, and applied in a manner consistent with Good Medical Practice. Factors considered in this context include the specific disorder being treated, the specific mammal being treated, the clinical state of the individual patient, the cause of the disorder, the site of delivery of the agent, the administration method, the timing of administration, and other factors known to medical practitioners. The anti-NPR1 antibody can be formulated with or without one or more agents currently used for preventing or treating the disorder. The effective amount of such additional agents depends on the amount present in the pharmaceutical composition, the type of the disorder or treatment, and other factors. These are generally used in the same dosages and routes of administration as described herein, or in about 1% to 99% of the dosages described herein, or in other dosages, and by any route empirically/clinically determined to be appropriate.

For the prevention or treatment of a disease, the appropriate dose of the anti-NPR1 antibody of the present disclosure (when used alone or in combination with one or more other additional therapeutic agents) will depend on the type of the disease to be treated, the type of the therapeutic molecule, the severity and course of the disease, whether the therapeutic molecule is administered for preventive or therapeutic purposes, previous therapy, the patient's clinical history and response to the therapeutic molecule, and the discretion of the attending physician. The therapeutic molecule is suitably administered to a patient in one or a series of treatments.

### Product

In another aspect of the present disclosure, provided is a product (e.g., a medication box) comprising materials useful for the treatment, prevention, and/or diagnosis of the above disorder. The product comprises a container and a label or package insert on or associated with the container. Suitable containers include, for example, bottles, vials, syringes, IV solution bags, and the like. The container may be formed of a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper pierceable by a hypodermic injection needle). At least one active agent in the composition is the anti-NPR1 antibody of the present disclosure. The label or package insert indicates that the composition is used to treat the selected condition. In addition, the product can comprise: (a) a first container containing a composition, wherein the composition comprises the anti-NPR1 antibody of the present disclosure; and (b) a second container containing a composition, wherein the composition comprises an additional therapeutic agent. The product in this embodiment of the present disclosure may further comprise a package insert indicating that the composition may be used to treat a particular condition. Alternatively or additionally, the product may further comprise a second (or third) container containing a pharmaceutically acceptable buffer. From a commercial and user standpoint, it may further comprise other required materials, including other buffers, diluents, filters, needles, and syringes.

The present disclosure is further described below with reference to examples and test examples. However, these examples and test examples do not limit the scope of the present disclosure. In the examples or test examples of the present disclosure, the experimental methods whose specific conditions are not specified were generally performed under conventional conditions, such as those described in Antibodies: A Laboratory Manual and Molecular Cloning: A Laboratory Manual by Cold Spring Harbor Laboratory, or under conditions recommended by the manufacturer of the starting material or commercial product. The reagents whose specific sources are not specified were commercially available.

### Examples

### Example 1: Preparation of NPR1 Antigens

With UniProt or NCBI NPR1 antigens (human NPR1 protein, Uniprot No. P16066; cynomolgus monkey NPR1 protein, NCBI No. XP-005541809.1; rat NPR1 protein, Uniprot No. P18910) used as templates for NPR1, the amino acid sequences of the antigens and proteins for detection used in the present disclosure were designed, and optionally, fusion tags such as hFc were added to the NPR1 proteins. After cloning into pTT5 vectors (Biovector, CAT#102762), CHO cells were transiently transfected with the vectors. After expression and purification, the antigens and proteins for detection of the present disclosure were obtained.

The sequence of a fusion protein of the human NPR1 protein extracellular domain and human-IgG1-Fc (abbreviated as hNPR1-hFc) was used as an immunization antigen: 
GNLTVAVVLPLANTSYPWSWARVGPAVELALAQVKARPDLLPGWTVRTVLGSSENALG
VCSDTAAPLAAVDLKWEHNPAVFLGPGCVYAAAPVGRFTAHWRVPLLTAGAPALGFGV
KDEYALTTRAGPSYAKLGDFVAALHRRLGWERQALMLYAYRPGDEEHCFFLVEGLFMR
VRDRLNITVDHLEFAEDDLSHYTRLLRTMPRKGRVIYICSSPDAFRTLMLLALEAGLCGE
DYVFFHLDIFGQSLQGGQGPAPRRPWERGDGQDVSARQAFQAAKIITYKDPDNPEYLEF
LKQLKHLAYEQFNFTMEDGLVNTIPASFHDGLLLYIQAVTETLAHGGTVTDGENITQRM
WNRSFQGVTGYLKIDSSGDRETDFSLWDMDPENGAFRVVLNYNGTSQELVAVSGRKLN
WPLGYPPPDIPKCGFDNEDPACNQDHLSTLEEPKSSDKTHTCPPCPAPELLGGPSVFLFPP
KPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGN
VFSCSVMHEALHNHYTQKSLSLSPGK
SEQ ID NO: 1
 Note: Human-IgG1-Fc is underlined, and the portion that is not underlined is the human NPR1 protein extracellular domain.

The sequence of a fusion protein of the cynomolgus monkey NPR1 protein extracellular domain and human-IgG1-Fc (abbreviated as cNPR1-hFc) was used as an immunization antigen: 
GNLTVAVVLPLANTSYPWSWARVGPAVELALARVKARPDLLPGWTVRTVLGSSENALG
VCSDTAAPLAAVDLKWEHNPAAFLGPGCVYAAAPVGRFTAHWRVPLLTAGAPALGFGV
KDEYALTTRAGPSYAKLGDFVAALHRRLGWERQALMLYAYRPGDEEHCFFLVEGLFMR
VRDRLNITVDHLEFAEDDLSHYTRLLRTMPRKGRVIYICSSPDAFRTLMLLALEAGLCGE
DYVFFHLDIFGQSLQGGQGPAPRRPWERGDGQDVSARQAFQAAKIITYKEPDNPEYLEF
LKQLKHLAREQFNFTMEDGLVNTIPASFHDGLLLYIQAVTETLAHGGTVTDGENITQRM
WNRSFQGVTGYLKIDSSGDRETDFSLWDMDPETGAFRVVLNYNGTSQELVAVSGRKLN
WPLGYPPPDIPKCGFDNEDPACNQDHLSTLEEPKSSDKTHTCPPCPAPELLGGPSVFLFPP
KPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGN
VFSCSVMHEALHNHYTQKSLSLSPGK
SEQ ID NO: 2
 Note: Human-IgG1-Fc is underlined, and the portion that is not underlined is the cynomolgus monkey NPR1 protein extracellular domain.

The sequence of a fusion protein of the rat (Rattus norvegicus) NPR1 protein extracellular domain and human-IgG1-Fc (abbreviated as rNPR1-hFc) was used as an immunization antigen: 
SDLTVAVVLPLTNTSYPWSWARVGPAVELALARVKARPDLLPGWTVRMVLGSSENAAG
VCSDTAAPLAAVDLKWEHSPAVFLGPGCVYSAAPVGRFTAHWRVPLLTAGAPALGIGVK
DEYALTTRTGPSHVKLGDFVTALHRRLGWEHQALVLYADRLGDDRPCFFIVEGLYMRVR
ERLNITVNHQEFVEGDPDHYPKLLRAVRRKGRVIYICSSPDAFRNLMLLALNAGLTGEDY
VFFHLDVFGQSLKSAQGLVPQKPWERGDGQDRSARQAFQAAKIITYKEPDNPEYLEFLK
QLKLLADKKFNFTVEDGLKNIIPASFHDGLLLYVQAVTETLAQGGTVTDGENITQRMWN
RSFQGVTGYLKIDRNGDRDTDFSLWDMDPETGAFRVVLNYNGTSQELMAVSEHKLYWP
LGYPPPDVPKCGFDNEDPACNQDHFSTLEEPKSSDKTHTCPPCPAPELLGGPSVFLFPPKP
KDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFS
CSVMHEALHNHYTQKSLSLSPGK
SEQ ID NO: 3
 Note: Human-IgG1-Fc is underlined, and the portion that is not underlined is the rat NPR1 protein extracellular domain.

### Example 2: Purification of NPR1-Related Recombinant Proteins

### Purification of NPR1-Fc fusion proteins:

The cell expression supernatant sample was centrifuged at high speed to remove impurities, and the supernatant was purified by MabSelect Sure (GE, 17-5438-01) affinity chromatography. The MabSelect Sure chromatography column was first regenerated with 0.2 M NaOH, rinsed with purified water, and then equilibrated with PBS. After the supernatant was bound, the column was washed with PBS until the A₂₈₀ reading dropped to the baseline. The target protein was eluted with a 0.1 M acetic acid buffer at pH 3.5 and neutralized with 1 M pH 8.0 Tris-HCl. The eluted sample was appropriately concentrated and further purified using a gel chromatography column Superdex200 (GE, 28-9893-35) equilibrated with PBS, and the target protein was collected in a receiving tube and concentrated to an appropriate concentration. This method was used to purify the NPR1-Fc fusion proteins. This method can also be used to purify the antibody proteins in the present disclosure.

### Example 3: Preparation of NPR1 Recombinant Cell Lines

With UniProt or NCBI NPR1 antigens (human NPRl protein, Uniprot No. P16066; cynomolgus monkey NPR1 protein, NCBI No. XP-005541809.1; rat NPR1 protein, Uniprot No. P18910) used as templates for NPR1, public recombinant cell lines for immunization and detection were designed; the recombinant cell line for immunization was NIH-3T3 cells overexpressing full-length proteins of human NPR1, cynomolgus monkey NPR1, and rat NPR1, and the recombinant cell line for detection was a CHO-K1 cell line overexpressing full-length proteins of human NPR1, cynomolgus monkey NPR1, and rat NPR1.
1. Full-length amino acid sequence of human NPR1 
MPGPRRPAGSRLRLLLLLLLPPLLLLLRGSHAGNLTVAVVLPLANTSYPWSWARVGPAVE
LALAQVKARPDLLPGWTVRTVLGSSENALGVCSDTAAPLAAVDLKWEHNPAVFLGPGC
VYAAAPVGRFTAHWRVPLLTAGAPALGFGVKDEYALTTRAGPSYAKLGDFVAALHRRLG
WERQALMLYAYRPGDEEHCFFLVEGLFMRVRDRLNITVDHLEFAEDDLSHYTRLLRTMP
RKGRVIYICSSPDAFRTLMLLALEAGLCGEDYVFFHLDIFGQSLQGGQGPAPRRPWERGD
GQDVSARQAFQAAKIITYKDPDNPEYLEFLKQLKHLAYEQFNFTMEDGLVNTIPASFHD
GLLLYIQAVTETLAHGGTVTDGENITQRMWNRSFQGVTGYLKIDSSGDRETDFSLWDMD
PENGAFRVVLNYNGTSQELVAVSGRKLNWPLGYPPPDIPKCGFDNEDPACNQDHLSTLE
VLALVGSLSLLGILIVSFFIYRKMQLEKELASELWRVRWEDVEPSSLERHLRSAGSRLTLS
GRGSNYGSLLTTEGQFQVFAKTAYYKGNLVAVKRVNRKRIELTRKVLFELKHMRDVQNE
HLTRFVGACTDPPNICILTEYCPRGSLQDILENESITLDWMFRYSLTNDIVKGMLFLHNGA
ICSHGNLKSSNCVVDGRFVLKITDYGLESFRDLDPEQGHTVYAKKLWTAPELLRMASPP
VRGSQAGDVYSFGIILQEIALRSGVFHVEGLDLSPKEIIERVTRGEQPPFRPSLALQSHLEE
LGLLMQRCWAEDPQERPPFQQIRLTLRKFNRENSSNILDNLLSRMEQYANNLEELVEERT
QAYLEEKRKAEALLYQILPHSVAEQLKRGETVQAEAFDSVTIYFSDIVGFTALSAESTPM
QVVTLLNDLYTCFDAVIDNFDVYKVETIGDAYMVVSGLPVRNGRLHACEVARMALALL
DAVRSFRIRHRPQEQLRLRIGIHTGPVCAGVVGLKMPRYCLFGDTVNTASRMESNGEAL
KIHLSSETKAVLEEFGGFELELRGDVEMKGKGKVRTYWLLGERGSSTRG
SEQ ID NO: 4
 Note:
   Human NPR1 is a transmembrane protein that exists as a homodimer on the cell membrane surface:
   the extracellular domain of NPR1 (33 to 473) is double-underlined;
   the transmembrane domain (474 to 494) is underlined with a dotted line;
   the cytoplasmic domain (495 to 1061) is single-underlined;
   the portion that is not underlined is a signal peptide.
2. Full-length amino acid sequence of cynomolgus monkey NPR1 
MPGPRRPAGSRLRLLLLLLLPPLLLLLRGSHAGNLTVAVVLPLANTSYPWSWARVGPAVE
LALARVKARPDLLPGWTVRTVLGSSENALGVCSDTAAPLAAVDLKWEHNPAAFLGPGC
VYAAAPVGRFTAHWRVPLLTAGAPALGFGVKDEYALTTRAGPSYAKLGDFVAALHRRLG
WERQALMLYAYRPGDEEHCFFLVEGLFMRVRDRLNITVDHLEFAEDDLSHYTRLLRTMP
RKGRVIYICSSPDAFRTLMLLALEAGLCGEDYVFFHLDIFGQSLQGGQGPAPRRPWERGD
GQDVSARQAFQAAKIITYKEPDNPEYLEFLKQLKHLAREQFNFTMEDGLVNTIPASFHDG
LLLYIQAVTETLAHGGTVTDGENITQRMWNRSFQGVTGYLKIDSSGDRETDFSLWDMDP
ETGAFRVVLNYNGTSQELVAVSGRKLNWPLGYPPPDIPKCGFDNEDPACNQDHLSTLEV
LALVGSLSLLSILIVSFFIYRKMQLEKELASELWRVRWEDVEPSSLERHLRSAGSRLTLSG
RGSNYGSLLTTEGQFQVFAKTAYYKGNLVAVKRVNRKRIELTRKVLFELKHMRDVQNEH
LTRFVGACTDPPNICILTEYCPRGSLQDILENESITLDWMFRYSLTNDIVKGMLFLHNGAI
CSHGNLKSSNCVVDGRFVLKITDYGLESFRDLDPEQGHTLYAKKLWTAPELLRMASPPV
RGSQAGDIYSFGIILQEIALRSGVFHVEGLDLSPKEIIERVTRGEQPPFRPSLALQSHLEELG
LLMQRCWAEDPQERPPFQQIRLTLRKFNRENSSNILDNLLSRMEQYANNLEELVEERTQA
YLEEKRKAEALLYQILPHSVAEQLKRGETVQAEAFDSVTIYFSDIVGFTALSAESTPMQV
VTLLNDLYTCFDAVIDNFDVYKVETIGDAYMVVSGLPVRNGRLHACEVARMALALLDA
VRSFRIRHRPQEQLRLRIGIHTGPVCAGVVGLKMPRYCLFGDTVNTASRMESNGEALKIH
LSSETKAVLEEFGGFELELRGDVEMKGKGKVRTYWLLGERGSSTRG
SEQ ID NO: 5
 Note:
   Cyno NPR1 is a transmembrane protein that exists as a homodimer on the cell membrane surface:
   the extracellular domain of NPR1 (33 to 473) is double-underlined;
   the transmembrane domain (474 to 494) is underlined with a dotted line;
   the cytoplasmic domain (495 to 1061) is single-underlined;
   the portion that is not underlined is a signal peptide.
3. Full-length amino acid sequence of rat (Rattus norvegicus) NPR1 
MPGSRRVRPRLRALLLLPPLLLLRGGHASDLTVAVVLPLTNTSYPWSWARVGPAVELALA
RVKARPDLLPGWTVRMVLGSSENAAGVCSDTAAPLAAVDLKWEHSPAVFLGPGCVYSA
APVGRFTAHWRVPLLTAGAPALGIGVKDEYALTTRTGPSHVKLGDFVTALHRRLGWEHQ
ALVLYADRLGDDRPCFFIVEGLYMRVRERLNITVNHQEFVEGDPDHYPKLLRAVRRKGR
VIYICSSPDAFRNLMLLALNAGLTGEDYVFFHLDVFGQSLKSAQGLVPQKPWERGDGQD
RSARQAFQAAKIITYKEPDNPEYLEFLKQLKLLADKKFNFTVEDGLKNIIPASFHDGLLLY
VQAVTETLAQGGTVTDGENITQRMWNRSFQGVTGYLKIDRNGDRDTDFSLWDMDPET
GAFRVVLNYNGTSQELMAVSEHKLYWPLGYPPPDVPKCGFDNEDPACNQDHFSTLEVL
ALVGSLSLISFLIVSFFIYRKMQLEKELVSELWRVRWEDLQPSSLERHLRSAGSRLTLSGR
GSNYGSLLTTEGQFQVFAKTAYYKGNLVAVKRVNRKRIELTRKVLFELKHMRDVQNEHL
TRFVGACTDPPNICILTEYCPRGSLQDILENESITLDWMFRYSLTNDIVKGMLFLHNGAIC
 
SHGNLKSSNCVVDGRFVLKITDYGLESFRDPEPEQGHTLFAKKLWTAPELLRMASPPAR
GSQAGDVYSFGIILQEIALRSGVFYVEGLDLSPKEIIERVTRGEQPPFRPSMDLQSHLEELG
QLMQRCWAEDPQERPPFQQIRLALRKFNKENSSNILDNLLSRMEQYANNLEELVEERTQ
AYLEEKRKAEALLYQILPHSVAEQLKRGETVQAEAFDSVTIYFSDIVGFTALSAESTPMQ
VVTLLNDLYTCFDAVIDNFDVYKVETIGDAYMVVSGLPVRNGQLHAREVARMALALLD
AVRSFRIRHRPQEQLRLRIGIHTGPVCAGVVGLKMPRYCLFGDTVNTASRMESNGEALKI
HLSSETKAVLEEFDGFELELRGDVEMKGKGKVRTYWLLGERGCSTRG
SEQ ID NO: 6
 Note:
   Rat NPR1 is a transmembrane protein that exists as a homodimer on the cell membrane surface:
   the extracellular domain of NPR1 (29 to 469) is double-underlined;
   the transmembrane domain (470 to 490) is underlined with a dotted line;
   the cytoplasmic domain (491 to 1057) is single-underlined;
   the portion that is not underlined is a signal peptide.

NIH-3T3 cells were stably transfected with the above proteins. After two weeks of pressure screening, subcloning was performed to screen for single clones, and recombinant cell lines highly expressing hNPR1-NIH-3T3, cNPR1-NIH-3T3, and rNPR1-NIH-3T3 were obtained by FACS and used for immunizing mice.

CHO-K1 cells were stably transfected with the above proteins. After two weeks of pressure screening, subcloning was performed to screen for single clones, and recombinant cell lines highly expressing hNPR1-CHO-K1, cNPR1-CHO-K1, and rNPR1-CHO-K1 were obtained by FACS and used for assessing the affinity and *in vitro* function of anti-NPR1 antibodies.

### Example 4: Screening for Murine Anti-hNPR1 Antibodies

### 1. Mouse immunization:

Anti-human NPR1 monoclonal antibodies were produced by immunization of mice. Laboratory SJL white mice, female, aged 6-8 weeks (Shanghai SLAC Laboratory Animal Co., Ltd., animal production license number: SCXK (Shanghai) 2017-0005). Housing environment: SPF. The purchased mice were housed for 1 week in a laboratory environment with a 12/12-hour light/dark cycle at a temperature of 20-25 °C with humidity at 40-60%. The acclimatized mice were immunized according to scheme 1 and scheme 2. The immunization antigens were hNPR1 NIH-3T3, cNPR1 NIH-3T3, rNPR1 NIH-3T3, hNPR1-hFc, cNPR1-hFc, rNPR1-hFc, and hANP (1-28 aa, GenScript Biotech Corporation, Cat. No. RP11927).

Immunization scheme 1: Each mouse was pre-immunized with TiterMax^{®} Gold Adjuvant (Sigma Cat. No. T2684) by intraperitoneal (IP) injection of 0.1 mL (first immunization). After 15 min, each mouse was intraperitoneally (IP) injected with 1 × 10⁷ hNPR1 NIH-3T3 cells. Cross-immunization was performed with antigens hNPR1 NIH-3T3 and cNPR1 NIH-3T3. Inoculation was performed on day 0, day 14, and day 28. Blood was collected on day 21 and day 35, and the antibody titer in mouse serum was determined by ELISA and FACS. After the third immunization, mice in which the antibody titer in serum was high and tended to plateau were selected for splenocyte fusion. Boost immunization was performed 3 days before splenocyte fusion by intraperitoneally (IP) injecting 50 µg of a solution of antigen hNPR1-hFc in normal saline into each mouse. 5 µg of hANP (1-28 aa) was added per mouse, per immunization inoculation. Immunization scheme 2: Each mouse was pre-immunized with TiterMax^{®} Gold Adjuvant (Sigma Cat. No. T2684) by intraperitoneal (IP) injection of 0.1 mL (first immunization). After 15 min, each mouse was intraperitoneally (IP) injected with 1 × 10^7 hNPR1 NIH-3T3 cells. Cross-immunization was performed with antigens hNPR1 NIH-3T3 and rNPR1 NIH-3T3. Inoculation was performed on day 0, day 14, day 28, day 42, day 56, day 70, day 84, and day 98. Blood was collected on day 35, day 63, day 91, and day 105, and the antibody titer in mouse serum was determined by ELISA and FACS. After the eighth immunization, mice in which the antibody titer in serum was high and tended to plateau were selected for splenocyte fusion. Boost immunization was performed 3 days before splenocyte fusion by intraperitoneally (IP) injecting 50 µg of a mixed solution of antigens hNPR1-hFc, cNPR1-hFc, and rNPR1-hFc in normal saline into each mouse. 5 µg of hANP (1-28 aa) was added per mouse, per immunization inoculation.

### 2. Splenocyte fusion:

Spleen lymphocytes and Sp2/0 cells (myeloma cells, ATCC^{®} CRL-8287^{™}) were fused using an optimized electrofusion method to obtain hybridoma cells.

The resulting hybridoma cells were resuspended in a complete culture medium (an IMDM culture medium containing 20% FBS, 1× HAT, and 1× OPI) at a density of 3 × 10⁵ to 4 × 10⁵ cells/mL according to the spleen cell count, and the suspension was seeded in a 96-well plate at 150 µL/well. After 4-5 days of incubation at 37 °C with 5% CO₂, the supernatant was removed, and an HT complete culture medium (an IMDM culture medium containing 20% FBS, 1× HT, and 1× OPI) was added at 200 µL/well. After 2 days of culture at 37 °C with 5% CO₂, an ELISA was performed.

### 3. Screening of hybridoma cells:

Hybridoma culture supernatant assays were performed using a cell-based binding assay and a cGMP production assay according to the growth density of hybridoma cells. The cells in positive wells were expanded and cryopreserved in time and subcloned once or twice until single cell clones were obtained. The single cell clones were subjected to expansion culture, and RNA was extracted. Reverse transcription amplification (RT-PCR) was performed using a degenerate primer of mouse-Ig to obtain the variable region sequences of antibodies. The obtained amino acid sequences corresponding to the DNA sequences of murine antibodies 102, 127, 128, 135, and 140 are shown below:
NPR1-CHAb-SFM-102-1
   HCVR: 
DVKLVESGEGLVKPGGSLKLSCAASGFTFSTYAMSWVRQTPEKRLEWVAYISRGGDYIY
YADTVKGRFTISRDNARNTLYLQMSSLKSEDTAMYYCTRDQTTVVVHWYFDVWGTGT
TVTVSS
SEQ ID NO: 7
   LCVR: QIVLTQSPAIMSASPGEKVTMTCSASSSINYMHWYQQKPGTSPKRWIYDTSKLASGVPAR 
FSGSGSGTSYSLTISSMEAGDAATYYCHQRNSYTFGGGTKLEIK
SEQ ID NO: 8
NPR1-CHAb-SFM-127-1
   HCVR: 
QAYLQQSGPELVKPGASVKISCKASGYSFTGYFMNWVMQSHGKSLEWIGRINPYNGDT
FYNQKFKGKATLTVDKSSSTVHMELRSLASEDSAVYYCARRGYGNRGYWYFDVWGAG
TTVTVSS
SEQ ID NO: 9
   LCVR: 
NIVLTQSPASLAVSLGQRATISCRASESVDSYGNSFMHWYQQKPGQPPKVLIYLASTLESG
VPARFSGSGSRTDFTLTIDPVEADDAATYYCQQNNEDPLTFGAGTKLELK
SEQ ID NO: 10
NPR1-CHAb-SFM-128
   HCVR: 
QVQLQQSGAELAKPGASVKLSCKASGYTFTSYWMHWVKQRPGRGLEWIGRIDPNSGV
TKYNEKFKSKATLTVDKPSSPAYMQLSSLTSEDSAVYYCARSSYLGRYFDVWGTGTTVT
VSS
SEQ ID NO: 11
   LCVR: 
QIVLTQSPAIMSASPGEKVTMTCSATSSVSYMYWYQQKPGSSPRLLIYDTYNLASRVPIRF
SGSGSGTSYSLTISRMEΛEDΛΛTYYCQQWSSYPPTFGSGTKLEIK
SEQ ID NO: 12
NPR1-CHAb-SFM-135
   HCVR: 
DVKLVESGEGLVKPGGSLKLSCVASGFSISTYGMSWVRQTPEKRLEWVAYINSGGNYIY
YADTVKGRFTISRDNARNTLYLQMSSLKSEDTAMYYCTRDRTTIVVHWYFDVWGTGTT
VTVSS
SEQ ID NO: 13
   LCVR: 
QIVLTQSPAIMSASPGEKVTMTCSASSSLNFMHWYQQKPGTSPKRWIYDTSKLASGVPAR
FSGSGSGTSYSLTISSMEAEDAATYYCHQRSSTFGGGTKLELK
SEQ ID NO: 14
NPR1-CHAb-SFM-140
   HCVR: 
QVQLQQSGAELVKPGASVKLSCKASGYTFTSYWMHWVKQRPGRGLEWIGRIDPNSGVT
KYNEKFKSKVTLTVDKPSSPAYIQLSSLTSEDSAVYYCARSGGLRRYFDVWGTGTTVTVS
S
SEQ ID NO: 15
   LCVR: 
QIVLTQSPAIMSASPGEKVTMTCSASSSVSYMYWYQQKPGSSPRLLIFDTYNLASGVPVR
FSGSGSGTSYSLTINRMEAEDAATYYCQQWSSYPPTFGAGTKLELK
 SEQ ID NO: 16
Note: In the above sequences, the regions are arranged in the following order: FR1-CDR1-FR2-CDR2-FR3-CDR3-FR4; the CDR sequences determined according to the Kabat numbering scheme are double-underlined.

The CDR sequences of the heavy and light chains of murine antibodies 102, 127, 128, 135, and 140 are shown in Table 3 below:

**Table 3. The CDR sequences of the heavy and light chains of antibodies**

| Antibody | Heavy chain | | Light chain | |
|---|---|---|---|---|
| 102 | HCDR1 | TYAMS (SEQ ID NO: 17) | LCDR1 | SASSSINYMH (SEQ ID NO:20) |
| | HCDR2 | YISRGGDYIYYADTVKG (SEQ ID NO:18) | LCDR2 | DTSKLAS (SEQ ID NO:21) |
| | HCDR3 | DQTTVVVHWYFDV (SEQ ID NO:19) | LCDR3 | HQRNSYT (SEQ ID NO:22) |
| 127 | HCDR1 | GYFMN (SEQ ID NO:23) | LCDR1 | RASESVDSYGNSFMH (SEQ ID NO:26) |
| | HCDR2 | RINPYNGDTFYNQKFKG (SEQ ID NO:24) | LCDR2 | LASTLES (SEQ ID NO:27) |
| | HCDR3 | RGYGNRGYWYFDV (SEQ ID NO:25) | LCDR3 | QQNNEDPLT (SEQ ID NO:28) |
| 128 | HCDR1 | SYWMH (SEQ ID NO:29) | LCDR1 | SATSSVSYMY (SEQ ID NO:32) |
| | HCDR2 | RIDPNSGVTKYNEKFKS (SEQ ID NO:30) | LCDR2 | DTYNLAS (SEQ ID NO:33) |
| | HCDR3 | SSYLGRYFDV (SEQ ID NO:31) | LCDR3 | QQWSSYPPT (SEQ ID NO:34) |
| 135 | HCDR1 | TYGMS (SEQ ID NO:35) | LCDR1 | SASSSLNFMH (SEQ ID NO:38) |
| | HCDR2 | YINSGGNYIYYADTVKG (SEQ ID NO:36) | LCDR2 | DTSKLAS (SEQ ID NO:21) |
| | HCDR3 | DRTTIVVHWYFDV (SEQ ID NO:37) | LCDR3 | HQRSST (SEQ ID NO:39) |
| 140 | HCDR1 | SYWMH (SEQ ID NO:29) | LCDR1 | SASSSVSYMY (SEQ ID NO:42) |
| | HCDR2 | RIDPNSGVTKYNEKFKS (SEQ ID NO:40) | LCDR2 | DTYNLAS (SEQ ID NO:33) |
| | HCDR3 | SGGLRRYFDV | LCDR3 | QQWSSYPPT |
| | | (SEQ ID NO:41) | | (SEQ ID NO:34) |

| | | | | |
|---|---|---|---|---|
| Note: The CDRs in the table are CDRs determined according to the Kabat numbering scheme. | | | | |

By linking the carboxy-termini of the heavy chain variable regions of the murine antibodies 102, 127, 128, 135, and 140 obtained above to the amino-terminus of a human heavy chain constant region set forth in SEQ ID NO: 43, and linking the carboxy-termini of the light chain variable regions of the murine antibodies to the amino-terminus of a human light chain constant region set forth in SEQ ID NO: 44, chimeric antibodies corresponding to 102, 127, 128, 135, and 140 can be obtained. These chimeric antibodies were denoted by CHI-102, CHI-127, CHI-128, CHI-135, and CHI-140, respectively.
Construction of human IgG1 heavy chain constant region sequence for chimeric antibodies: 






SEQ ID NO: 43
Construction of human light chain constant region sequence for chimeric antibodies (human kappa light chain constant region): 


SEQ ID NO: 44
Full-length heavy chain sequence of CHI-102 (NPR1-CHAb-SFM-102-1): 
DVKLVESGEGLVKPGGSLKLSCAASGFTFSTYAMSWVRQTPEKRLEWVAYISRGGDYIY
YADTVKGRFTISRDNARNTLYLQMSSLKSEDTAMYYCTRDQTTVVVHWYFDVWGTGT
TVTVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSV
MHEALHNHYTQKSLSLSPGK
SEQ ID NO: 102
Full-length light chain sequence of CHI-102 (NPR1-CHAb-SFM-102-1): 
QIVLTQSPAIMSASPGEKVTMTCSASSSINYMHWYQQKPGTSPKRWIYDTSKLASGVPAR
FSGSGSGTSYSLTISSMEAGDAATYYCHQRNSYTFGGGTKLEIKRTVAAPSVFIFPPSDEQL
KSGTASVVCLLNNFYPREAKVQWKVDNALQSGNSQESVTEQDSKDSTYSLSSTLTLSKADYEK
HKVYACEVTHQGLSSPVTKSFNRGEC
SEQ ID NO: 103
Full-length heavy chain sequence of CHI-127 (NPR1-CHAb-SFM-127-1): 
QAYLQQSGPELVKPGASVKISCKASGYSFTGYFMNWVMQSHGKSLEWIGRINPYNGDT
FYNQKFKGKATLTVDKSSSTVHMELRSLASEDSAVYYCARRGYGNRGYWYFDVWGAG
TTVTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELLGGP
SVFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCS
VMHEALHNHYTQKSLSLSPGK
SEQ ID NO: 104
Full-length light chain sequence of CHI-127 (NPR1-CHAb-SFM-127-1): 
NIVLTQSPASLAVSLGQRATISCRASESVDSYGNSFMHWYQQKPGQPPKVLIYLASTLESG
VPARFSGSGSRTDFTLTIDPVEADDAATYYCQQNNEDPLTFGAGTKLELKRTVAAPSVFIF
PPSDEQLKSGTASVVCLLNNFYPREAKVQWKVDNALQSGNSQESVTEQDSKDSTYSLSSTLTLS
KADYEKHKVYACEVTHQGLSSPVTKSFNRGEC
SEQ ID NO: 105
Full-length heavy chain sequence of CHI-128 (NPR1-CHAb-SFM-128): 
QVQLQQSGAELAKPGASVKLSCKASGYTFTSYWMHWVKQRPGRGLEWIGRIDPNSGV
TKYNEKFKSKATLTVDKPSSPAYMQLSSLTSEDSAVYYCARSSYLGRYFDVWGTGTTVT
VSSASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMH
EALHNHYTQKSLSLSPGK
SEQ ID NO: 106
Full-length light chain sequence of CHI-128 (NPR1-CHAb-SFM-128): 
QIVLTQSPAIMSASPGEKVTMTCSATSSVSYMYWYQQKPGSSPRLLIYDTYNLASRVPIRF
SGSGSGTSYSLTISRMEAEDAATYYCQQWSSYPPTFGSGTKLEIKRTVAAPSVFIFPPSDEQ
LKSGTASVVCLLNNFYPREAKVQWKVDNALQSGNSQESVTEQDSKDSTYSLSSTLTLSKADYEK
HKVYACEVTHQGLSSPVTKSFNRGEC
SEQ ID NO: 107
Full-length heavy chain sequence of CHI-135 (NPR1-CHAb-SFM-135): 
DVKLVESGEGLVKPGGSLKLSCVASGFSISTYGMSWVRQTPEKRLEWVAYINSGGNYIY
YADTVKGRFTISRDNARNTLYLQMSSLKSEDTAMYYCTRDRTTIVVHWYFDVWGTGTT
VTVSSASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGK
 SEQ ID NO: 108
Full-length light chain sequence of CHI-135 (NPR1-CHAb-SFM-135): 
QIVLTQSPAIMSASPGEKVTMTCSASSSLNFMHWYQQKPGTSPKRWIYDTSKLASGVPAR
FSGSGSGTSYSLTISSMEAEDAATYYCHQRSSTFGGGTKLELKRTVAAPSVFIFPPSDEQLK
SGTASVVCLLNNFYPREAKVQWKVDNALQSGNSQESVTEQDSKDSTYSLSSTLTLSKADYEKH
KVYACEVTHQGLSSPVTKSFNRGEC
SEQ ID NO: 109
Full-length heavy chain sequence of CHI-140 (NPR1-CHAb-SFM-140): 
QVQLQQSGAELVKPGASVKLSCKASGYTFTSYWMHWVKQRPGRGLEWIGRIDPNSGVT
KYNEKFKSKVTLTVDKPSSPAYIQLSSLTSEDSAVYYCARSGGLRRYFDVWGTGTTVTVS
SASTKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLFPP
KPKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSCSVMHEALH
NHYTQKSLSLSPGK
SEQ ID NO: 110
Full-length light chain sequence of CHI-140 (NPR1-CHAb-SFM-140):

QIVLTQSPAIMSASPGEKVTMTCSASSSVSYMYWYQQKPGSSPRLLIFDTYNLASGVPVR
FSGSGSGTSYSLTINRMEAEDAATYYCQQWSSYPPTFGAGTKLELKRTVAAPSVFIFPPSD
EQLKSGTASVVCLLNNFYPREAKVQWKVDNALQSGNSQESVTEQDSKDSTYSLSSTLTLSKADY
EKHKVYACEVTHQGLSSPVTKSFNRGEC
SEQ ID NO: 111

### 4. FACS binding assay for anti-NPR1 chimeric antibodies:

An *in vitro* binding assay was performed to confirm that the variable region sequences of the obtained monoclonal antibodies were correct. After hNPR1-expressing CHO-K1 cells or cNPR1-expressing CHO-K1 cells (1 × 10⁶ cells/mL) were blocked with a 1% BSA PBS buffer, anti-NPR1 chimeric antibody samples diluted to different concentrations (pre-mixed with 10 nM ANP when the *in vitro* binding assay was performed in the presence of ANP) were added, and the cells were incubated for 1 h. After the cells were washed twice with PBS (pH 7.4), APC anti-human IgG (Biolegend Cat. No. 409306) was added, and the cells were incubated for 45 min. After the cells were washed twice with PBS (pH 7.4), they were resuspended in 150 µL of PBS (pH 7.4), and fluorescence signal values were read using a flow cytometer. The experimental results are shown in Table 4.

**Table 4. FACS binding of anti-NPR1 chimeric antibodies**

| | hNPR1-CHOK1 | | | | cNPR1-CHOK1 | | | |
|---|---|---|---|---|---|---|---|---|
| | ANP- | | ANP+ | | ANP- | | ANP+ | |
| | Emax | EC₅₀ (nM) | Emax | EC₅₀ (nM) | Emax | EC₅₀ (nM) | Emax | EC₅₀ (nM) |
| CHI-127 | 64506.00 | 1.73 | 64246.00 | 1.74 | 28364.0 | 1.19 | 28305.00 | 1.28 |
| CHI-135 | 69699.00 | 4.39 | 74978.00 | 2.49 | 28497.0 | 3.59 | 31568.00 | 1.63 |

Conclusion: The chimeric antibodies of the present disclosure were all able to bind to hNPR1 CHO-K1 and cNPR1 CHO-K1.

### Example 5: Humanization of Anti-NPR1 Murine Antibodies

By comparing the IMGT human antibody heavy and light chain variable region germline gene database and MOE software, heavy and light chain variable region germline genes highly homologous with 102, 127, 128, 135, and 140 were selected as templates. The CDRs of the murine antibodies were grafted into corresponding human templates to form variable region sequences with the following order: FR1-CDR1-FR2-CDR2-FR3-CDR3-FR4. Illustratively, the CDR amino acid residues in the specific examples below were determined and annotated using the Kabat numbering scheme.

### Humanization of murine antibody 102

IGHV3-21 *01 was used for the FR1, FR2, and FR3 of the humanized heavy chain variable region of murine antibody 102, and IGHJ6*01 was used as a template for the FR4. IGKV3-11*01 or IGKV1-39*01 was used for the FR1, FR2, and FR3 of the light chain variable region, and IGKJ4*01 was used as a template for the FR4. The CDRs of murine antibody 102 were grafted into its human templates. Optionally, some amino acids in the FRs of humanized antibodies were substituted: the amino acid residue(s) at position(s) 1, 3, 9, 44, 49, 87, and/or 93 (determined according to the Kabat numbering scheme) in the FRs of their heavy chain variable regions was/were substituted, and/or the amino acid residue(s) at position(s) 4, 43, 45, 46, 47, 58, and/or 71 (determined according to the Kabat numbering scheme) in the FRs of their light chain variable regions was/were substituted. The mutation design for humanized antibodies of antibody 102 is shown in Table 5 below:

**Table 5. Mutations for humanized antibodies of 102**

| **VL** | | **VH** | |
|---|---|---|---|
| hu102VL3 | Graft(IGKV3-11*01) + L46R, L47W, I58V, F71Y | hu102VH1 | Graft(IGHV3-21*01) + G44R, A93T |
| hu102VL4 | Graft(IGKV3-11*01) + A43S, R45K, L46R, L47W, I58V, F71Y | hu102VH2 | Graft(IGHV3-21*01) + G9E, G44R, S49A, A93T |
| hu102VL5 | Graft(IGKV1-39*01) + L46R, L47W | hu102VH3 | Graft(IGHV3-21*01) + E1D, Q3K, G9E, G44R, S49A, A93T |
| hu102VL6 | Graft(IGKV1-39*01) + M4L, A43S, L46R, L47W, F71Y | hu102VH4 | Graft(IGHV3-21*01) + E1D, Q3K, G9E, G44R, S49A, T87S, A93T+T31S, T62S |
| | | hu102VH5 | Graft(IGHV3-21*01) + E1D, Q3K, G9E, G44R, S49A, A93T + A33G |

| | | | |
|---|---|---|---|
| Note: Graft means that the CDRs of the murine antibody were grafted into the human germline FRs; the positions of the back mutation sites were determined according to the Kabat numbering scheme; for example, "T31S" means that the T at position 31 was mutated to S according to the Kabat numbering scheme. | | | |

**Table 6. The CDRs of humanized antibodies of 102**

| Variable region | CDR | Sequence | |
|---|---|---|---|
| hu102VH4 | HCDR1 | SYAMS | SEQ ID NO:45 |
| hu102VH4 | HCDR2 | YISRGGDYIYYADSVKG | SEQ ID NO:46 |
| hu102VH5 | HCDR1 | TYGMS | SEQ ID NO:35 |

The light/heavy chain variable region sequences of humanized antibodies of 102 are shown below:
> hu102VH1 
EVQLVESGGGLVKPGGSLRLSCAASGFTFSTYAMSWVRQAPGKRLEWVSYISRGGDYIY
YADTVKGRFTISRDNAKNSLYLQMNSLRAEDTAVYYCTRDQTTVVVHWYFDVWGQGT
TVTVSS
SEQ ID NO: 48
> hu102VH2 
EVQLVESGEGLVKPGGSLRLSCAASGFTFSTYAMSWVRQAPGKRLEWVAYISRGGDYIY
YADTVKGRFTISRDNAKNSLYLQMNSLRAEDTAVYYCTRDQTTVVVHWYFDVWGQGT
TVTVSS
SEQ ID NO: 49
> hu102VH3 
DVKLVESGEGLVKPGGSLRLSCAASGFTFSTYAMSWVRQAPGKRLEWVAYISRGGDYIY
YADTVKGRFTISRDNAKNSLYLQMNSLRAEDTAVYYCTRDQTTVVVHWYFDVWGQGT
TVTVSS
SEQ ID NO: 50
> hu102VH4 
DVKLVESGEGLVKPGGSLRLSCAASGFTFSSYAMSWVRQAPGKRLEWVAYISRGGDYIY
YADSVKGRFTISRDNAKNSLYLQMNSLRAEDSAVYYCTRDQTTVVVHWYFDVWGQGT
TVTVSS
SEQ ID NO: 51
> hu102VH5 DVKLVESGEGLVKPGGSLRLSCAASGFTFSTYGMSWVRQAPGKRLEWVAYISRGGDYIY 
YADTVKGRFTISRDNAKNSLYLQMNSLRAEDTAVYYCTRDQTTVVVHWYFDVWGQGT
TVTVSS
SEQ ID NO: 52
> hu102VL3 
EIVLTQSPATLSLSPGERATLSCSASSSINYMHWYQQKPGQAPRRWIYDTSKLASGVPARF
SGSGSGTDYTLTISSLEPEDFAVYYCHQRNSYTFGGGTKVEIK
SEQ ID NO: 53
> hu102VL4 
EIVLTQSPATLSLSPGERATLSCSASSSINYMHWYQQKPGQSPKRWIYDTSKLASGVPARF
SGSGSGTDYTLTISSLEPEDFAVYYCHQRNSYTFGGGTKVEIK
SEQ ID NO: 54
> hu102VL5 
DIQMTQSPSSLSASVGDRVTITCSASSSINYMHWYQQKPGKAPKRWIYDTSKLASGVPSR
FSGSGSGTDFTLTISSLQPEDFATYYCHQRNSYTFGGGTKVEIK
SEQ ID NO: 55
> hu102VL6 
DIQLTQSPSSLSASVGDRVTITCSASSSINYMHWYQQKPGKSPKRWIYDTSKLASGVPSRF
SGSGSGTDYTLTISSLQPEDFATYYCHQRNSYTFGGGTKVEIK
SEQ ID NO: 56
Note: The antibody CDR sequences are double-underlined; the CDRs are numbered according to Kabat.

### Humanization of murine antibody 127

IGHV1-46*01 was used for the FR1, FR2, and FR3 of the humanized heavy chain variable region of murine antibody 127, and IGHJ6*01 was used as a template for the FR4. IGKV4-1*01 or 1-39*01 was used for the FR1, FR2, and FR3 of the light chain variable region, and IGKJ2*01 was used as a template for the FR4. The CDRs of murine antibody 127 were grafted into its human templates. Optionally, some amino acids in the FRs of humanized antibodies were substituted: the amino acid residue(s) at position(s) 1, 2, 3, 28, 43, 69, 71, and/or 73 (determined according to the Kabat numbering scheme) in the FRs of their heavy chain variable regions was/were substituted, with a glutamic acid mutation designed for position 1 in some variable regions to avoid pyroglutamate formation, and/or the amino acid residue(s) at position(s) 1, 4, 17, 43, 46, 60, 68, and/or 79 (determined according to the Kabat numbering scheme) in the FRs of their light chain variable regions was/were substituted. The mutation design for the variable regions of humanized antibodies of antibody 127 is shown in Table 7 below:

**Table 7. Mutations for humanized antibodies of 127**

| **VL** | | **VH** | |
|---|---|---|---|
| hu127VL1 | Graft(IGKV4-1*01) + L46V, G68R | hu127VH4 | Graft(IGHV1-46*01) + V2A, Q3Y, T28S, Q43K, M69L, R71V, T73K + Q1E+ N54S |
| hu127VL3 | Graft(IGKV4-1*01) + D1N, M4L, L46V, G68R | hu127VH7 | Graft(IGHV1-46*01) + R71V, T73K + N54S |
| hu127VL4 | Graft(IGKV4-1*01) + D1N, M4L, L46V, D60A, G68R, Q79E | hu127VH8 | Graft(IGHV1-46*01) + R71V, T73K + Q1E+ N54S |
| hu127VL5 | Graft(IGKV1-39*01) + D1N, M4L, L46V, G68R | | |
| hu127VL6 | Graft(IGKV1-39*01) + D1N, M4L, D17Q, A43P, L46V, G68R | | |

| | | | |
|---|---|---|---|
| Note: Grafted means that the CDRs of the murine antibody were grafted into the human germline FRs; the positions of the mutation sites were determined according to the Kabat numbering scheme; for example, "V2A" means that the V at position 2 was mutated to A according to the Kabat numbering scheme. | | | |

**Table 8. The CDRs of humanized antibodies of 127**

| Variable region | CDR | Sequence | |
|---|---|---|---|
| hu127VH4/ VH7/ VH8 | HCDR2 | RINPYSGDTFYNQKFKG | SEQ ID NO:57 |

The light/heavy chain variable region sequences of humanized antibodies of 127 are shown below:
> hu127VH4 
EAYLVQSGAEVKKPGASVKVSCKASGYSFTGYFMNWVRQAPGKGLEWMGRINPYSGD
TFYNQKFKGRVTLTVDKSTSTVYMELSSLRSEDTAVYYCARRGYGNRGYWYFDVWGQ
GTTVTVSS
SEQ ID NO: 58
> hu127VH7 
QVQLVQSGAEVKKPGASVKVSCKASGYTFTGYFMNWVRQAPGQGLEWMGRINPYSGD
TFYNQKFKGRVTMTVDKSTSTVYMELSSLRSEDTAVYYCARRGYGNRGYWYFDVWGQ
GTTVTVSS
SEQ ID NO: 59
> hu127VH8 
EVQLVQSGAEVKKPGASVKVSCKASGYTFTGYFMNWVRQAPGQGLEWMGRINPYSGD
TFYNQKFKGRVTMTVDKSTSTVYMELSSLRSEDTAVYYCARRGYGNRGYWYFDVWGQ
GTTVTVSS
 SEQ ID NO: 60
> hu127VL1 
DIVMTQSPDSLAVSLGERATINCRASESVDSYGNSFMHWYQQKPGQPPKVLIYLASTLES
GVPDRFSGSGSRTDFTLTISSLQAEDVAVYYCQQNNEDPLTFGQGTKLEIK
SEQ ID NO: 61
> hul 27VL3 
NIVLTQSPDSLAVSLGERATINCRASESVDSYGNSFMHWYQQKPGQPPKVLIYLASTLES
GVPDRFSGSGSRTDFTLTISSLQAEDVAVYYCQQNNEDPLTFGQGTKLEIK
SEQ ID NO: 62
> hu127VL4 
NIVLTQSPDSLAVSLGERATINCRASESVDSYGNSFMHWYQQKPGQPPKVLIYLASTLES
GVPARFSGSGSRTDFTLTISSLEAEDVAVYYCQQNNEDPLTFGQGTKLEIK
SEQ ID NO: 63
> hu127VL5 
NIQLTQSPSSLSASVGDRVTITCRASESVDSYGNSFMHWYQQKPGKAPKVLIYLASTLES
GVPSRFSGSGSRTDFTLTISSLQPEDFATYYCQQNNEDPLTFGQGTKLEIK
SEQ ID NO: 64
> hu127VL6 
NIQLTQSPSSLSASVGQRVTITCRASESVDSYGNSFMHWYQQKPGKPPKVLIYLASTLES
GVPSRFSGSGSRTDFTLTISSLQPEDFATYYCQQNNEDPLTFGQGTKLEIK
SEQ ID NO: 65
Note: The antibody CDR sequences are double-underlined; the CDRs are numbered according to Kabat.

### Humanization of murine antibody 128

IGHV1-46*01 was used for the FR1, FR2, and FR3 of the humanized heavy chain variable region of murine antibody 128, and IGHJ6*01 was used as a template for the FR4. IGKV3-20*02 or 6-21*02 was used for the FR1, FR2, and FR3 of the light chain variable region, and IGKJ2*01 was used as a template for the FR4. The CDRs of murine antibody 128 were grafted into its human templates. Optionally, some amino acids in the FRs of humanized antibodies were substituted: the amino acid residue(s) at position(s) 1, 40, 43, 69, 71, 73, 74, 75, 77, and/or 78 (determined according to the Kabat numbering scheme) in the FRs of their heavy chain variable regions was/were substituted, with the amino acid residue at position 1 mutated to glutamic acid to avoid pyroglutamate formation, and/or the amino acid residue(s) at position(s) 22, 42, 43, 49, 57, and/or 71 (determined according to the Kabat numbering scheme) in the FRs of their light chain variable regions was/were substituted. The mutation design for the variable regions of humanized antibodies of antibody 128 is shown in Table 9 below:

**Table 9. Mutations for humanized antibodies of 128**

| **VL** | | **VH** | |
|---|---|---|---|
| hu128VL1 | Graft(IGKV3-20*02) + G57R, F71Y | hu128VH1 | Graft(IGHV1-46*01) + Q1E, R71V, T73K |
| hu128VL2 | Graft(IGKV3-20*02) + Q42S, A43S, G57R, F71Y | hu128VH2 | Graft(IGHV1-46*01) + Q1E, R71V, T73K, S74P, T75S |
| hu128VL3 | Graft(IGKV6-21*02) + K49Y, G57R, F71Y | hu128VH3 | Graft(IGHV1-46*01) + Q1E, M69L, R71V, T73K, S74P, T75S, T77P, V78A |
| hu128VL1-6 | Graft(IGKV3-20*02) + S22K, G57R, F71Y+ A55G | hu128VH4 | Graft(IGHV1-46*01) + Q1E, A40R, Q43R, M69L, R71V, T73K, S74P, T75S, T77P, V78A |

| | | | |
|---|---|---|---|
| Note: Grafted means that the CDRs of the murine antibody were grafted into the human germline FRs; the positions of the back mutation sites were determined according to the Kabat numbering scheme. For example, "G57R" means that the G at position 57 was mutated to R according to the Kabat numbering scheme. | | | |

> hu128VH1 
EVQLVQSGAEVKKPGASVKVSCKASGYTFTSYWMHWVRQAPGQGLEWMGRIDPNSG
VTKYNEKFKSRVTMTVDKSTSTVYMELSSLRSEDTAVYYCARSSYLGRYFDVWGQGTT
VTVSS
SEQ ID NO: 66
> hu128VH2 
EVQLVQSGAEVKKPGASVKVSCKASGYTFTSYWMHWVRQAPGQGLEWMGRIDPNSG
VTKYNEKFKSRVTMTVDKPSSTVYMELSSLRSEDTAVYYCARSSYLGRYFDVWGQGTT
VTVSS
SEQ ID NO: 67
> hu128VH3 
EVQLVQSGAEVKKPGASVKVSCKASGYTFTSYWMHWVRQAPGQGLEWMGRIDPNSG
VTKYNEKFKSRVTLTVDKPSSPAYMELSSLRSEDTAVYYCARSSYLGRYFDVWGQGTTV
TVSS
SEQ ID NO: 68
> hu128VH4 
EVQLVQSGAEVKKPGASVKVSCKASGYTFTSYWMHWVRQRPGRGLEWMGRIDPNSGV
TKYNEKFKSRVTLTVDKPSSPAYMELSSLRSEDTAVYYCARSSYLGRYFDVWGQGTTVT
VSS
SEQ ID NO: 69
> hu128VL1 
EIVLTQSPATLSLSPGERATLSCSATSSVSYMYWYQQKPGQAPRLLIYDTYNLASRIPARFS
GSGSGTDYTLTISRLEPEDFAVYYCQQWSSYPPTFGQGTKLEIK
SEQ ID NO: 70
> hu128VL2 
EIVLTQSPATLSLSPGERATLSCSATSSVSYMYWYQQKPGSSPRLLIYDTYNLASRIPARFS
GSGSGTDYTLTISRLEPEDFAVYYCQQWSSYPPTFGQGTKLEIK
SEQ ID NO: 71
> hu128VL3

EIVLTQSPDFQSVTPKEKVTITCSATSSVSYMYWYQQKPDQSPKLLIYDTYNLASRVPSRF
SGSGSGTDYTLTINSLEAEDAATYYCQQWSSYPPTFGQGTKLEIK
SEQ ID NO: 72

In addition, a new humanized antibody light chain variable region sequence was obtained by engineering individual amino acids in the light chain FR1 (S22) and LCDR2 (A55) of hu128VL1; the amino acid sequence of LCDR2 was engineered, changing from DTYNLAS (SEQ ID NO: 33) to DTYNLGS (SEQ ID NO: 73). The specific sequence is shown below:
> hu128VL1-6 
EIVLTQSPATLSLSPGERATLKCSATSSVSYMYWYQQKPGQAPRLLIYDTYNLGSRIPARF
SGSGSGTDYTLTISRLEPEDFAVYYCQQWSSYPPTFGQGTKLEIK
SEQ ID NO: 74
Note: The antibody CDR sequences are double-underlined; the CDRs are numbered according to Kabat.

### Humanization of murine antibody 135

IGHV3-21*01 was used for the FR1, FR2, and FR3 of the humanized heavy chain variable region of murine antibody 135, and IGHJ6*01 was used as a template for the FR4. IGKV1-39*01 was used for the FR1, FR2, and FR3 of the light chain variable region, and IGKJ4*01 was used as a template for the FR4. The CDRs of murine antibody 135 were grafted into its human templates. Optionally, some amino acids in the FRs of humanized antibodies were substituted: the amino acid residue(s) at position(s) 1, 3, 9, 28, 29, 44, 49, and/or 93 (determined according to the Kabat numbering scheme) in the FRs of their heavy chain variable regions was/were substituted, and/or the amino acid residue(s) at position(s) 4, 43, 46, 47, and/or 71 (determined according to the Kabat numbering scheme) in the FRs of their light chain variable regions was/were substituted. The mutation design for the variable regions of humanized antibodies of antibody 135 is shown in Table 10 below:

**Table 10. Mutations for humanized antibodies of 135**

| **VL** | | **VH** | |
|---|---|---|---|
| hu135VL1 | Graft(IGKV1-39*01) + M4L, | hu135VH1 | Graft(IGHV3-21 *01) + E1D, |
| | A43S, L46R, L47W, F71Y | | Q3K, G9E, G44R, S49A, A93T |
| | | hu135VH2 | Graft(IGHV3-21*01) + E1D, Q3K, G9E, T28S, F29I, G44R, S49A, A93T |
| | | hul35VH3 | Graft(IGHV3-21*01) + E1D, Q3K, G9E, T28S, F29I, G44R, S49A, A93T + T62S |

| | | | |
|---|---|---|---|
| Note: Grafted means that the CDRs of the murine antibody were grafted into the human germline FRs; the positions of the back mutation sites were determined according to the Kabat numbering scheme. For example, "M4L" means that the M at position 4 was mutated to L according to the Kabat numbering scheme. | | | |

**Table 11. The CDRs of humanized antibodies of 135**

| Variable region | CDR | Sequence | |
|---|---|---|---|
| hu135VH3 | HCDR2 | YINSGGNYIYYADSVKG | SEQ ID NO:75 |

> hu135VH1 
DVKLVESGEGLVKPGGSLRLSCAASGFTFSTYGMSWVRQAPGKRLEWVAYINSGGNYIY
YADTVKGRFTISRDNAKNSLYLQMNSLRAEDTAVYYCTRDRTTIVVHWYFDVWGQGTT
VTVSS
SEQ ID NO: 76
> hu135VH2 
DVKLVESGEGLVKPGGSLRLSCAASGFSISTYGMSWVRQAPGKRLEWVAYINSGGNYIY
YADTVKGRFTISRDNAKNSLYLQMNSLRAEDTAVYYCTRDRTTIVVHWYFDVWGQGTT
VTVSS
SEQ ID NO: 77
> hu135VH3 
DVKLVESGEGLVKPGGSLRLSCAASGFSISTYGMSWVRQAPGKRLEWVAYINSGGNYIY
YADSVKGRFTISRDNAKNSLYLQMNSLRAEDTAVYYCTRDRTTIVVHWYFDVWGQGTT
VTVSS
SEQ ID NO: 78
> hu135VL1 
DIQLTQSPSSLSASVGDRVTITCSASSSLNFMHWYQQKPGKSPKRWIYDTSKLASGVPSR
FSGSGSGTDYTLTISSLQPEDFATYYCHQRSSTFGGGTKVEIK
SEQ ID NO: 79
Note: The antibody CDR sequences are double-underlined; the CDRs are numbered according to Kabat.

### Humanization of murine antibody 140

IGHV1-46*01 was used for the FR1, FR2, and FR3 of the humanized heavy chain variable region of murine antibody 140, and IGHJ6*01 was used as a template for the FR4. IGKV3-11*01 was used for the FR1, FR2, and FR3 of the light chain variable region, and IGKJ2*01 was used as a template for the FR4. The CDRs of murine antibody 140 were grafted into its human templates. Optionally, some amino acids in the FRs of humanized antibodies were substituted: the amino acid residue(s) at position(s) 1, 40, 43, 48, 69, 71, 73, 74, 75, 77, 78, 80, and/or 81 (determined according to the Kabat numbering scheme) in the FRs of their heavy chain variable regions was/were substituted, with a glutamic acid mutation designed for position 1 in some variable regions to avoid pyroglutamate formation, and/or the amino acid residue(s) at position(s) 20, 60, 58, 70, 71, 77, and/or 100 (determined according to the Kabat numbering scheme) in the FRs of their light chain variable regions was/were substituted. The mutation design for the variable regions of humanized antibodies of antibody 140 is shown in Table 12 below:

**Table 12. Mutations for humanized antibodies of 140**

| **VL** | | **VH** | |
|---|---|---|---|
| hu140VL3 | Graft(IGKV3-11*01) + I58V, F71Y | hu140VH3 | Graft(IGHV1-46*01) + A40R, Q43R, M69L, R71V, T73K, S74P, T75S, V78A+ Q1E |
| hu140VL5 | Graft(IGKV3-11*01) + I58V, D70S, F71Y, S77R, Q100A | hu140VH4 | Graft(IGHV1-46*01) + M48I, M69L, R71V, T73K, S74P, V78A, M80I, E81Q |
| hu140VL5-11 | Graft(IGKV3-11*01) + T20Q, I58V, A60D, D70S, F71Y, S77R, Q100A | hu140VH5 | Graft(IGHV1-46*01) + M69L, R71V, T73K, S74P, T75S, T77P, V78A |

| | | | |
|---|---|---|---|
| Note: Grafted means that the CDRs of the murine antibody were grafted into the human germline FRs; the positions of the back mutation sites were determined according to the Kabat numbering scheme. For example, "I58V" means that the I at position 58 was mutated to V according to the Kabat numbering scheme. | | | |

> hu140VH3 
EVQLVQSGAEVKKPGASVKVSCKASGYTFTSYWMHWVRQRPGRGLEWMGRIDPNSGV
TKYNEKFKSRVTLTVDKPSSTAYMELSSLRSEDTAVYYCARSGGLRRYFDVWGQGTTVT
VSS
SEQ ID NO: 80
> hu140VH4 
QVQLVQSGAEVKKPGASVKVSCKASGYTFTSYWMHWVRQAPGQGLEWIGRIDPNSGV
TKYNEKFKSRVTLTVDKPTSTAYIQLSSLRSEDTAVYYCARSGGLRRYFDVWGQGTTVT
 
VSS
SEQ ID NO: 81
> hu140VH5 
QVQLVQSGAEVKKPGASVKVSCKASGYTFTSYWMHWVRQAPGQGLEWMGRIDPNSG
VTKYNEKFKSRVTLTVDKPSSPAYMELSSLRSEDTAVYYCARSGGLRRYFDVWGQGTTV
TVSS
SEQ ID NO: 82
> hu140VL3 
EIVLTQSPATLSLSPGERATLSCSASSSVSYMYWYQQKPGQAPRLLIYDTYNLASGVPARF
SGSGSGTDYTLTISSLEPEDFAVYYCQQWSSYPPTFGQGTKLEIK
SEQ ID NO: 83
> hu140VL5

EIVLTQSPATLSLSPGERATLSCSASSSVSYMYWYQQKPGQAPRLLIYDTYNLASGVPARF
SGSGSGTSYTLTISRLEPEDFAVYYCQQWSSYPPTFGAGTKLEIK
SEQ ID NO: 84

In addition, a new sequence was obtained by engineering individual amino acids in the light chain FR1 (T20) and FR3 (A60) of hu140VL5.
> hu140VL5-11 
EIVLTQSPATLSLSPGERAQLSCSASSSVSYMYWYQQKPGQAPRLLIYDTYNLASGVPDR
FSGSGSGTSYTLTISRLEPEDFAVYYCQQWSSYPPTFGAGTKLEIK
SEQ ID NO: 85
Note: The antibody CDR sequences are double-underlined; the CDRs are numbered according to Kabat.

### Construction and expression of IgG1 LALA formats of anti-NPR1 humanized antibodies

Primers were designed, and VH/VK gene fragments of humanized antibodies were constructed by PCR and then homologously recombined with the expression vector pTT5 (with a signal peptide and a constant region gene (CH1-FC/CL) fragment, constructed in the laboratory) to construct antibody full-length expression vector VH-CH1-FC-pTT5/VK-CL-pTT5. The heavy chain constant regions of the antibodies may be selected from the group consisting of the heavy chain constant regions of human IgG1, IgG2, IgG3, and IgG4, and variants thereof, and the light chain constant regions may be selected from the group consisting of the light chain constant regions of human κ and λ chains and variants thereof. Illustratively, in the following examples, the heavy chain constant regions of the antibodies were selected from the group consisting of the human IgG1 heavy chain constant region, which is set forth in SEQ ID NO: 86, the light chain constant regions were selected from the group consisting of the human light chain constant region set forth in SEQ ID NO: 44, and LALA mutations (double-underlined) were introduced.
Human IgG1 LALA heavy chain constant region sequence: 
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPEAAGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKS
RWQQGNVFSCSVMHEALHNHYTQKSLSLSPGK
SEQ ID NO: 86
Human light chain constant region sequence:



SEQ ID NO: 44

By linking the carboxy-termini of the heavy chain variable regions of humanized antibodies of 102, 127, 128, 135, and 140 constructed above to the amino-terminus of the human heavy chain constant region set forth in SEQ ID NO: 86 to form antibody full-length heavy chains, and linking the carboxy-termini of the light chain variable regions of humanized antibodies to the amino-terminus of the human light chain constant region set forth in SEQ ID NO: 44 to form antibody full-length light chains, the humanized antibodies shown in Table 13 to Table 17 below can be obtained:

**Table 13. Humanized antibodies of 102**

| | | | |
|---|---|---|---|
| Hu102H1L3 | Hu102H1L4 | Hu102H1L5 | Hu102H1L6 |
| Hu102H2L3 | Hu102H2L4 | Hu102H2L5 | Hu102H2L6 |
| Hu102H3L3 | Hul02H3L4 | Hu102H3L5 | Hu102H3L6 |
| Hu102H4L3 | Hul02H4L4 | Hu102H4L5 | Hu102H4L6 |
| Hu102H5L3 | Hu102H5L4 | Hu102H5L5 | Hu102H5L6 |

| | | | |
|---|---|---|---|
| Note: In the table, "Hu102H1L3" represents a humanized antibody whose heavy chain variable region is hu102VH1 (SEQ ID NO: 48), whose light chain variable region is hu102VL3 (SEQ ID NO: 53), whose heavy chain constant region is set forth in SEQ ID NO: 86, and whose light chain constant region is set forth in SEQ ID NO: 44; this similarly applies to the other antibodies. | | | |

**Table 14. Humanized antibodies of 127**

| | | | | |
|---|---|---|---|---|
| Hu127H4L1 | Hu127H4L3 | Hu127H4L4 | Hu127H4L5 | Hu127H4L6 |
| Hu127H7L1 | Hu127H7L3 | Hu127H7L4 | Hu127H7L5 | Hu127H7L6 |
| Hu127H8L1 | Hu127H8L3 | Hu127H8L4 | Hu127H8L5 | Hu127H8L6 |

| | | | | |
|---|---|---|---|---|
| Note: In the table, "Hu127H4L1" represents a humanized antibody whose heavy chain variable region is hu127VH4 (SEQ ID NO: 58), whose light chain variable region is hu127VL1 (SEQ ID NO: 61), whose heavy chain constant region is set forth in SEQ ID NO: 86, and whose light chain constant region is set forth in SEQ ID NO: 44; this similarly applies to the other antibodies. | | | | |

**Table 15. Humanized antibodies of 128**

| | | | |
|---|---|---|---|
| Hu128H1L1 | Hu128H1L2 | Hu128H1L3 | |
| Hu128H2L1 | Hu128H2L2 | Hu128H2L3 | |
| Hu128H3L1 | Hu128H3L2 | Hu128H3L3 | Hu128H3L1-6 |
| Hu128H4L1 | Hu128H4L2 | Hu128H4L3 | |

| | | | |
|---|---|---|---|
| Note: In the table, "Hu128H1L3" represents a humanized antibody whose heavy chain variable region is hu128VH1 (SEQ ID NO: 66), whose light chain variable region is hu128VL3 (SEQ ID NO: 72), whose heavy chain constant region is set forth in SEQ ID NO: 86, and whose light chain constant region is set forth in SEQ ID NO: 44; this similarly applies to the other antibodies. | | | |

**Table 16. Humanized antibodies of 135**

| |
|---|
| Hul35H1L1 |
| Hul35H2L1 |
| Hul35H3L1 |

| |
|---|
| Note: In the table, "Hu135H1L1" represents a humanized antibody whose heavy chain variable region is hu135VH1 (SEQ ID NO: 76), whose light chain variable region is hu135VL1 (SEQ ID NO: 79), whose heavy chain constant region is set forth in SEQ ID NO: 86, and whose light chain constant region is set forth in SEQ ID NO: 44; this similarly applies to the other antibodies. |

**Table 17. Humanized antibodies of 140**

| | | |
|---|---|---|
| Hu140H3L3 | Hu140H3L5 | Hu140H5L5-11 |
| Hu140H4L3 | Hu140H4L5 | |
| Hu140H5L3 | Hu140H5L5 | |

| | | |
|---|---|---|
| Note: In the table, "Hu140H5L5" represents a humanized antibody whose heavy chain variable region is hu140VH5 (SEQ ID NO: 82) and whose light chain variable region is hu140VL5 (SEQ ID NO: 84), and Hu140H5L5-11 represents a humanized antibody whose heavy chain variable region is Hu140VH5 (SEQ ID NO: 82) and whose light chain variable region is hu140VL5-11 (SEQ ID NO: 85); their heavy chain constant region is set forth in SEQ ID NO: 86, and their light chain constant region is set forth in SEQ ID NO: 44; this similarly applies to the other antibodies. | | |

Exemplary humanized antibody light/heavy chain full-length sequences are shown below:
Heavy chain sequence of Hu102H2L6: 
EVQLVESGEGLVKPGGSLRLSCAASGFTFSTYAMSWVRQAPGKRLEWVAYISRGGDYIY
YADTVKGRFTISRDNAKNSLYLQMNSLRAEDTAVYYCTRDQTTVVVHWYFDVWGQGT
TVTVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPEAAGGPS
 
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSV
MHEALHNHYTQKSLSLSPGK
SEQ ID NO: 112
Heavy chain sequence of Hu102H3L6: 
DVKLVESGEGLVKPGGSLRLSCAASGFTFSTYAMSWVRQAPGKRLEWVAYISRGGDYIY
YADTVKGRFTISRDNAKNSLYLQMNSLRAEDTAVYYCTRDQTTVVVHWYFDVWGQGT
TVTVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSV
MHEALHNHYTQKSLSLSPGK
SEQ ID NO: 113
Heavy chain sequence of Hu102H4L6: 
DVKLVESGEGLVKPGGSLRLSCAASGFTFSSYAMSWVRQAPGKRLEWVAYISRGGDYIY
YADSVKGRFTISRDNAKNSLYLQMNSLRAEDSAVYYCTRDQTTVVVHWYFDVWGQGT
TVTVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSV
MHEALHNHYTQKSLSLSPGK
SEQ ID NO: 87
Heavy chain sequence of Hu102H5L6: 
DVKLVESGEGLVKPGGSLRLSCAASGFTFSTYGMSWVRQAPGKRLEWVAYISRGGDYIY
YADTVKGRFTISRDNAKNSLYLQMNSLRAEDTAVYYCTRDQTTVVVHWYFDVWGQGT
TVTVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPEAAGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSV
MHEALHNHYTQKSLSLSPGK
SEQ ID NO: 114
Light chain sequences of Hu102H2L6, Hu102H3L6, Hu102H4L6, and Hu102H5L6: 
DIQLTQSPSSLSASVGDRVTITCSASSSINYMHWYQQKPGKSPKRWIYDTSKLASGVPSRF
SGSGSGTDYTLTISSLQPEDFATYYCHQRNSYTFGGGTKVEIKRTVAAPSVFIFPPSDEQLK
SGTASVVCLLNNFYPREAKVQWKVDNALQSGNSQESVTEQDSKDSTYSLSSTLTLSKADYEKH
 
KVYACEVTHQGLSSPVTKSFNRGEC
SEQ ID NO: 88
Heavy chain sequences of Hu127H4L1, Hu127H4L3, Hu127H4L5, and Hu127H4L6: 
EAYLVQSGAEVKKPGASVKVSCKASGYSFTGYFMNWVRQAPGKGLEWMGRINPYSGD
TFYNQKFKGRVTLTVDKSTSTVYMELSSLRSEDTAVYYCARRGYGNRGYWYFDVWGQ
GTTVTVSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPEAAGG
PSVFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFSC
SVMIIEALIINIIYTQKSLSLSPGK
SEQ ID NO: 115
Heavy chain sequences of Hu127H7L1, Hu127H7L3, Hu127H7L5, and Hu127H7L6: 
QVQLVQSGAEVKKPGASVKVSCKASGYTFTGYFMNWVRQAPGQGLEWMGRINPY
SGDTFYNQKFKGRVTMTVDKSTSTVYMELSSLRSEDTAVYYCARRGYGNRGYWY
FDVWGQGTTVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCDKT
HTCPPCPAPEAAGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAKGQ
PREPQVYTLPPSRDELTKNQVSLTCLVKGFYPSDIAVEWESNGQPENNYKTTPPVLDSDG
SFFLYSKLTVDKSRWQQGNVFSCSVMHEALHNHYTQKSLSLSPGK
SEQ ID NO: 116
Heavy chain sequences of Hu127H8L1, Hu127H8L3, Hu127H8L5, and Hu127H8L6: 
EVQLVQSGAEVKKPGASVKVSCKASGYTFTGYFMNWVRQAPGQGLEWMGRINPY
SGDTFYNQKFKGRVTMTVDKSTSTVYMELSSLRSEDTAVYYCARRGYGNRGYWY
FDVWGQGTTVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCDKT
HTCPPCPAPEAAGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAKGQ
PREPQVYTLPPSRDELTKNQVSLTCLVKGFYPSDIAVEWESNGQPENNYKTTPPVLDSDG
SFFLYSKLTVDKSRWQQGNVFSCSVMHEALHNHYTQKSLSLSPGK
SEQ ID NO: 117
Light chain sequences of Hu127H4L1, Hu127H7L1, and Hu127H8L1: 
DIVMTQSPDSLAVSLGERATINCRASESVDSYGNSFMHWYQQKPGQPPKVLIYLAS
TLESGVPDRFSGSGSRTDFTLTISSLQAEDVAVYYCQQNNEDPLTFGQGTKLEIKRTV
AAPSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC
SEQ ID NO: 118
Light chain sequences of Hu127H4L3, Hu127H7L3, and Hu127H8L3: 
NIVLTQSPDSLAVSLGERATINCRASESVDSYGNSFMHWYQQKPGQPPKVLIYLAST
LESGVPDRFSGSGSRTDFTLTISSLQAEDVAVYYCQQNNEDPLTFGQGTKLEIKRTVA
APSVFIFPPSDEQLKSGTASVVCLLNNFYPREAKVQWKVDNALQSGNSQESVTEQDSKDS
 
TYSLSSTLTLSKADYEKHKVYACEVTHQGLSSPVTKSFNRGEC
SEQ ID NO: 119
Light chain sequences of Hu127H4L5, Hu127H7L5, and Hu127H8L5: 
NIQLTQSPSSLSASVGDRVTITCRASESVDSYGNSFMHWYQQKPGKAPKVLIYLAST
LESGVPSRFSGSGSRTDFTLTISSLQPEDFATYYCQQNNEDPLTFGQGTKLEIKRTVAA
PSVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC
SEQ ID NO: 120
Light chain sequences of Hu127H4L6, Hu127H7L6, and Hu127H8L6: 
NIQLTQSPSSLSASVGQRVTITCRASESVDSYGNSFMHWYQQKPGKPPKVLIYLAST
LESGVPSRFSGSGSRTDFTLTISSLQPEDFATYYCQQNNEDPLTFGQGTKLEIKRTVAA
PSVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC
SEQ ID NO: 121
Heavy chain sequences of Hu128H2L1 and Hu128H2L2: 
EVQLVQSGAEVKKPGASVKVSCKASGYTFTSYWMHWVRQAPGQGLEWMGRIDP
NSGVTKYNEKFKSRVTMTVDKPSSTVYMELSSLRSEDTAVYYCARSSYLGRYFDV
WGQGTTVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSFF
LYSKLTVDKSRWQQGNVFSCSVMHEALHNHYTQKSLSLSPGK
SEQ ID NO: 122
Heavy chain sequences of Hu128H3L1-6, Hu128H3L1, and Hu128H3L2: 
EVQLVQSGAEVKKPGASVKVSCKASGYTFTSYWMHWVRQAPGQGLEWMGRIDPNSG
VTKYNEKFKSRVTLTVDKPSSPAYMELSSLRSEDTAVYYCARSSYLGRYFDVWGQGTTV
TVSSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPEAAGGPSVF
LFPPKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGK
SEQ ID NO: 99
Light chain sequences of Hu128H2L1 and Hu128H3L1: 
EIVLTQSPATLSLSPGERATLSCSATSSVSYMYWYQQKPGQAPRLLIYDTYNLASRIPARFS
GSGSGTDYTLTISRLEPEDFAVYYCQQWSSYPPTFGQGTKLEIKRTVAAPSVFIFPPSDEQL
KSGTASVVCLLNNFYPREAKVQWKVDNALQSGNSQESVTEQDSKDSTYSLSSTLTLSKADYEK
HKVYACEVTHQGLSSPVTKSFNRGEC
SEQ ID NO: 123
Light chain sequences of Hu128H2L2 and Hu128H3L2: EIVLTQSPATLSLSPGERATLSCSATSSVSYMYWYQQKPGSSPRLLIYDTYNLASRIPARFS 
GSGSGTDYTLTISRLEPEDFAVYYCQQWSSYPPTFGQGTKLEIKRTVAAPSVFIFPPSDEQL
KSGTASVVCLLNNFYPREAKVQWKVDNALQSGNSQESVTEQDSKDSTYSLSSTLTLSKADYEK
HKVYACEVTHQGLSSPVTKSFNRGEC
SEQ ID NO: 124
Light chain sequence of Hu128H3L1-6: 
EIVLTQSPATLSLSPGERATLKCSATSSVSYMYWYQQKPGQAPRLLIYDTYNLGSRIPARF
SGSGSGTDYTLTISRLEPEDFAVYYCQQWSSYPPTFGQGTKLEIKRTVAAPSVFIFPPSDEQ
LKSGTASVVCLLNNFYPREAKVQWKVDNALQSGNSQESVTEQDSKDSTYSLSSTLTLSKADYEK
HKVYACEVTHQGLSSPVTKSFNRGEC
SEQ ID NO: 100
Heavy chain sequence of Hu135H1L1: 
DVKLVESGEGLVKPGGSLRLSCAASGFTFSTYGMSWVRQAPGKRLEWVAYINSGGNYIY
YADTVKGRFTISRDNAKNSLYLQMNSLRAEDTAVYYCTRDRTTIVVHWYFDVWGQGTT
VTVSSASTKGPSVFPLAPSSKSTSGGTAALGCLVKDYFPEPVTVSWNSGALTSGVIITFPAVLQS
SGLYSLSSVVTVPSSSLGTQTYICNVNHKPSNTKVDKKVEPKSCDKTHTCPPCPAPEAAGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGK
SEQ ID NO: 125
Heavy chain sequence of Hu135H2L1: 
DVKLVESGEGLVKPGGSLRLSCAASGFSISTYGMSWVRQAPGKRLEWVAYINSGGNYIY
YADTVKGRFTISRDNAKNSLYLQMNSLRAEDTAVYYCTRDRTTIVVHWYFDVWGQGTT
VTVSSASTKGPSVFPLAPSSKSTSGGTAALGCLVKDYFPEPVTVSWNSGALTSGVIITFPAVLQS
SGLYSLSSVVTVPSSSLGTQTYICNVNHKPSNTKVDKKVEPKSCDKTHTCPPCPAPEAAGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGK
SEQ ID NO: 126
Heavy chain sequence of Hu135H3L1: 
DVKLVESGEGLVKPGGSLRLSCAASGFSISTYGMSWVRQAPGKRLEWVAYINSGGNYIY
YADSVKGRFTISRDNAKNSLYLQMNSLRAEDTAVYYCTRDRTTIVVHWYFDVWGQGTT
VTVSSASTKGPSVFPLAPSSKSTSGGTAALGCLVKDYFPEPVTVSWNSGALTSGVIITFPAVLQS
SGLYSLSSVVTVPSSSLGTQTYICNVNHKPSNTKVDKKVEPKSCDKTHTCPPCPAPEAAGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGK
SEQ ID NO: 101
Light chain sequences of Hu135H1L1, Hu135H2L1, and Hu135H3L1: 
DIQLTQSPSSLSASVGDRVTITCSASSSLNFMHWYQQKPGKSPKRWIYDTSKLASGVPSR
FSGSGSGTDYTLTISSLQPEDFATYYCHQRSSTFGGGTKVEIKRTVAAPSVFIFPPSDEQLKS
GTASVVCLLNNFYPREAKVQWKVDNALQSGNSQESVTEQDSKDSTYSLSSTLTLSKADYEKHK
 
VYACEVTHQGLSSPVTKSFNRGEC
SEQ ID NO: 47
Heavy chain sequence of Hu140H4L5: 
QVQLVQSGAEVKKPGASVKVSCKASGYTFTSYWMHWVRQAPGQGLEWIGRIDPNSGV
TKYNEKFKSRVTLTVDKPTSTAYIQLSSLRSEDTAVYYCARSGGLRRYFDVWGQGTTVT
VSSASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPEAAGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMH
EALHNHYTQKSLSLSPGK
SEQ ID NO: 127
Heavy chain sequences of Hu140H5L5 and Hu140H5L5-11: 
QVQLVQSGAEVKKPGASVKVSCKASGYTFTSYWMHWVRQAPGQGLEWMGRIDPNSG
VTKYNEKFKSRVTLTVDKPSSPAYMELSSLRSEDTAVYYCARSGGLRRYFDVWGQGTTV
TVSSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPEAAGGPSVF
LFPPKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGK
SEQ ID NO: 89
Light chain sequences of Hu140H4L5 and Hu140H5L5: 
EIVLTQSPATLSLSPGERATLSCSASSSVSYMYWYQQKPGQAPRLLIYDTYNLASGVPARF
SGSGSGTSYTLTISRLEPEDFAVYYCQQWSSYPPTFGAGTKLEIKRTVAAPSVFIFPPSDEQL
KSGTASVVCLLNNFYPREAKVQWKVDNALQSGNSQESVTEQDSKDSTYSLSSTLTLSKADYEK
HKVYACEVTHQGLSSPVTKSFNRGEC
SEQ ID NO: 128
Light chain sequence of Hu140H5L5-11: 
EIVLTQSPATLSLSPGERAQLSCSASSSVSYMYWYQQKPGQAPRLLIYDTYNLASGVPDR
FSGSGSGTSYTLTISRLEPEDFAVYYCQQWSSYPPTFGAGTKLEIKRTVAAPSVFIFPPSDEQ
LKSGTASVVCLLNNFYPREAKVQWKVDNALQSGNSQESVTEQDSKDSTYSLSSTLTLSKADYEK
HKVYACEVTHQGLSSPVTKSFNRGEC
SEQ ID NO: 90
Note: In the above antibody full-length sequences, the antibody CDR sequences are single-underlined, and the antibody constant region sequences are italicized; the CDRs are numbered according to Kabat.

The light and heavy chain sequences of the positive control antibodies REGN-5381 (see WO2020086406A2 for its variable region sequences) and XX16 V (see WO2020250159A1 for its variable region sequences) in the examples of the present disclosure are shown below:
Heavy chain sequence of REGN-5381: 
QVQLVQSGAEVKKPGASVTVSCKASGYTFTDYYMHWVRQAPGQGLEWMGWIKPNSG
GTNSAQRFQGRITMTWDTSISTAYMELSRLRSDDTAVYYCSRGGPVMNYYYYYGMDV
WGQGTTVTVSSASTKGPSVFPLAPCSRSTSESTAALGCLVKDYFPEPVTVSWNSGALTSG
VHTFPAVLQSSGLYSLSSVVTVPSSSLGTKTYTCNVDHKPSNTKVDKRVESKYGPPCPPC
PAPEFLGGPSVFLFPPKPKDTLMISRTPEVTCVVVDVSQEDPEVQFNWYVDGVEVHNAK
TKPREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSFFLY
SRLTVDKSRWQEGNVFSCSVMHEALHNHYTQKSLSLSLGK
SEQ ID NO: 91
Light chain sequence of REGN-5381: 
NIQMTQSPSSLSASVGDRVTITCRASQSIDSYLNWYQQKPGKAPKLLIYVASSLQSGVPSR
FSGSGSGKDFTLTISSLQPEDFATYYCQQSYSIPTFGQGTRLEIKRTVAAPSVFIFPPSDEQL
KSGTASVVCLLNNFYPREAKVQWKVDNALQSGNSQESVTEQDSKDSTYSLSSTLTLSKA
DYEKHKVYACEVTHQGLSSPVTKSFNRGEC
SEQ ID NO: 92
Heavy chain sequence of REGN-5381-m (variable regions of REGN-5381 + mouse IgG2a heavy chain, IMGT accession No. V0085): 
QVQLVQSGAEVKKPGASVTVSCKASGYTFTDYYMHWVRQAPGQGLEWMGWIKPNSG
GTNSAQRFQGRITMTWDTSISTAYMELSRLRSDDTAVYYCSRGGPVMNYYYYYGMDV
WGQGTTVTVSSAKTTAPSVYPLAPVCGDTTGSSVTLGCLVKGYFPEPVTLTWNSGSLSS
GVHTFPAVLQSDLYTLSSSVTVTSSTWPSQSITCNVAHPASSTKVDKKIEPRGPTIKPCPPC
KCPAPNLLGGPSVFIFPPKIKDVLMISLSPIVTCVVVDVSEDDPDVQISWFVNNVEVHTAQ
TQTHREDYNSTLRVVSALPIQHQDWMSGKEFKCKVNNKDLPAPIERTISKPKGSVRAPQ
VYVLPPPEEEMTKKQVTLTCMVTDFMPEDIYVEWTNNGKTELNYKNTEPVLDSDGSYF
MYSKLRVEKKNWVERNSYSCSVVHEGLHNHHTTKSFSRTPGK
SEQ ID NO: 93
Light chain sequence of REGN-5381-m (variable regions of REGN-5381 + mouse kappa light chain, Uniprot accession No. P01837): 
NIQMTQSPSSLSASVGDRVTITCRASQSIDSYLNWYQQKPGKAPKLLIYVASSLQSGVPSR
FSGSGSGKDFTLTISSLQPEDFATYYCQQSYSIPTFGQGTRLEIKRADAAPTVSIFPPSSEQL
TSGGASVVCFLNNFYPKDINVKWKIDGSERQNGVLNSWTDQDSKDSTYSMSSTLTLTKD
EYERHNSYTCEATHKTSTSPIVKSFNRNEC
SEQ ID NO: 94
Heavy chain sequence of XX16 V: 
QVQLLESGGGLVQPGGSLRLSCAASGFTFSSYWMNWVRQAPGKGLEWVSVIESKGNYI
FYADSVKGRFTISRDNSKNTLYLQMNSLRAEDTAVYYCARDRYSMIYSYGAGAFDYWG
 
QGTLVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTCPPC
PAPEAAGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFFLYS
KLTVDKSRWQQGNVFSCSVMHEALHNHYTQKSLSLSPGK
SEQ ID NO: 95
Light chain sequence of XX16 V:

DIQMTQSPSSLSASVGDRVTITCRASQGISSYLAWYQQKPGKAPKLLIYTASTLQSGVPSR
FSGSGSGTDFTLTISSLQPEDFATYYCQQTWRKPRTFGQGTKVEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVTKSFNRGEC
SEQ ID NO: 96

The light and heavy chains of the negative control antibody RC25 V (see RC25 in US6114143A for its variable regions) disclosed in the present disclosure are shown below:
Heavy chain of RC25 V: 
QVQLVQSGAEVKKPGASVKVSCKASGYTFTNSWIGWFRQAPGQGLEWIGDIYPGGGYT
NYNEIFKGKATMTADTSTNTAYMELSSLRSEDTAVYYCSRGIPGYAMDYWGQGTLVTVS
SASTKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVESKYGPPCPPCPAPEFLGGPSV
FLFPPKPKDTLMISRTPEVTCVVVDVSQEDPEVQFNWYVDGVEVHNAKTKPREEQFNST
YRVVSVLTVLHQDWLNGKEYKCKVSNKGLPSSIEKTISKAKGQPREPQVYTLPPSQEEM
TKNQVSLTCLVKGFYPSDIAVEWESNGQPENNYKTTPPVLDSDGSFFLYSRLTVDKSRWQ
EGNVFSCSVMHEALHNHYTQKSLSLSLG
SEQ ID NO: 97
Light chain of RC25 V:

DIQMTQSPSSLSASVGDRVTMSCKSSQSLLNSGDQKNYLTWYQQKPGKAPKLLIYWAST
GESGVPSRFSGSGSGTDFTFTISSLQPEDIATYYCQNDYSYPWTFGQGTKVEIKRTVAAPS
VFIFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC
SEQ ID NO: 98.

### Test Examples

### Test Example 1: Assessment of Binding of Anti-NPR1 Antibodies to hNPR Proteins by ELISA

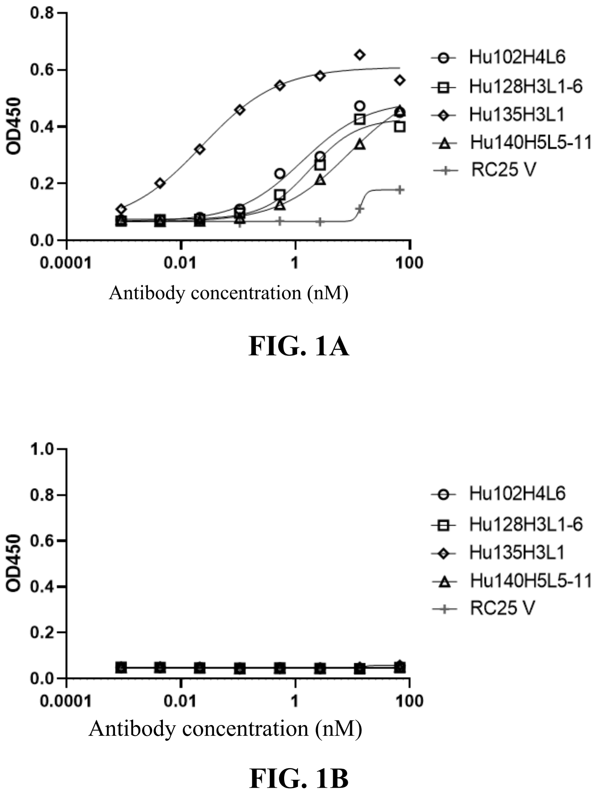

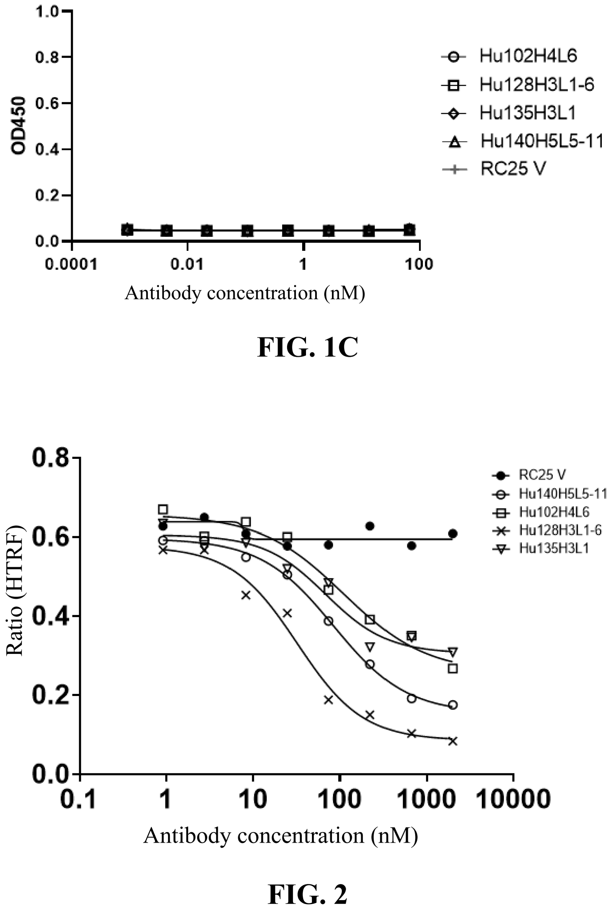

The binding activity of anti-NPR1 antibodies to hNPR1, hNPR2, and hNPR3 was assessed by ELISA. The specific experimental method was as follows: Plates were coated with 4 µg/mL hNPR1 antigen (hNPR1, SEQ ID NO: 1; hNPR2, Abcam, Cat. No. Ab201371; hNPR3, Abcam, Cat. No. Ab114355), respectively, at 37 °C for 3 h, then blocked with PBS containing 5% skim milk powder, and incubated overnight at 4 °C. After the plates were washed three times with PBS (pH 7.4), 20 µg/mL anti-NPR1 antibodies were added to the first wells, and 5-fold serial dilutions were performed in subsequent wells. The plates were then incubated at 37 °C for 1.5 h. After the plates were washed three times with PBS (pH 7.4), Anti-human IgG HRP (Jackson, Cat. No. 109-035-003) was added. The plates were then incubated for 45 min. After the plates were washed six times with PBS (pH 7.4), color development was performed, and readings were taken. The experimental results are shown in FIG. 1A to FIG. 1C and Table 18. The experimental results show that all the anti-NPR1 antibodies of the present disclosure bound to hNPR1 but did not bind to hNPR2 or hNPR3, which belongs to the same family as hNPR1.

**Table 18. ELISA results for the binding of anti-NPR1 antibodies to hNPR proteins**

| | hNPR1 EC₅₀ (nM) | hNPR2 EC₅₀ (nM) | hNPR3 EC₅₀ (nM) |
|---|---|---|---|
| Hul02H4L6 | 1.32 | N.B | N.B |
| Hu128H3L1-6 | 1.84 | N.B | N.B |
| Hu135H3L1 | 0.02 | N.B | N.B |
| Hu140H5L5-11 | 8.65 | N.B | N.B |
| RC25 V | N.B | N.B | N.B |

| | | | |
|---|---|---|---|
| Note: N.B means no binding. | | | |

### Test Example 2: Stimulation of cGMP Production by Anti-NPR1 Antibodies

Upon binding to NPR1, anti-NPR1 antibodies stimulate downstream signaling pathways, leading to cGMP production. In this experiment, the agonistic activity of antibodies can be determined by detecting cGMP. The specific experimental method was as follows: A 1× reaction buffer (5 mM MgCl₂, 10 mM HEPES pH 7.4, and 0.5 mM IBMX in EBSS) was prepared, and solutions of the anti-NPR1 antibodies with different concentrations were prepared using the reaction buffer. The concentration in the first wells was 300 µg/mL, and 3-fold serial dilutions were performed in subsequent wells. The prepared antibody solutions were added to a 384-well plate at 5 µL/well. Meanwhile, hNPR1-CHOK1 cells were washed twice with PBS (pH 7.4) and resuspended in the reaction buffer at 3E6 cells/mL, and the cell suspension was added to the 384-well plate at 5 µL/well. After mixing, the plate was centrifuged at 300 g for 1 min and incubated in an oven at 37 °C for 30 min. Subsequently, follow-up procedures were performed according to the kit instructions (Cisbio, Cat. No. 62GM2PEG). The experimental results are shown in Table 19-1 to Table 19-5 and FIG. 2.

**Table 19-1. The abilities of anti-NPR1 antibodies to induce cGMP production through binding to hNPR1-CHOK1 cells**

| Assay 1 | | | |
|---|---|---|---|
| Antibody | Emin | Emax | EC₅₀ (nM) |
| CHI-102 | 0.23 | 0.57 | 54.9 |
| Hu102H3L6 | 0.16 | 0.61 | 48.5 |
| Hu102H4L6 | 0.17 | 0.65 | 45.9 |
| Hu102H5L6 | 0.21 | 0.59 | 43.6 |
| RC25 V | Cannot be fitted | 0.61 | Cannot be fitted |

**Table 19-2. The abilities of anti-NPR1 antibodies to induce cGMP production through binding to hNPR1-CHOK1 cells**

| Assay 2 | | | |
|---|---|---|---|
| Antibody | Emin | Emax | EC₅₀ (nM) |
| CHI-127 | 0.077 | 0.43 | 42.7 |
| Hu127H4L1 | 0.077 | 0.60 | 45.6 |
| Hu127H4L3 | 0.087 | 0.48 | 51.4 |
| Hu127H4L5 | 0.068 | 0.59 | 32.4 |
| Hu127H4L6 | 0.073 | 0.54 | 38.5 |
| Hu127H7L1 | 0.081 | 0.50 | 55.8 |
| Hu127H7L3 | 0.066 | 0.54 | 56.5 |
| Hu127H7L5 | 0.068 | 0.51 | 42.6 |
| Hu127H7L6 | 0.063 | 0.49 | 35.5 |
| Hu127H8L1 | 0.083 | 0.45 | 62.6 |
| Hu127H8L3 | 0.072 | 0.46 | 48.6 |
| Hu127H8L5 | 0.065 | 0.43 | 42.0 |
| Hu127H8L6 | 0.068 | 0.42 | 36.6 |

**Table 19-3. The abilities of anti-NPR1 antibodies to induce cGMP production through binding to hNPR1-CHOK1 cells**

| Assay 3 | | | |
|---|---|---|---|
| Antibody | Emin | Emax | EC₅₀ (nM) |
| Hu128H3L1 | 0.055 | 0.54 | 5.8 |
| Hu128H3L2 | 0.057 | 0.54 | 4.6 |
| RC25 V | Cannot be fitted | 0.51 | Cannot be fitted |

**Table 19-4. The abilities of anti-NPR1 antibodies to induce cGMP production through binding to hNPR1-CHOK1 cells**

| Assay 4 | | | |
|---|---|---|---|
| Antibody | Emin | Emax | EC₅₀ (nM) |
| Hu128H3L1-6 | 0.050 | 0.59 | 7.9 |
| RC25 V | 0.550 | 0.60 | Cannot be fitted |

**Table 19-5. The abilities of anti-NPR1 antibodies to induce cGMP production through binding to hNPR1-CHOK1 cells**

| Assay 5 | | | |
|---|---|---|---|
| Antibody | Emin | Emax | EC₅₀ (nM) |
| Hu135H1L1 | 0.220 | 0.43 | 116.2 |
| Hu135H2L1 | 0.143 | 0.45 | 185.5 |
| Hu135H3L1 | 0.179 | 0.43 | 170.9 |
| RC25 V | 0.383 | 0.45 | Cannot be fitted |

**Table 19-6. The abilities of anti-NPR1 antibodies to induce cGMP production through binding to hNPR1-CHOK1 cells**

| Assay 6 | | | |
|---|---|---|---|
| Antibody | Emin | Emax | EC₅₀ (nM) |
| Hu140H5L5 | 0.060 | 0.34 | 40.9 |
| Hu140H5L5-11 | 0.053 | 0.37 | 36.0 |
| RC25 V | 0.425 | 0.43 | Cannot be fitted |

The experimental results show that the anti-NPR1 antibodies of the present disclosure all stimulated cGMP production in hNPR1-CHOK1 cells.

### Test Example 3: Endocytosis of Anti-NPR1 Antibodies After Binding to NPR1

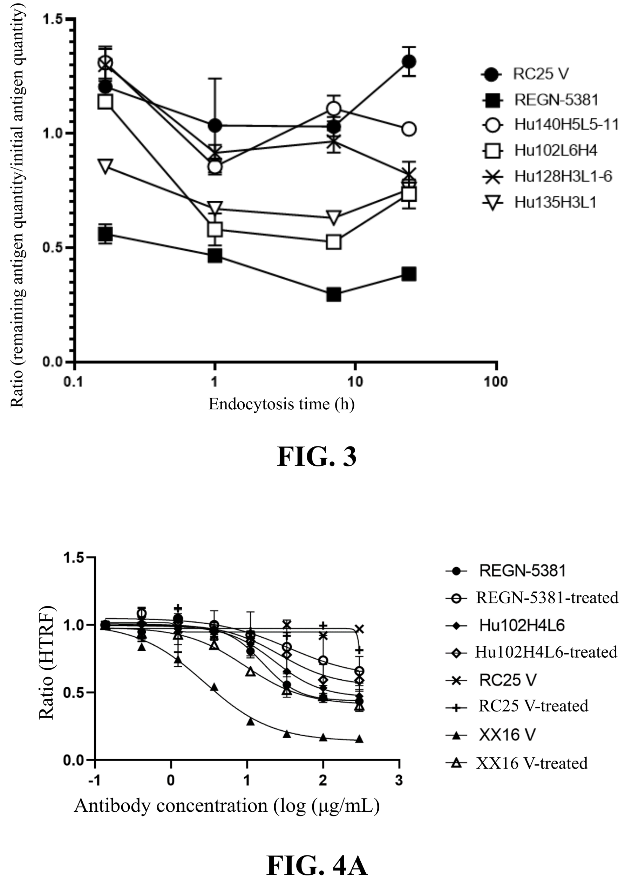

After binding to NPR1, anti-NPR1 antibodies are endocytosed. In this experiment, the intensity of endocytosis of anti-NPR1 antibodies was measured. The specific experimental method was as follows: The density of hNPR1-CHOK1 cells was adjusted to 4E5 cells/mL, and the cell suspension was added to a 96-well cell plate at 50 µL/well. Solutions of the test antibodies were prepared using a CHO-K1 culture medium (DMEM/F12 + 10% FBS, Cat. No. Hyclone SH30023.01; Sangon Biotech, A500023-0100). 20 µg/mL anti-NPR1 antibodies were added to the 96-well cell plate, and the plate was incubated in an incubator for 10 min, 1 h, 7 h, or 24 h. At 10 min, 1 h, 7 h, or 24 h, the culture supernatants were discarded, and trypsin was added to digest the cells. The cells were then transferred to a new 96-well plate, centrifuged at 300 g for 5 min, and washed twice with 1% BSA/PBS. A solution of a detection antibody (REGN-5381-m) was prepared using 1% BSA/PBS and added at 100 µL/well to resuspend the cells, and the cells were incubated at 4 °C for 1 h. Subsequently, the plate was washed twice with 1% BSA/PBS, and APC anti-mouse IgG (Biolegend Cat. No. 405308) was added. The plate was then incubated at 4 °C for 40 min. The cells were washed twice with PBS and resuspended in 1% BSA/PBS, and the fluorescence signal intensity was measured using a flow cytometer (Thermo fisher, Attune Nxt). Data from 10,000 cells were analyzed per well. The experimental results are shown in FIG. 3 and Table 20.

**Table 20. The endocytosis of anti-NPR1 antibodies after binding to hNPR1-CHOK1**

| | | hNPR1-CHOK1 (Ratio* (without ANP)) | | | |
|---|---|---|---|---|---|
| | con (µg/mL) | 10 min | 1 h | 7 hrs | 24 hrs |
| REGN-5381 | 20 | 0.56±0.04 | 0.46±0.01 | 0.30±0.01 | 0.39±0.01 |
| Hu102H4L6 | 20 | 1.14±0.01 | 0.58±0.07 | 0.53±0.02 | 0.73±0.06 |
| Hu128H3L1-6 | 20 | 1.30±0.07 | 0.91±0.04 | 0.96±0.05 | 0.82±0.06 |
| Hu135H3L1 | 20 | 0.85±0.02 | 0.67±0.00 | 0.63±0.03 | 0.76±0.01 |
| Hu140H5L5-11 | 20 | 1.31±0.07 | 0.86±0.04 | 1.11±0.06 | 1.02±0.00 |
| RC25 V | 20 | 1.21±0.01 | 1.03±0.2 | 1.03±0.04 | 1.32±0.06 |

| | | | | | |
|---|---|---|---|---|---|
| *Ratio: The ratio of the number of NPR1 antigens remaining on the cell surface after endocytosis to the initial number of NPR1 antigens. | | | | | |

The experimental results show that the endocytosis of the anti-NPR1 antibodies of the present disclosure was weaker than that of the positive control antibody, thus resulting in a larger quantity of NPR1 antigens remaining on the cell surface. The weaker endocytosis can ensure that the anti-NPR1 antibodies have longer-lasting and more stable pharmacokinetics and efficacy.

### Test Example 4: Stimulation of cGMP Production by Anti-NPR1 Antibodies in Cells After Endocytosis Treatment

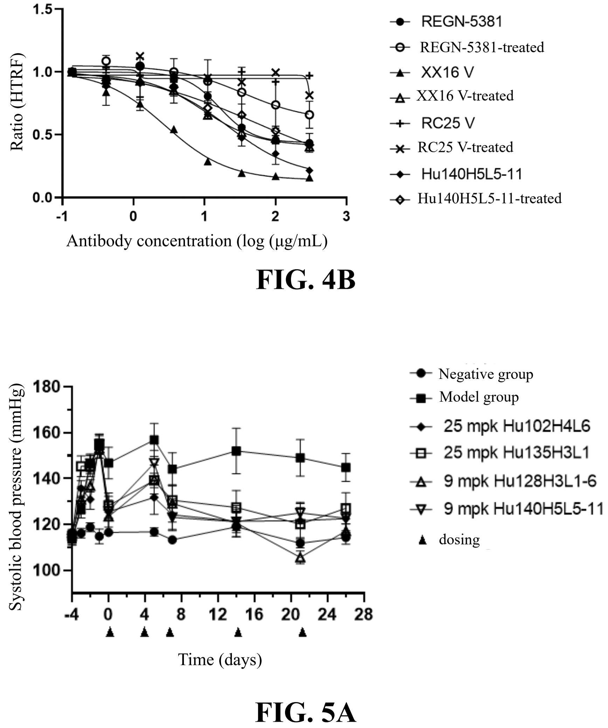

After binding to NPR1, NPR1 antibodies are endocytosed. After endocytosis, the antigen expression level decreases, which, in turn, affects the agonistic activity of anti-NPR1 agonist antibodies. In this experiment, the agonistic activity of NPR1 agonist antibodies on cells after NPR1 endocytosis treatment was assessed. The specific experimental method was as follows: The density of hNPR1-CHOK1 cells was adjusted to 4E5 cells/mL, and the cell suspension was added to a 6-well cell plate at 2 mL/well. Solutions of the test antibodies were prepared using a culture medium and added to the 6-well cell plate at 1 mL/well (with a final concentration of 20 µg/mL). The plate was incubated overnight in an incubator. After the cells were washed twice with PBS (pH 7.4), a cGMP production stimulation experiment with anti-NPR1 antibodies was performed using cells after endocytosis treatment with the anti-NPR1 antibodies and the method in Test Example 1. The experimental results are shown in FIG. 4A, FIG. 4B, and Table 21. The data in the table characterize window values of the stimulation of cGMP production in cells before and after endocytosis treatment by the sum of signal changes, i.e., the sum of the absolute values of the differences between the signal values of cGMP detection in cells before endocytosis treatment and the signal values of cGMP detection in cells after endocytosis treatment, at each test concentration.

**Table 21. Window values of the stimulation of cGMP production in cells by anti-NPR1 antibodies before and after endocytosis treatment**

| Antibody | Sum of signal changes |
|---|---|
| REGN-5381 | 1.08 |
| XX16 V | 1.83 |
| Hu102H4L6 | 0.48 |
| Hu140H5L5-11 | 0.67 |
| RC25 V | 0.11 |

The experimental results show that the anti-NPR1 antibodies of the present disclosure had cGMP production with smaller differences on cells after endocytosis occurred than the positive antibodies did.

### Test Example 5: In Vivo Efficacy of Anti-NPR1 Antibodies in hNPR1 Transgenic Mouse ANGII Model

To evaluate the *in vivo* efficacy of anti-NPR1 antibodies, a mouse model was established by inducing progressive heart failure accompanied by hypertension in hNPR1 homozygous transgenic mice using ANGII. Blood pressure was evaluated, and the hypotensive efficacy of the antibodies was evaluated based on parameters such as cGMP in plasma and urine and biochemical indicators of urine.

The specific modeling procedure and experimental method were as follows: After mice were pre-acclimatized to the heat-retaining cylinder of a blood pressure monitor and their basal blood pressure was measured, an Alzet Micro-Osmotic Pump (model: 1004; containing angiotensin II acetate, with the average pump speed set at 0.11 µL/h to deliver AngII at 1.5 mg/kg/day) was subcutaneously implanted into the scapular region of the mice 3 days before the start of administration. Their blood pressure was measured for three consecutive days, and mice with difficult-to-measure or greatly fluctuating blood pressure were excluded. The remaining mice were divided into groups of 8, primarily according to their mean arterial pressure (MAP) and secondarily according to their body weight, and dosed via subcutaneous injection on day 0, day 4, day 7, day 14, and day 21 at a volume of 10 mL/kg. Blood pressure was measured as scheduled. In addition, plasma was collected on day 2, day 6, day 9, day 11, day 16, day 18, day 25, and day 29, and cumulative urine was collected on day 2, day 6, day 9, day 11, day 16, day 18, and day 25. The corresponding cGMP content was measured.

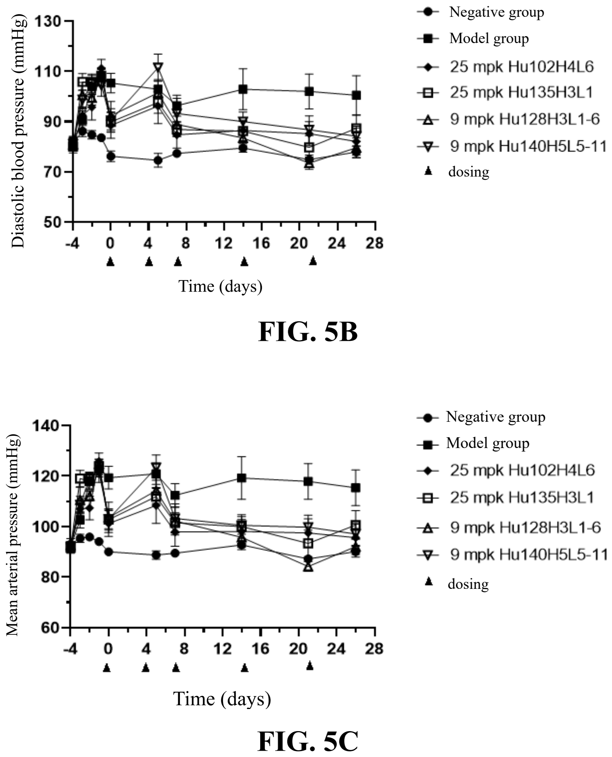

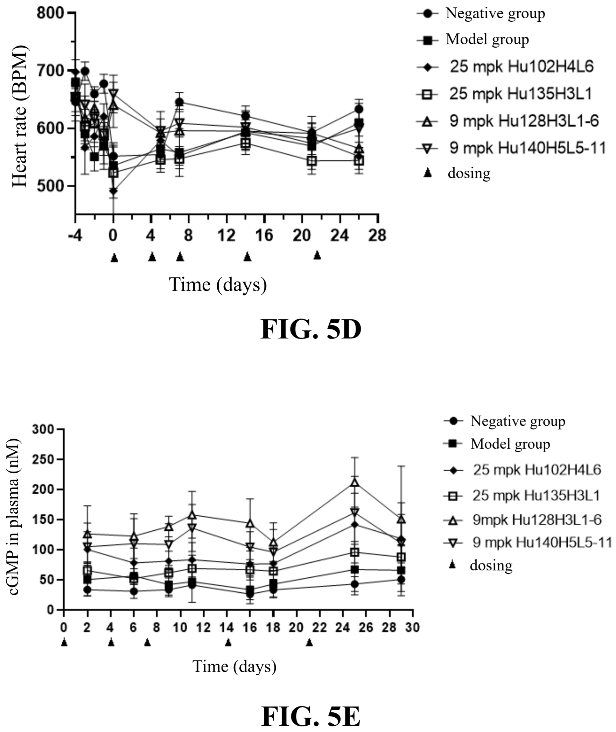

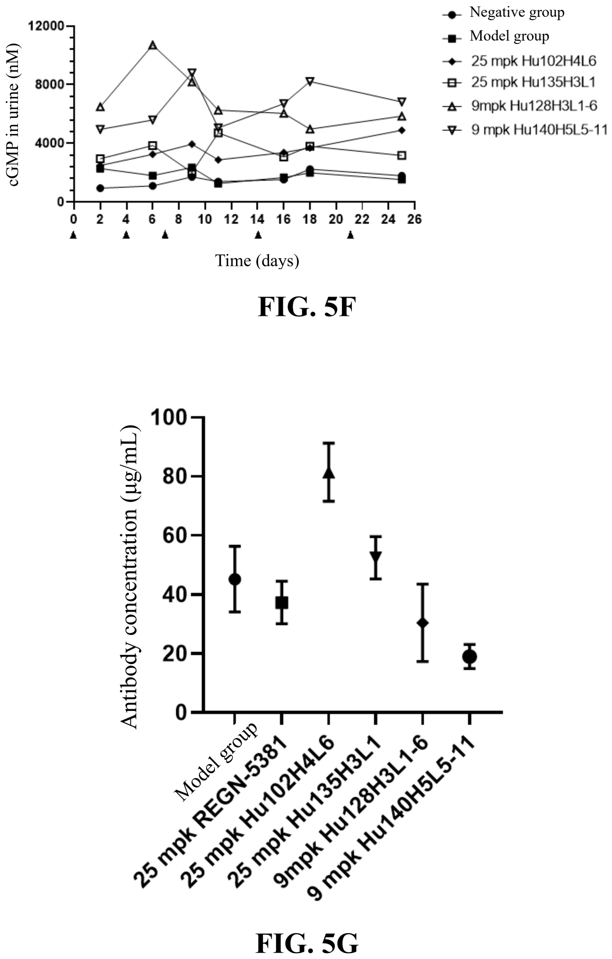

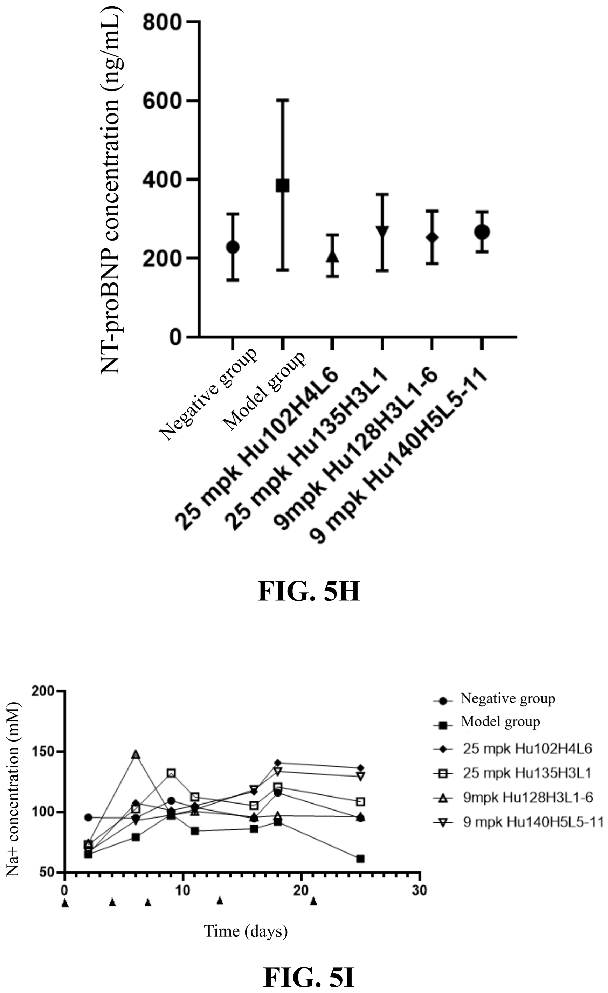

The experimental results are shown in FIG. 5A to FIG. 5I and Table 22, with mean indicators of 29 days summarized in the Table.

**Table 22. The in vivo efficacy of anti-NPR1 antibodies in an hNPR1 transgenic mouse ANGII model**

| **Antibody** | **Mean arterial blood pressure (MAP mmHG)** | **Mean cGMP concentration in plasma (nM)** | **Mean cGMP concentration in urine (nM)** | **Mean Na+ concentration in urine (mM)** | **Mean NT-proBNP concentration in plasma (ng/mL)** |
|---|---|---|---|---|---|
| **Negative group** | 91.67 **** | 36.57 ** | 1534.16 | 101.47 | 229.03 |
| **Model group** | 114.10 | 50.68 | 1845.57 | 80.93 | 385.88 |
| **25 mpk Hu102H4L6** | 103.67 **** | 94.49*** | 3504.46 * | 110.60 (p=0.07) | 207.27** |
| **25 mpk Hu135H3L1** | 106.10 *** | 70.41 ** | 3370.33 | 107.92 (p=0.12) | 265.62 (p=0.11) |
| **9 mpk Hu128H3L1-6** | 103.06 **** | 145.74 *** | 6931.09 ** | 101.35 (p=0.35) | 253.51 (p=0.08) |
| **9 mpk Hu140H5L5-11** | 106.81 *** | 116.59 *** | 6591.68 *** | 105.94 (p=0.17) | 267.43 (p=0.12) |

| | | | | | |
|---|---|---|---|---|---|
| Note: NT-proBNP is a biomarker for cardiac dysfunction. Statistical analysis: With the model group as a control, analysis was performed using the two-way ANOVA method; *p < 0.05, **p < 0.01, ***p < 0.001, ****p < 0.0001 | | | | | |

The experimental results show that the anti-NPR1 antibodies of the present disclosure all achieved significant blood pressure control effects and cGMP production effects, and exhibited non-statistically significant trends of accelerating sodium excretion in urine and lowering NT-proBNP. In another aspect, when administered at the same dose and frequency, the weak-endocytosis molecule Hu102H4L6 achieved a significantly higher endpoint plasma concentration than the positive control REGN-5381 did (FIG. 5G).

### Test Example 6: In Vivo Pharmacokinetic Experiment of Anti-NPR1 Antibodies

hNPR1 transgenic mice were divided into groups of 3 and dosed via intravenous injection at 3 mpk. Whole blood samples (0.15 mL each) were collected at 5 min, 1 d, 2 d, 4 d, 7 d, 10 d, 14 d, and 21 d post-dose, and no anticoagulant was added. After collection, the blood samples were left to stand at room temperature for 30 min and centrifuged at 1000 g for 15 min. The supernatants (serum) were collected, placed into EP tubes, and stored at -20 °C. Standard curves for different samples were made using the Total IgG method, and the concentrations of the anti-NPR1 antibodies in serum at different time points were calculated using OD450 values. The resulting data were analyzed using Phoenix WinNonlin software to calculate related pharmacokinetic parameters. The experimental results are shown in Table 23.

**Table 23. The results of an in vivo pharmacokinetic experiment of anti-NPR1 antibodies**

| | |
|---|---|
| | 140H5L5-11 3 mpk |
| t1/2 (d) | 7.5 |
| Cmax (µg/mL) | 87.01 |
| AUC ₀₋ₜ (µg/mL*h) | 9383.51 |
| AUC _{0-∞} (µg/mL*h) | 11034.04 |
| Vz (mL/kg) | 72.23 |
| CL (mL/day/kg) | 6.94 |
| MRT 0-∞ (h) | 247.73 |

| | |
|---|---|
| Note: t1/2 = terminal elimination half-life; Cmax = peak concentration; AUC₀₋ₜ = area under the plasma concentration-time curve; AUC_{0-∞} = AUC₀₋ₜ + terminal point concentration/terminal elimination rate; Vz = apparent volume of distribution; CL = clearance of antibody over time; MRT 0-∞ = mean residence time. | |

The experimental results show that 140H5L5-11 achieved relatively good pharmacokinetic performance in mice at 3 mpk.

Although the above invention has been described in detail using drawings and examples for a clear understanding, the description and examples should not be construed as limiting the scope of the present disclosure. The disclosures of all the patents and scientific documents cited herein are clearly incorporated by reference in their entirety.

## Claims

1. An anti-NPR1 antibody, comprising a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
a. the HCDR1, the HCDR2, and the HCDR3 of the heavy chain variable region comprise the amino acid sequences of a HCDR1, a HCDR2, and a HCDR3 in SEQ ID NO: 82, 80, 81, or 15, respectively, and the LCDR1, the LCDR2, and the LCDR3 of the light chain variable region comprise the amino acid sequences of a LCDR1, a LCDR2, and a LCDR3 in SEQ ID NO: 85, 83, 84, or 16, respectively; or
b. the HCDR1, the HCDR2, and the HCDR3 of the heavy chain variable region comprise the amino acid sequences of a HCDR1, a HCDR2, and a HCDR3 of any one of SEQ ID NO: 51, 48, 49, 50, 52, or 7, respectively, and the LCDR1, the LCDR2, and the LCDR3 of the light chain variable region comprise the amino acid sequences of a LCDR1, a LCDR2, and a LCDR3 in SEQ ID NO: 56, 53, 54, 55, or 8, respectively; or
c. the HCDR1, the HCDR2, and the HCDR3 of the heavy chain variable region comprise the amino acid sequences of a HCDR1, a HCDR2, and a HCDR3 in SEQ ID NO: 68, 66, 67, 69, or 11, respectively, and the LCDR1, the LCDR2, and the LCDR3 of the light chain variable region comprise the amino acid sequences of a LCDR1, a LCDR2, and a LCDR3 in SEQ ID NO: 74, 70, 71, 72, or 12, respectively; or
d. the HCDR1, the HCDR2, and the HCDR3 of the heavy chain variable region comprise the amino acid sequences of a HCDR1, a HCDR2, and a HCDR3 in SEQ ID NO: 78, 76, 77, or 13, respectively, and the LCDR1, the LCDR2, and the LCDR3 of the light chain variable region comprise the amino acid sequences of a LCDR1, a LCDR2, and a LCDR3 in SEQ ID NO: 79 or 14, respectively; or
e. the HCDR1, the HCDR2, and the HCDR3 of the heavy chain variable region comprise the amino acid sequences of a HCDR1, a HCDR2, and a HCDR3 in SEQ ID NO: 58, 59, 60, or 9, respectively, and the LCDR1, the LCDR2, and the LCDR3 of the light chain variable region comprise the amino acid sequences of a LCDR1, a LCDR2, and a LCDR3 in SEQ ID NO: 61, 62, 63, 64, 65, or 10, respectively;
preferably,
a. the HCDR1, the HCDR2, and the HCDR3 of the heavy chain variable region comprise the amino acid sequences of a HCDR1, a HCDR2, and a HCDR3 in SEQ ID NO: 82, respectively, and the LCDR1, the LCDR2, and the LCDR3 of the light chain variable region comprise the amino acid sequences of a LCDR1, a LCDR2, and a LCDR3 in SEQ ID NO: 85, respectively; or
b. the HCDR1, the HCDR2, and the HCDR3 of the heavy chain variable region comprise the amino acid sequences of a HCDR1, a HCDR2, and a HCDR3 in SEQ ID NO: 51, respectively, and the LCDR1, the LCDR2, and the LCDR3 of the light chain variable region comprise the amino acid sequences of a LCDR1, a LCDR2, and a LCDR3 in SEQ ID NO: 56, respectively; or
c. the HCDR1, the HCDR2, and the HCDR3 of the heavy chain variable region comprise the amino acid sequences of a HCDR1, a HCDR2, and a HCDR3 in SEQ ID NO: 68, respectively, and the LCDR1, the LCDR2, and the LCDR3 of the light chain variable region comprise the amino acid sequences of a LCDR1, a LCDR2, and a LCDR3 in SEQ ID NO: 74, respectively; or
d. the HCDR1, the HCDR2, and the HCDR3 of the heavy chain variable region comprise the amino acid sequences of a HCDR1, a HCDR2, and a HCDR3 in SEQ ID NO: 78, respectively, and the LCDR1, the LCDR2, and the LCDR3 of the light chain variable region comprise the amino acid sequences of a LCDR1, a LCDR2, and a LCDR3 in SEQ ID NO: 79, respectively; or
e. the HCDR1, the HCDR2, and the HCDR3 of the heavy chain variable region comprise the amino acid sequences of a HCDR1, a HCDR2, and a HCDR3 in SEQ ID NO: 58, respectively, and the LCDR1, the LCDR2, and the LCDR3 of the light chain variable region comprise the amino acid sequences of a LCDR1, a LCDR2, and a LCDR3 in SEQ ID NO: 61, respectively.

2. The anti-NPR1 antibody according to claim 1, wherein:
a. in the heavy chain variable region, the HCDR1 comprises the amino acid sequence of SEQ ID NO: 29, the HCDR2 comprises the amino acid sequence of SEQ ID NO: 40, and the HCDR3 comprises the amino acid sequence of SEQ ID NO: 41, and in the light chain variable region, the LCDR1 comprises the amino acid sequence of SEQ ID NO: 42, the LCDR2 comprises the amino acid sequence of SEQ ID NO: 33, and the LCDR3 comprises the amino acid sequence of SEQ ID NO: 34; or
b. in the heavy chain variable region, the HCDR1 comprises the amino acid sequence of SEQ ID NO: 45, 17, or 35, the HCDR2 comprises the amino acid sequence of SEQ ID NO: 46 or 18, and the HCDR3 comprises the amino acid sequence of SEQ ID NO: 19, and in the light chain variable region, the LCDR1 comprises the amino acid sequence of SEQ ID NO: 20, the LCDR2 comprises the amino acid sequence of SEQ ID NO: 21, and the LCDR3 comprises the amino acid sequence of SEQ ID NO: 22; or
c. in the heavy chain variable region, the HCDR1 comprises the amino acid sequence of SEQ ID NO: 29, the HCDR2 comprises the amino acid sequence of SEQ ID NO: 30, and the HCDR3 comprises the amino acid sequence of SEQ ID NO: 31, and in the light chain variable region, the LCDR1 comprises the amino acid sequence of SEQ ID NO: 32, the LCDR2 comprises the amino acid sequence of SEQ ID NO: 73 or 33, and the LCDR3 comprises the amino acid sequence of SEQ ID NO: 34; or
d. in the heavy chain variable region, the HCDR1 comprises the amino acid sequence of SEQ ID NO: 35, the HCDR2 comprises the amino acid sequence of SEQ ID NO: 75 or 36, and the HCDR3 comprises the amino acid sequence of SEQ ID NO: 37, and in the light chain variable region, the LCDR1 comprises the amino acid sequence of SEQ ID NO: 38, the LCDR2 comprises the amino acid sequence of SEQ ID NO: 21, and the LCDR3 comprises the amino acid sequence of SEQ ID NO: 39; or
e. in the heavy chain variable region, the HCDR1 comprises the amino acid sequence of SEQ ID NO: 23, the HCDR2 comprises the amino acid sequence of SEQ ID NO: 57 or 24, and the HCDR3 comprises the amino acid sequence of SEQ ID NO: 25, and in the light chain variable region, the LCDR1 comprises the amino acid sequence of SEQ ID NO: 26, the LCDR2 comprises the amino acid sequence of SEQ ID NO: 27, and the LCDR3 comprises the amino acid sequence of SEQ ID NO: 28;
preferably,
a. in the heavy chain variable region, the HCDR1 comprises the amino acid sequence of SEQ ID NO: 29, the HCDR2 comprises the amino acid sequence of SEQ ID NO: 40, and the HCDR3 comprises the amino acid sequence of SEQ ID NO: 41, and in the light chain variable region, the LCDR1 comprises the amino acid sequence of SEQ ID NO: 42, the LCDR2 comprises the amino acid sequence of SEQ ID NO: 33, and the LCDR3 comprises the amino acid sequence of SEQ ID NO: 34; or
b. in the heavy chain variable region, the HCDR1 comprises the amino acid sequence of SEQ ID NO: 45, the HCDR2 comprises the amino acid sequence of SEQ ID NO: 46, and the HCDR3 comprises the amino acid sequence of SEQ ID NO: 19, and in the light chain variable region, the LCDR1 comprises the amino acid sequence of SEQ ID NO: 20, the LCDR2 comprises the amino acid sequence of SEQ ID NO: 21, and the LCDR3 comprises the amino acid sequence of SEQ ID NO: 22; or
c. in the heavy chain variable region, the HCDR1 comprises the amino acid sequence of SEQ ID NO: 29, the HCDR2 comprises the amino acid sequence of SEQ ID NO: 30, and the HCDR3 comprises the amino acid sequence of SEQ ID NO: 31, and in the light chain variable region, the LCDR1 comprises the amino acid sequence of SEQ ID NO: 32, the LCDR2 comprises the amino acid sequence of SEQ ID NO: 73, and the LCDR3 comprises the amino acid sequence of SEQ ID NO: 34; or
d. in the heavy chain variable region, the HCDR1 comprises the amino acid sequence of SEQ ID NO: 35, the HCDR2 comprises the amino acid sequence of SEQ ID NO: 75, and the HCDR3 comprises the amino acid sequence of SEQ ID NO: 37, and in the light chain variable region, the LCDR1 comprises the amino acid sequence of SEQ ID NO: 38, the LCDR2 comprises the amino acid sequence of SEQ ID NO: 21, and the LCDR3 comprises the amino acid sequence of SEQ ID NO: 39; or
e. in the heavy chain variable region, the HCDR1 comprises the amino acid sequence of SEQ ID NO: 23, the HCDR2 comprises the amino acid sequence of SEQ ID NO: 57, and the HCDR3 comprises the amino acid sequence of SEQ ID NO: 25, and in the light chain variable region, the LCDR1 comprises the amino acid sequence of SEQ ID NO: 26, the LCDR2 comprises the amino acid sequence of SEQ ID NO: 27, and the LCDR3 comprises the amino acid sequence of SEQ ID NO: 28;
more preferably,
a. in the heavy chain variable region, the HCDR1 comprises the amino acid sequence of SEQ ID NO: 29, the HCDR2 comprises the amino acid sequence of SEQ ID NO: 40, and the HCDR3 comprises the amino acid sequence of SEQ ID NO: 41, and in the light chain variable region, the LCDR1 comprises the amino acid sequence of SEQ ID NO: 42, the LCDR2 comprises the amino acid sequence of SEQ ID NO: 33, and the LCDR3 comprises the amino acid sequence of SEQ ID NO: 34; or
b. in the heavy chain variable region, the HCDR1 comprises the amino acid sequence of SEQ ID NO: 45, the HCDR2 comprises the amino acid sequence of SEQ ID NO: 46, and the HCDR3 comprises the amino acid sequence of SEQ ID NO: 19, and in the light chain variable region, the LCDR1 comprises the amino acid sequence of SEQ ID NO: 20, the LCDR2 comprises the amino acid sequence of SEQ ID NO: 21, and the LCDR3 comprises the amino acid sequence of SEQ ID NO: 22.

3. The anti-NPR1 antibody according to claim 1 or 2, being a murine antibody, a chimeric antibody, a humanized antibody, or a fully human-derived antibody, preferably a humanized antibody.

4. The anti-NPR1 antibody according to any one of claims 1 to 3, wherein:
a. the heavy chain variable region comprises SEQ ID NO: 82, 80, or 81 or an amino acid sequence having at least 85% sequence identity thereto, and the light chain variable region comprises SEQ ID NO: 85, 83, or 84 or an amino acid sequence having at least 85% sequence identity thereto; or
b. the heavy chain variable region comprises SEQ ID NO: 51, 48, 49, 50, or 52 or an amino acid sequence having at least 85% sequence identity thereto, and the light chain variable region comprises SEQ ID NO: 56, 53, 54, or 55 or an amino acid sequence having at least 85% sequence identity thereto; or
c. the heavy chain variable region comprises SEQ ID NO: 68, 66, 67, or 69 or an amino acid sequence having at least 85% sequence identity thereto, and the light chain variable region comprises SEQ ID NO: 74, 70, 71, or 72 or an amino acid sequence having at least 85% sequence identity thereto; or
d. the heavy chain variable region comprises SEQ ID NO: 78, 76, or 77 or an amino acid sequence having at least 85% sequence identity thereto, and the light chain variable region comprises SEQ ID NO: 79 or an amino acid sequence having at least 85% sequence identity thereto; or
e. the heavy chain variable region comprises SEQ ID NO: 58, 59, or 60 or an amino acid sequence having at least 85% sequence identity thereto, and the light chain variable region comprises SEQ ID NO: 61, 62, 63, 64, or 65 or an amino acid sequence having at least 85% sequence identity thereto; or
f. the heavy chain variable region comprises SEQ ID NO: 15 or an amino acid sequence having at least 80% sequence identity thereto, and the light chain variable region comprises SEQ ID NO: 16 or an amino acid sequence having at least 80% sequence identity thereto; or
g. the heavy chain variable region comprises SEQ ID NO: 7 or an amino acid sequence having at least 80% sequence identity thereto, and the light chain variable region comprises SEQ ID NO: 8 or an amino acid sequence having at least 80% sequence identity thereto; or
h. the heavy chain variable region comprises SEQ ID NO: 11 or an amino acid sequence having at least 80% sequence identity thereto, and the light chain variable region comprises SEQ ID NO: 12 or an amino acid sequence having at least 80% sequence identity thereto; or
i. the heavy chain variable region comprises SEQ ID NO: 13 or an amino acid sequence having at least 80% sequence identity thereto, and the light chain variable region comprises SEQ ID NO: 14 or an amino acid sequence having at least 80% sequence identity thereto; or
j. the heavy chain variable region comprises SEQ ID NO: 9 or an amino acid sequence having at least 80% sequence identity thereto, and the light chain variable region comprises SEQ ID NO: 10 or an amino acid sequence having at least 80% sequence identity thereto;
preferably,
a. the heavy chain variable region comprises the amino acid sequence of SEQ ID NO: 82, and the light chain variable region comprises the amino acid sequence of SEQ ID NO: 85; or
the heavy chain variable region comprises the amino acid sequence of SEQ ID NO: 81 or 82, and the light chain variable region comprises the amino acid sequence of SEQ ID NO: 84; or
b. the heavy chain variable region comprises the amino acid sequence of SEQ ID NO: 51, 49, 50, or 52, and the light chain variable region comprises the amino acid sequence of SEQ ID NO: 56; or
c. the heavy chain variable region comprises the amino acid sequence of SEQ ID NO: 68, and the light chain variable region comprises the amino acid sequence of SEQ ID NO: 74, 70, or 71; or
the heavy chain variable region comprises the amino acid sequence of SEQ ID NO: 67, and the light chain variable region comprises the amino acid sequence of SEQ ID NO: 70 or 71; or
d. the heavy chain variable region comprises the amino acid sequence of SEQ ID NO: 78, 76, or 77, and the light chain variable region comprises the amino acid sequence of SEQ ID NO: 79; or
e. the heavy chain variable region comprises the amino acid sequence of SEQ ID NO: 58, and the light chain variable region comprises the amino acid sequence of SEQ ID NO: 61, 62, 64, or 65; or
the heavy chain variable region comprises the amino acid sequence of SEQ ID NO: 59, and the light chain variable region comprises the amino acid sequence of SEQ ID NO: 61, 62, 64, or 65; or
the heavy chain variable region comprises the amino acid sequence of SEQ ID NO: 60, and the light chain variable region comprises the amino acid sequence of SEQ ID NO: 61, 62, 64, or 65; or
f. the heavy chain variable region comprises the amino acid sequence of SEQ ID NO: 15, and the light chain variable region comprises the amino acid sequence of SEQ ID NO: 16; or
g. the heavy chain variable region comprises the amino acid sequence of SEQ ID NO: 7, and the light chain variable region comprises the amino acid sequence of SEQ ID NO: 8; or
h. the heavy chain variable region comprises the amino acid sequence of SEQ ID NO: 11, and the light chain variable region comprises the amino acid sequence of SEQ ID NO: 12; or
i. the heavy chain variable region comprises the amino acid sequence of SEQ ID NO: 13, and the light chain variable region comprises the amino acid sequence of SEQ ID NO: 14; or
j. the heavy chain variable region comprises the amino acid sequence of SEQ ID NO: 9, and the light chain variable region comprises the amino acid sequence of SEQ ID NO: 10;
more preferably,
a. the heavy chain variable region comprises the amino acid sequence of SEQ ID NO: 82, and the light chain variable region comprises the amino acid sequence of SEQ ID NO: 85; or
b. the heavy chain variable region comprises the amino acid sequence of SEQ ID NO: 51, and the light chain variable region comprises the amino acid sequence of SEQ ID NO: 56; or
c. the heavy chain variable region comprises the amino acid sequence of SEQ ID NO: 68, and the light chain variable region comprises the amino acid sequence of SEQ ID NO: 74; or
d. the heavy chain variable region comprises the amino acid sequence of SEQ ID NO: 78, and the light chain variable region comprises the amino acid sequence of SEQ ID NO: 79;
most preferably,
a. the heavy chain variable region comprises the amino acid sequence of SEQ ID NO: 82, and the light chain variable region comprises the amino acid sequence of SEQ ID NO: 85; or
b. the heavy chain variable region comprises the amino acid sequence of SEQ ID NO: 51, and the light chain variable region comprises the amino acid sequence of SEQ ID NO: 56.

5. The anti-NPR1 antibody according to any one of claims 1 to 4, wherein the anti-NPR1 antibody is an antibody fragment; preferably, the antibody fragment is a Fab, Fab', F(ab')2, Fd, Fv, scFv, dsFv, or dAb.

6. The anti-NPR1 antibody according to any one of claims 1 to 4, wherein the anti-NPR1 antibody comprises a heavy chain constant region and a light chain constant region;
preferably, the heavy chain constant region is a heavy chain constant region of human IgG1, IgG2, IgG3, IgG4, or a variant thereof, and the light chain constant region is a human κ or λ light chain constant region;
more preferably, the heavy chain constant region comprises the amino acid sequence of SEQ ID NO: 86 or 43, and the light chain constant region comprises the amino acid sequence of SEQ ID NO: 44.

7. The anti-NPR1 antibody according to claim 6, wherein the anti-NPR1 antibody comprises a heavy chain and a light chain, wherein:
a. the heavy chain comprises SEQ ID NO: 89 or 127 or an amino acid sequence having at least 85% sequence identity thereto, and the light chain comprises SEQ ID NO: 90 or 128 or an amino acid sequence having at least 85% sequence identity thereto; or
the heavy chain comprises SEQ ID NO: 110 or an amino acid sequence having at least 85% sequence identity thereto, and the light chain comprises SEQ ID NO: 111 or an amino acid sequence having at least 85% sequence identity thereto; or
b. the heavy chain comprises SEQ ID NO: 87, 112, 113, or 114 or an amino acid sequence having at least 85% sequence identity thereto, and the light chain comprises SEQ ID NO: 88 or an amino acid sequence having at least 85% sequence identity thereto; or
the heavy chain comprises SEQ ID NO: 102 or an amino acid sequence having at least 85% sequence identity thereto, and the light chain comprises SEQ ID NO: 103 or an amino acid sequence having at least 85% sequence identity thereto; or
c. the heavy chain comprises SEQ ID NO: 99 or 122 or an amino acid sequence having at least 85% sequence identity thereto, and the light chain comprises SEQ ID NO: 100, 123, or 124 or an amino acid sequence having at least 85% sequence identity thereto; or the heavy chain comprises SEQ ID NO: 106 or an amino acid sequence having at least 85% sequence identity thereto, and the light chain comprises SEQ ID NO: 107 or an amino acid sequence having at least 85% sequence identity thereto; or
d. the heavy chain comprises SEQ ID NO: 101, 125, or 126 or an amino acid sequence having at least 85% sequence identity thereto, and the light chain comprises SEQ ID NO: 47 or an amino acid sequence having at least 85% sequence identity thereto;
the heavy chain comprises SEQ ID NO: 108 or an amino acid sequence having at least 85% sequence identity thereto, and the light chain comprises SEQ ID NO: 109 or an amino acid sequence having at least 85% sequence identity thereto; or
e. the heavy chain comprises SEQ ID NO: 115, 116, or 117 or an amino acid sequence having at least 85% sequence identity thereto, and the light chain comprises SEQ ID NO: 118, 119, 120, or 121 or an amino acid sequence having at least 85% sequence identity thereto; or
the heavy chain comprises SEQ ID NO: 104 or an amino acid sequence having at least 85% sequence identity thereto, and the light chain comprises SEQ ID NO: 105 or an amino acid sequence having at least 85% sequence identity thereto;
preferably,
a. the heavy chain comprises the amino acid sequence of SEQ ID NO: 89, and the light chain comprises the amino acid sequence of SEQ ID NO: 90; or
b. the heavy chain comprises the amino acid sequence of SEQ ID NO: 87, and the light chain comprises the amino acid sequence of SEQ ID NO: 88; or
c. the heavy chain comprises the amino acid sequence of SEQ ID NO: 99, and the light chain comprises the amino acid sequence of SEQ ID NO: 100; or
d. the heavy chain comprises the amino acid sequence of SEQ ID NO: 101, and the light chain comprises the amino acid sequence of SEQ ID NO: 47;
more preferably,
a. the heavy chain comprises the amino acid sequence of SEQ ID NO: 89, and the light chain comprises the amino acid sequence of SEQ ID NO: 90; or
b. the heavy chain comprises the amino acid sequence of SEQ ID NO: 87, and the light chain comprises the amino acid sequence of SEQ ID NO: 88.

8. A pharmaceutical composition, comprising the anti-NPR1 antibody according to any one of claims 1 to 7 and one or more pharmaceutically acceptable carriers, diluents, or excipients, wherein preferably, the pharmaceutical composition further comprises at least one second therapeutic agent.

9. An immunoconjugate, comprising the anti-NPR1 antibody according to any one of claims 1 to 7 and an effector molecule, wherein the effector molecule is conjugated to the anti-NPR1 antibody; preferably, the effector molecule is selected from the group consisting of an anti-tumor agent, an immunomodulator, a biological response modifier, a lectin, a cytotoxic drug, a chromophore, a fluorophore, a chemiluminescent compound, an enzyme, a metal ion, and any combination thereof.

10. An isolated nucleic acid, wherein the isolated nucleic acid encodes the anti-NPR1 antibody according to any one of claims 1 to 7.

11. A host cell, comprising the isolated nucleic acid according to claim 10.

12. A method for detecting an NPR1 peptide or a fragment thereof in a sample, comprising contacting the sample with the anti-NPR1 antibody according to any one of claims 1 to 7 and detecting the presence of a complex between the anti-NPR1 antibody and the NPR1 peptide or the fragment thereof, wherein that the complex is detected indicates the presence of the NPR1 peptide or the fragment thereof.

13. A method for treating, preventing, or ameliorating an NPR1-related disease or disorder, comprising administering to a subject a therapeutically or prophylactically effective amount of the anti-NPR1 antibody according to any one of claims 1 to 7, or the pharmaceutical composition according to claim 8, or the immunoconjugate according to claim 9.

14. The method according to claim 13, wherein the NPR1-related disease or disorder is selected from the group consisting of heart failure, hypertension, peripheral vascular disease, coronary artery disease (CAD), ischemic heart disease (IHD), mitral stenosis and regurgitation, angina pectoris, hypertrophic cardiomyopathy (HCM), diabetic cardiomyopathy, supraventricular and ventricular cardiac arrhythmias, arrhythmia, atrial fibrillation (AF), cardiac fibrosis, atrial flutter, adverse vascular remodeling, plaque stabilization, myocardial infarction (MI), preeclampsia, obesity, renal failure, renal disorder, cytokine release syndrome, chronic kidney disease, macular edema, glaucoma, stroke, pulmonary disease, inflammation, asthma, skeletal growth disorder, fracture, and diabetes.

15. The method according to claim 14, wherein the heart failure is selected from the group consisting of heart failure with preserved ejection fraction (HFpEF), heart failure with reduced ejection fraction (HFrEF), heart failure with mildly reduced ejection fraction (HFmrEF), heart failure after acute myocardial infarction, and acute decompensated heart failure, wherein the hypertension is resistant hypertension.
